# EUROPÄISCHE PATENTSCHRIFT

(11) **EP 0 848 700 B1**
(45) Veröffentlichungstag und Bekanntmachung des Hinweises auf die Patenterteilung: **02.07.2003**
(21) Anmeldenummer: 96929273.9
(22) Anmeldetag: 19.08.1996
(51) Int. Cl.: C07C 237/44, C07C 235/58, C07C 235/64, C07C 235/62, A01N 37/18, A01N 37/24

(54) **ACYLAMINOSALICYLSÄUREAMIDE UND IHRE VERWENDUNG ALS SCHÄDLINGSBEKÄMPFUNGSMITTEL**
ACYLAMINOSALICYLIC ACID AMIDES AND THEIR USES AS PESTICIDES
AMIDES D'ACIDE ACYLAMINOSALICYLIQUE ET LEUR UTILISATION COMME PESTICIDES

(30) Priorität: 30.08.1995 DE 19531891; 19.04.1996 DE 19615453; 01.07.1996 DE 19626311
(43) Veröffentlichungstag der Anmeldung: 24.06.1998
(73) Patentinhaber: Bayer CropScience AG, 40789 Monheim (DE)
(72) Erfinder: SEITZ, Thomas, D-40764 Langenfeld (DE); NAUMANN, Klaus, D-51375 Leverkusen (DE); TIEMANN, Ralf, D-51375 Leverkusen (DE); STENZEL, Klaus, D-40595 Düsseldorf (DE); HÄNSSLER, Gerd, D-51381 Leverkusen (DE); DUTZMANN, Stefan, D-40721 Hilden (DE)
(86) Internationale Anmeldenummer: EP9603637
(87) Internationale Veröffentlichungsnummer: WO97008135

(56) Entgegenhaltungen:
- DE-A- 2 126 149
- FR-A- 1 501 151
- US-A- 3 148 995
- US-A- 3 929 879
- JOURNAL OF PHARMACEUTICAL SCIENCES, Bd. 52, Nr. 6, 1963, WASHINGTON US, Seiten 542-545, XP002020731
- CHEMICAL ABSTRACTS, vol. 122, no. 3, 16.Januar 1995 Columbus, Ohio, US; abstract no. 29879, SUZUKI, AKINORI ET AL.: "Pesticides TOD-4403 manufacture with streptomyces" Seite 792; XP002020733 & JP 06 239 844 A (KUMIAI CHEMICAL INDUSTRY)
- CHEMICAL ABSTRACTS, vol. 119, no. 15, 11.Oktober 1993 Columbus, Ohio, US; abstract no. 158366, IMAMARU, NOBUTAKA ET AL.: "Fungicidal urauchimycin A und B and their manufacture with streptomyces species" XP002020734 & JP 05 255 877 A (KAIYO BAIO TEKUNOROJII KENKYUS)
- BULLETIN OF THE CHEMICAL SOCIETY OF JAPAN, Bd. 44, Nr. 12, 1971, TOKYO JP, Seiten 3395-9, XP002020732 MITSUHIRO KINOSHITA ET AL.: "Synthesis of (2S, 4R, 15S)-4,15-dimethyl-1,5-dioxa-3-(3'-formami dosalicylamido)cyclopentadecane-2,6-dione and its (15R)-epimer, new antimycin analogs"
- CHEMICAL ABSTRACTS, vol. 76, no. 1, 3.Januar 1972 Columbus, Ohio, US; abstract no. 1725, P. P. BATRA ET AL.: "Relation of structure and activity of antimycin A in the induction of carotenoid synthesis in mycobacterium marinum" Seite 169; XP002020735 & J. BIOL. CHEM., Bd. 246, Nr. 23, 1971, Seiten 7125-30,
- JOURNAL OF CHROMATOGRAPHY, Bd. 522, 1990, AMSTERDAM NL, Seiten 179-94, XP000611197 S. L. ABIDI ET AL.: "Liquid chromatography-thermospray mass spectrometric study of N-acylamino dilactones and 4-butyrolactones derived from antimycin A"

## Beschreibung

Die Erfindung betrifft bekannte und neue Acylaminosalicylsäureamide, mehrere Verfahren zu ihrer Herstellung und ihre Verwendung als Schädlingsbekämpfungsmittel, sowie neue Zwischenprodukte und mehrere Verfahren zu deren Herstellung.

Bestimmte Acylaminosalicylsäureamide, wie beispielsweise die Verbindungen 3-Formamido-salicylanilid und 3-(Formylamino)-2-hydroxy-N-(phenylmethyl)-benzamid, sind bereits bekannt (vergleiche z. B. Biochim. Biophys. Acta (1993), 1142(3), 262-8, J. Med. Chem. (1990), 33(1), 136-42 oder J. Biol. Chem. (1971), 246(23), 7125-30), US-3,148,995. Taborsky et al. beschreiben Salicylanilide mit antimikrobischer Wirkung (vgl. J. of Pharm. Sciences, 1963, 52, 542-545. Die Gewinnung von Acylaminosalicylsäure-Derivaten aus Streptomyceten ist ebefalls bereits bekannt (vgl. Chem. Abst., vol. 122, no. 3, 1995, abst. 29879, Chem. Abst., vol. 119, no. 15, 1993, abst. 158366, J. of Chrom. 1990, 522, 179-194). Kinoshita et al. beschreiben Anitmycin-Analoga, die eine fungizide Wirkung gegen Pyricularia oryzae zeigen (Bull. Chem. Soc. of Japan, 1971, 44, 3395-3399). 3-Nitrohalosalicylanilide, die eine Wirkung gegen Pilze zeigen, sind bereits bekannt aus US-3 929 879. 3-Nitrohalosalicylaniliden mit einer keimtötenden Wirkung werden in FR-A-1 501 151 genannt.

Eine Wirkung gegen Schädlinge ist von diesen vorbekannten Verbindungen bisher jedoch nicht beschrieben.

Es wurde nun gefunden, daß die Acylaminosalicylsäureamide der allgemeinen Formel (I), 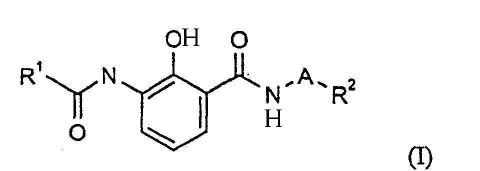 in welcher
- A: für eine Einfachbindung oder für eine Alkylenkette mit 1 bis 6 Kohlenstoffatomen steht,
- R¹: für Wasserstoff, Alkyl oder Alkoxy mit jeweils 1 bis 4 Kohlenstoffatomen steht,
- R²: für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substituiertes Cycloalkyl oder Cycloalkenyl mit 3 bis 12 Kohlenstoffatomen;
für jeweils gegebenenfalls einfach oder mehrfach, gleich oder verschieden substituiertes Aryl mit bis zu 12 Ringgliedern oder für Heterocyclyl mit 3 bis 8 Ringgliedern steht, wobei die möglichen Substituenten aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
substituiertes Aryl, Aryloxy, Arylthio, Arylalkyl, Arylalkyloxy, Arylalkylthio, Aryloxyalkyl, Arylthioalkyl, Heterocyclyl, Heterocyclyloxy, Heterocyclylthio, Heterocyclylalkyl, Heterocyclylalkyloxy oder Heterocyclylalkylthio,
sich zur Bekämpfung von Schädlingen an Pflanzen und technischen Materialien, vorzugsweise Pilzen und Bakterien, eignen.

In den Definitionen sind die Kohlenwasserstoffketten, wie Alkyl, Alkylen, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, wie z.B. Phenyl, Naphthyl, vorzugsweise für Phenyl oder Naphthyl, insbesondere für Phenyl.

Heterocyclyl steht für gesättigte oder ungesättigte, sowie aromatische, ringförmige Verbindungen mit bis zu acht Ringgliedern, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Ebenfalls Gegenstand der vorliegenden Anmeldung sind neue Acylaminosalicylsäurearnide der allgemeinen Formel (I), 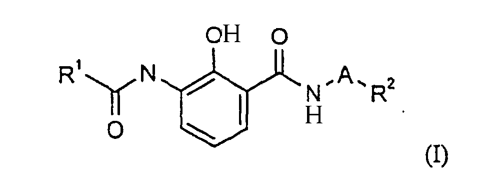 wobei die Substituenten R¹, R² und A wie oben angegeben definiert sind, und die Verbindungen 3-(Formylamino)-2-hydroxy-N-{4-[2,4,6-tris-(1-methylpropyl)-phenoxy]-phenyl}-benzamid, N-{4-[3,5-Bis-(1,1-dimethylethyl)-phenoxy]-phenyl}-3-(formylamino)-2-hydroxy-benzamid, N-{4-[2,4-Bis-(1,1-dimethylethyl)-phenoxy]-phenyl}-3-(formylamino)-2-hydroxy-benzamid, N-{4-[2,6-Bis-(1-methylpropyl)-phenoxy]-phenyl}-3-(formylamino)-2-hydroxybenzamid, 3-(Formylamino)-2-hydroxy-N-{4-[3-(trifluoromethyl)-phenoxy]-phenyl}-benzamid, N-{4-[4-(1,1-dimethylethyl)-phenoxy]-phenyl}-3-(formylamino)-2-hydroxy-benzamid, 3-(Formylamino)-2-hydroxy-N-(4-phenoxyphenyl)-benzamid, N-(4-Butylphenyl)-3-(formylamino)-2-hydroxy-benzamid und N-{3-chloro-4-(4-chlorophenoxy)phenyl}-3-(formylamino)-2-hydroxy-benzamid, 3-(Formylamino)-2-hydroxy-N-(phenylmethyl)-benzamid, 3-Formamidosalicylanilid, 3-(Formylamino)-2-hydroxy-N-(2-phenylethyl)-benzamid und 4'-Chlor-3-acetylaminosalicylanilid ausgenommen sind.

Die Erfindung betrifft insbesondere die Verwendung der Verbindungen, bzw. die neuen Verbindungen der Formel (I), in welcher
- A: für eine Einfachbindung oder für Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- R¹: für Wasserstoff, Methyl, Ethyl, n- oder i-Propyl, n-, i-, s- oder t-Butyl, Methoxy, Ethoxy, n- oder i-Propoxy steht,
- R²: für jeweils gegebenenfalls einfach bis sechsfach durch Fluor, Chlor, Brom, Cyano, Carboxy, Methyl, Ethyl, Methoxy, Ethoxy, n- oder i-Propyl, Methoxycarbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbomyl oder Adamantyl;
oder für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
substituiertes Phenyl, Phenoxy, Phenylalkyl, Phenylthio, Phenoxyalkyl, Phenylthioalkyl, Phenylalkyloxy oder Phenylalkylthio, mit jeweils 1 bis 4 Kohlenstoffatomen in den jeweiligen Alkylketten.

Besonders bevorzugt ist die Verwendung der Verbindungen, bzw. sind die neuen Verbindungen der Formel (I), in welcher
- A: für eine Einfachbindung oder für Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- R¹: für Wasserstoff, Methyl, Ethyl, Methoxy oder Ethoxy steht,
- R²: für jeweils gegebenenfalls einfach bis sechsfach durch Fluor, Chlor, Brom, Cyano, Carboxy, Methyl, Ethyl, Methoxy, Ethoxy, n- oder i-Propyl, Methoxycarbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
substituiertes Phenyl, Phenoxy, Phenylthio, Benzyl, Phenylethyl, Phenylpropyl, Benzyloxy, Benzylthio, Phenoxymethyl, Phenoxyethyl, Phenylthiomethyl oder Phenylthioethyl.

Ganz besonders bevorzugt ist die Verwendung der Verbindungen, bzw. sind die neuen Verbindungen der Formel (I), in welcher
- A: für eine Einfachbindung oder für Methylen, 1,1-Ethylen, 1,2-Ethylen, 1,1-Propylen, 1,2-Propylen oder 2,2-Propylen steht,
- R¹: für Wasserstoff steht,
- R²: für jeweils gegebenenfalls einfach oder zweifach durch Methyl, Ethyl, Methoxy oder Ethoxy substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten aus der nachstehenden Aufzählung ausgewählt sind:
Fluor, Chlor, Brom, Cyano, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl,
Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
substituiertes Phenyl, Phenoxy, Phenylthio, Benzyl, Phenyl-1-ethyl, Phenyl-2-ethyl, Benzyloxy, Benzylthio, Phenoxymethyl oder Phenylthiomethyl.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Weiterhin wurde gefunden, daß man die neuen Acylaminosalicylsäureamide der allgemeinen Formel (I) erhält, wenn man
a) Aminosalicylsäureamide der allgemeinen Formel (II), 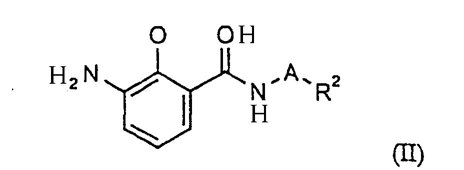 in welcher
   - A und R²: die oben angegebenen Bedeutungen haben,
   mit Acylierungsmitteln der allgemeinen Formel (III), 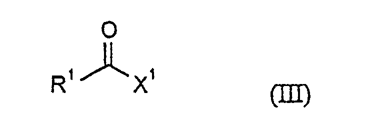 in welcher
   - R¹: die oben angegebene Bedeutung hat und
   - X¹: für Halogen, Hydroxy, Alkoxy oder Alkylcarbonyloxy steht,
   gegebenenfalls in Gegenwart eines Verdünnungsmittels, gegebenenfalls in Gegenwart eines Säureakzeptors, und gegebenenfalls in Gegenwart eines weiteren Reaktionshilfsmittels, umsetzt, oder wenn man
b) Nitrosalicylsäureamide der allgemeinen Formel (IV) 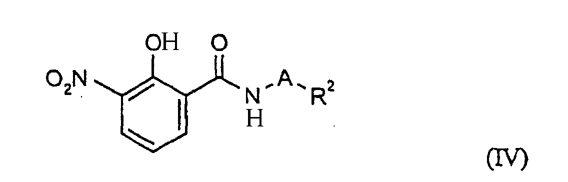 in welcher
   - A und R²: die oben angegebenen Bedeutungen haben,
   mit Ameisensäure, gegebenenfalls in Gegenwart eines Katalysators und gegebenenfalls in Gegenwart eines weiteren Reaktionshilfsmittels, umsetzt, oder wenn man
c) O-Benzyl-nitrosalicylsäureamide der allgemeinen Formel (V), 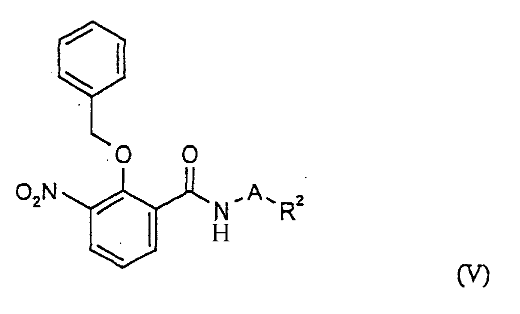 in welcher
   - A und R²: die oben angegebenen Bedeutungen haben,
   mit Ameisensäure, gegebenenfalls in Gegenwart von Wasserstoff oder eines unedlen Metalls, gegebenenfalls in Gegenwart eines Katalysators und gegebenenfalls in Gegenwart eines weiteren Reaktionshilfsmittels, umsetzt.

Die erfindungsgemäßen Verbindungen liegen gegebenenfalls als Mischungen verschiedener möglicher isomerer Formen, insbesondere von Stereoisomeren, wie z.B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beansprucht.

Die zur Durchführung des erfindungsgemäßen Verfahrens a) als Ausgangsstoffe benötigten Aminosalicylsäureamide sind durch die Formel (II) allgemein definiert. In dieser Formel (II) haben A und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und R² angegeben wurden.

Die Ausgangsstoffe der Formel (II) sind mit Ausnahme von 4-{4-[(3-Amino-2-hydroxybenzoyl)-amino]-3-hydroxy-1-piperidyl}-N,N,4-trimethyl-2,2-diphenylbutanamid und 3-Aminosalicylanilid neu und ebenfalls Gegenstand der vorliegenden Anmeldung.

Die Aminosalicylsäureamide der Formel (II) werden erhalten, wenn man (Verfahren a-1a) Nitrosalicylsäureamide der allgemeinen Formel (IV), 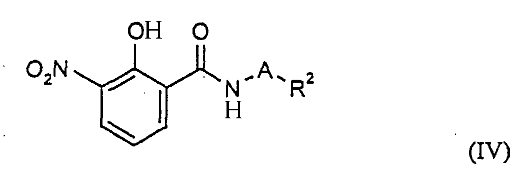 in welcher
- A und R²: die oben angegebenen Bedeutungen haben,
mit Wasserstoff, gegebenenfalls in Gegenwart eines Verdünnungsmittels, vorzugsweise eines Esters wie Essigsäuremethylester oder Essigsäureethylester; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; Wasser, einer Salzlösung, wie beispielsweise Ammoniumchloridlösung, einer Säure, wie beispielsweise Salzsäure oder Essigsäure, sowie beliebigen Mischungen der genannten Verdünnungsmittel und gegebenenfalls in Gegenwart eines Katalysators, wie beispielsweise Raney-Nickel, Palladium oder Platin, gegebenenfalls auf einem Trägermaterial, wie Aktivkohle, umsetzt,
oder wenn man (Verfahren a-1b) O-Benzyl-nitrosalicylsäureamide der allgemeinen Formel (V), 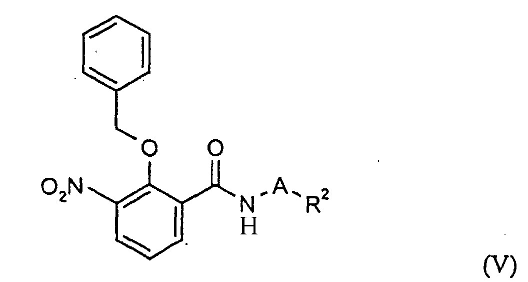 in welcher
- A und R²: die oben angegebenen Bedeutungen haben,
mit Wasserstoff, gegebenenfalls in Gegenwart eines Verdünnungsmittels, vorzugsweise eines Esters wie Essigsäuremethylester oder Essigsäureethylester; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; einer Säure, wie beispielsweise Essigsäure; Wasser; sowie beliebigen Mischungen der genannten Verdünnungsmittel und gegebenenfalls in Gegenwart eines Katalysators, wie beispielsweise Raney-Nickel, Palladium oder Platin, gegebenenfalls auf einem Trägermaterial, wie beispielsweise Aktivkohle, umsetzt.

Die zur Durchführung des erfindungsgemäßen Verfahrens a-1b) als Ausgangsstoffe benötigten O-Benzyl-nitrosalicylsäureamide sind durch die Formel (V), allgemein definiert. In dieser Formel (V) haben A und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und R² angegeben wurden.

Die O-Benzyl-nitrosalicylsäureamide der Formel (V) sind noch nicht bekannt, sie sind als neue Stoffe Gegenstand der vorliegenden Anmeldung.

Die O-Benzyl-nitrosalicylsäureamide der Formel (V) werden erhalten, wenn man (Verfahren a-2) O-Benzyl-nitrosalicylsäurederivate der Formel (VI), 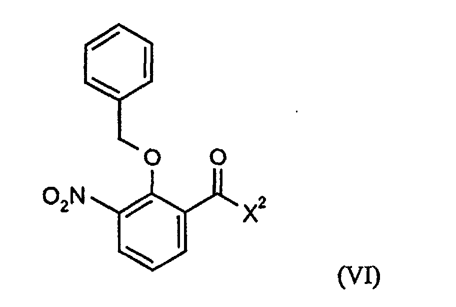 in welcher
- X²: für Halogen, Hydroxy oder Alkoxy steht,
mit einem Amin der Formel (VII),

H₂N-A-R² (VII)

in welcher
- A und R²: die oben angegebenen Bedeutungen haben,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Amids, wie N,N-Dimethylformamid, N,N-Dimethylacetamid, N-Methylformanilid, N-Methylpyrrolidon oder Hexamethylphosphorsäuretriamid; eines Esters wie Essigsäuremethylester oder Essigsäureethylester; eines Sulfoxids, wie Dimethylsulfoxid; oder eines Sulfons, wie Sulfolan, gegebenenfalls in Gegenwart eines Kondensationsmittel, beispielsweise eines Säurehalogenidbildners wie Phosgen, Phosphortribromid, Phosphortrichlorid, Phosphorpentachlorid, Phosphoroxychlorid oder Thionylchlorid; eines Anhydridbildners wie Chlorameisensäureethylester, Chlorameisensäuremethylester, Chlorameisensäureisopropylester, Chlorameisensäureisobutylester oder Methansulfonylchlorid; eines Carbodiimides, wie N,N'-Dicyclohexylcarbodiimid (DCC) oder eines anderen üblichen Kondensationsmittels, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/-Tetrachlorkohlenstoff und gegebenenfalls in Gegenwart eines Säureakzeptors, vorzugsweise eines Erdalkalimetall- oder Alkalimetallhydrides, -hydroxides, -amids, -alkoholates, -acetates, -carbonates oder -hydrogencarbonates, wie beispielsweise Natriumhydrid, Natriumamid, Natrium-methylat, Natrium-ethylat, Kalium-tert.butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), umsetzt.

Die zur Durchführung des erfindungsgemäßen Verfahrens a-2) als Ausgangsstoffe benötigten O-Benzyl-nitrosalicylsäurederivate sind durch die Formel (VI), allgemein definiert. In dieser Formel (VI) steht X² für Halogen, vorzugsweise Chlor, Hydroxy oder Alkoxy, vorzugsweise Methoxy oder Ethoxy.

Die O-Benzyl-nitrosalicylsäurederivate der Formel (VI) sind bekannt und können nach bekannten Methoden hergestellt werden (vergleiche z. B. J. Am. Chem. Soc. 1959, 5215-5217).

Die zur Durchführung des erfindungsgemäßen Verfahrens a-2) weiterhin als Ausgangsstoffe benötigten Amine sind durch die Formel (VII) allgemein definiert. In dieser Formel (VII) haben A und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und R² angegeben wurden.

Die Amine der Formel (VII) sind bekannte Reagentien in der organischen Chemie.

Die zur Durchführung des erfindungsgemäßen Verfahrens a) weiterhin als Ausgangsstoffe benötigten Acylierungsmittel sind durch die Formel (III), allgemein definiert. In dieser Formel (III) hat R¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R¹ angegeben wurde. X¹ steht für Halogen, Hydroxy, Alkoxy oder Alkylcarbonyloxy, vorzugsweise für Chlor, Hydroxy, Methoxy, Ethoxy oder Acetoxy.

Die Acylierungsmittel der allgemeinen Formel (III) sind bekannte Reagenzien in der organischen Chemie.

Die zur Durchführung des erfindungsgemäßen Verfahrens b) als Ausgangsstoffe benötigten Nitrosalicylsäureamide sind durch die Formel (IV) allgemein definiert. In dieser Formel (IV) haben A und R² vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und R² angegeben wurden.

Die Nitrosalicylsäureamide der Formel (IV) sind teilweise bekannt und/oder können nach bekannten Methoden hergestellt werden (vergleiche z. B. Arzneim.-Forsch (1978), 28(9), 1550-3).

Neu, und auch Gegenstand der vorliegenden Anmeldung sind Nitrosalicylsäureamide der Formel (IV-a), 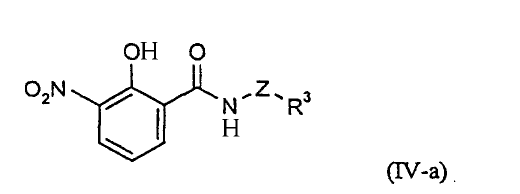 in welcher
- Z: für eine Einfachbindung oder für, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- R³: für jeweils gegebenenfalls einfach bis dreifach durch Fluor, Chlor, Brom, Cyano, Carboxy, Methyl, Ethyl, Methoxy, Ethoxy, n- oder i-Propyl, Methoxycarbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Cyano, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl,
Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis vie rfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
substituiertes Phenyl, Phenoxy, Phenylthio, Benzyl, Phenylethyl, Phenylpropyl, Benzyloxy, Benzylthio, Phenoxymethyl, Phenoxyethyl, Phenylthiomethyl oder Phenylthioethyl.

Ganz besonders bevorzugt sind die neuen Nitrosalicylsäureamide der Formel (IV-a), in welcher
- Z: für eine Einfachbindung oder für 1,1-Ethylen, 1,2-Ethylen, 1,1-Propylen, 1,2-Propylen oder 2,2-Propylen steht,
- R³: für jeweils gegebenenfalls einfach oder zweifach durch Methyl, Ethyl, Methoxy oder Ethoxy substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten aus der nachstehenden Aufzählung ausgewählt sind:
Cyano, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
substituiertes Phenyl, Phenoxy, Phenylthio, Benzyl, Phenyl-1-ethyl, Phenyl-2-ethyl, Benzyloxy, Benzylthio, Phenoxymethyl oder Phenylthiomethyl,

Die Nitrosalicylsäureamide der Formel (IV-a) werden erhalten, Verfahren b-1), wenn man 2-Hydroxy-3-nitrobenzoesäure oder 2-Hydroxy-3-nitrobenzoylchlorid mit einem Amin der Formel (VIII),

H₂N-A-R³ (VIII)

in welcher
- A und R³: die oben angegebenen Bedeutungen haben,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Amids, wie N,N-Dimethylformamid, N,N-Dimethylacetamid, N-Methylformanilid, N-Methylpyrrolidon oder Hexamethylphosphorsäuretriamid; eines Esters wie Essigsäuremethylester oder Essigsäureethylester; eines Sulfoxids, wie Dimethylsulfoxid; oder eines Sulfons, wie Sulfolan, gegebenenfalls in Gegenwart eines Kondensationsmittel, beispielsweise eines Säurehalogenidbildners wie Phosgen, Phosphortribromid, Phosphortrichlorid, Phosphorpentachlorid, Phosphoroxychlorid oder Thionylchlorid; eines Anhydridbildners wie Chlorameisensäureethylester, Chlorameisensäuremethylester, Chlorameisensäureisopropylester, Chlorameisensäureisobutylester oder Methansulfonylchlorid; eines Carbodiimides, wie N,N'-Dicyclohexylcarbodiimid (DCC) oder eines anderen üblichen Kondensationsmittels, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff und gegebenenfalls in Gegenwart eines Säureakzeptors, vorzugsweise eines Erdalkalimetall- oder Alkalimetallhydrides, -hydroxides, -amids, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), umsetzt.

Ferner wurde gefunden, daß die neuen Nitrosalicylsäureamide der Formel (IV-a) sich zur Bekämpfung von Schädlingen an Pflanzen und technischen Materialien, vorzugsweise Pilzen, Insekten und Bakterien, eignen.

Die zur Durchführung des erfindungsgemäßen Verfahrens b-1) als Ausgangsstoffe benötigte 2-Hydroxy-3-nitrobenzoesäure oder 2-Hydroxy-3-nitrobenzoesäurechlorid sind bekannt (vergleiche z. B. J.Chem.Soc., 1953, 2049-2050 oder US-Patent 3,527,865).

Die zur Durchführung des erfindungsgemäßen Verfahrens b-1) weiterhin als Ausgangsstoffe benötigten Amine sind durch die Formel (VIII) allgemein definiert. In dieser Formel (VIII) haben A und R³ vorzugsweise bzw. insbesondere diejenigen Bedeutungen, die bereits im Zusammenhang mit der Beschreibung der erfindungsgemäßen Verbindungen der Formel (IV-a) als bevorzugt bzw. als insbesondere bevorzugt für A und R³ angegeben wurden.

Die Amine der Formel (VIII) sind bekannte Reagentien in der organischen Chemie.

Die zur Durchführung des erfindungsgemäßen Verfahrens c) als Ausgangsstoffe benötigten O-Benzyl-nitrosalicylsäureamide der Formel (V) sind bereits weiter oben im Zusammenhang mit der Beschreibung des erfindungsgemäßen Verfahrens a-1b) beschrieben worden.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b) und c) werden gegebenenfalls in Gegenwart eines Katalysators durchgeführt. Als solche kommen alle Katalysatoren infrage, die auch für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Das erfindungsgemäße Verfahren c) wird gegebenenfalls auch in Gegenwart von Wasserstoff oder gegebenenfalls in Gegenwart eines unedlen Metalls durchgeführt. Als unedle Metalle seien beispielhaft genannt: Zink, Zinn, Eisen, Aluminium oder Magnesium.

Als weitere Reaktionshilfsmittel zur Durchführung der erfindungsgemäßen Verfahren a), b) und c) kommen alle wasserentziehenden Mittel, insbesondere Essigsäureanhydrid, infrage.

Die Reaktionstemperaturen können bei der Durchführung der erfindungsgemäßen Verfahrens a), b) und c) in einem größeren Bereich variiert werden. Im allgemeinen arbeitet man bei Temperaturen von 0°C bis 180°C, vorzugsweise bei Temperaturen von 0°C bis 130°C.

Zur Durchführung des erfindungsgemäßen Verfahrens a) zur Herstellung der Verbindungen der Formel (I) setzt man pro Mol des Aminosalicylsäureamids der Formel (II) im allgemeinen 1 bis 2000 Mol, vorzugsweise 1 bis 800 Mol Acylierungsmittel der Formel (III) ein.

Zur Durchführung der erfindungsgemäßen Verfahren b) und c) zur Herstellung der Verbindungen der Formel (I) setzt man pro Mol des Nitrosalicylsäureamides der Formel (IV), bzw. des O-Benzyl-nitrosalicylsäureamides der Formel (V) im allgemeinen 100 bis 2000 Mol, vorzugsweise 200 bis 1000 Mol Ameisensäure ein.

Die erfindungsgemäßen Verfahren a), b) und c) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Sphaerotheca- und Venturia-Arten, zur Bekämpfung von Getreidekrankheiten, wie beispielsweise gegen Pseudocercosporella-Arten, oder zur Bekämpfung von Reiskrankheiten, wie beispielsweise gegen Pyricularia oryzae, eingesetzt. Mit gutem Erfolg werden auch weitere Getreidekrankheiten, wie Septoria-, Cochliobolus- und Pyrenophora-Arten, bekämpft. Mit gutem Erfolg werden auch weitere Krankheiten im Wein-, Obst- und Gemüsebau, wie Phytophthora, Plasmopara, Podosphaera und Botrytis bekämpft.

Die Wirkstoffe werden in Abhängigkeit von ihren jeweiligen physikalischen und/oder chemischen Eigenschaften in die üblichen Formulierungen übergeführt, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kaltund Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfosufen(IBP), Iprodione,Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Boerdeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozcen(PCNB),
Schwefel und Schwefel-Zubereitungen,
Tebuconazol, Tecloftalam, Tecnhazen, Tetcyclacis, Tetraconazol, Thiabendazol, Thicyofen, Thifluzamide, Tthiophanate-methyl, Thiram, Tioxymid, Tolclofos-methyl, Tolylfluanid, Triadimefon, Triadimenol, Triazbutanil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
2',6'-Dibrom-2-methyl-4'-trifluormethoxy-4'-trifluor-methyl-1,3-thiazol-5-carboxanilid,
2,6-Dichlor-N-(4-trifluormethylbenzyl)-benzamid,
2-Aminobutan,
2-Phenylphenol(OPP),
8-Hydroxychinolinsulfat,
cis-1-(4-Chlorphenyl)-2-(1H-1,2,4-triazol-1-yl)-cycloheptanol,
(5RS,6RS)-6-Hydroxy-2,2,7,7-tetramethyl-5-(1H-1,2,4-triazol-1-yl)-3-octanon,
α-(2,4-Dichlorphenyl)-β-methoxy-α-methyl-1H-1,2,4-triazol-1-ethanol,
α-(1,1-Dimethylethyl)-β-(2-phenoxyethyl)-1H-1,2,4-triazol-1-ethanol,
1-[1-[2-[(2,4-Dichlorphenyl)methoxy]phenyl]ethenyl]-1H-imidazol,
bis-(1-Methylethyl)-3-methyl-4-[(3-methylbenzoyl)oxy]-2,5-thiophendicarboxylat,
2,6-Dichlor-N-[[4-(trifluormethyl)phenyl]methyl]-benzamid,
(E)-α-(Methoxyimino)-N-methyl-2-phenoxy-phenylacetamid,
9H-Xanthen-9-carbonsäure-2-[(phenylamino)-carbonyl]-hydrazid,
O-Methyl-S-phenyl-phenylpropylphosphoramidothioate,
N-(5-Chlor-2-methylphenyl)-2-methoxy-N-(2-.oxo-3-oxazolidinyl)-acetamid,
1-(2,4-Dichlorphenyl)-2-(1H-1,2,4-triazol-1-yl)ethanon-O-(phenylmethyl)-oxim,
N-(2,6-Dimethylphenyl)-2-methoxy-N-(tetrahydro-2-oxo-3-thienyl)-acetamid,
cis-4-[3-[4-(1,1-Dimethylpropyl)-phenyl-2-methylpropyl]-2,6-dimethyl-morpholinhydrochlorid,
1-(3,5-Dichlorphenyl)-3-(2-propenyl)-2,5-pyrrolidindion,
1-Methyl-5-nonyl-2-(phenylmethyl)-3-pyrrolidinol,
1-[[2-(4-Chlorphenyl)-3-phenyloxiranyl]-methyl]-1H-1,2,4-triazol,
Methantetrathiol, -Natriumsalz,
2-(2,3,3-Triiod-2-propenyl)-2H-tetrazol,
N-(2,3-Dichlor-4-hydroxyphenyl)-1-methyl-cyclohexancarboxamid.
N-[3-Chlor-4,5-bis(2-propinyloxy)-phenyl]-N'-methoxy-methanimidamid,
α-(5-Methyl-1,3-dioxan-5-yl)-β-[[4-(trifluormethyl)-phenyl]-methylen]-1H-1,2,4-triazol-1-ethanol,
2,2-Dichlor-N-[1-(4-chlorphenyl)ethyl]-1-ethyl-3-methyl-cyclopropancarboxamid,
1-(2-Methyl-1-naphthalenyl)-1H-pyrrol-2,5-dion,
N-(2,6-Dimethylphenyl)-2-methoxy-N-(tetrahydro-2-oxo-3-furanyl)-acetamid,
3,4-Dichlor-1-[4-(difluormethoxy)-phenyl]-1H-pyrrol-2,5-dion,
8-(1,1-Dimethylethyl)-N-ethyl-N-propyl-1,4-dioxaspiro[4.5]decan-2-methanamin,
N-[2,2,2-Trichlor-1-[(chloracetyl)-amino]-ethyl]-benzamid,
N-Formyl-N-hydroxy-DL-alanin, -Mono-Natriumsalz,
4-Chlor-2-cyan-N,N-dimethyl-5-(4-methylphenyl)-1H-imidazol-1-sulfonamid,
N-(4-Cyclohexylphenyl)-1,4,5,6-tetrahydro-2-pyrimidinamin,
4-Methyl-tetrazolo[1,5-a]quinazolin-5(4H)-onm,2-Chlor-N-(2,6-dimethylphenyl)-N-(isothiocyanatomethyl)-acetamid,
Ethyl-[(4-chlorphenyl)-azo]-cyanoacetat,
3,5-Dichlor-N-[cyan[(1-methyl-2-propynyl)oxy]methyl]-benzamid,
N-(4-Hexylphenyl)-1,4,5,6-tetrahydro-2-pyrimidinamin,
N-(2-Chlor-4-nitrophenyl)-4-methyl-3-nitro-benzolsulfonamid,
N-(6-Methoxy)-3-pyridinyl)-cyclopropancarboxamid,
Methyl-N-(chloracetyl)-N-(2,6-dimethylphenyl)-DL-alaninat,
3-[2-(4-Chlorphenyl)-5-ethoxy-3-isoxazolidinyl]-pyridin,
S-Methyl-1,2,3-benzothiadiazol-7-carbothioat,
2-[(1-Methylethyl)sulfonyl]-5-(trichlormethyl)-1,3,4-thiadiazol,
spiro[2H]-1-Benzopyran-2,1'(3'H)-isobenzofuran]-3'-on,
[2-Methyl-1-[[[1-(4-methylphenyl)ethyl]amino]carbonyl]-propyl]-carbaminsäure-1-methylethylester,
Methyl-N-(2,6-dimethylphenyl)-N-(5-isoxazolylcarbonyl)-DL-alaninat,
Kaliumhydrogencarbonat,
1-[[2-(2,4-Dichlorphenyl)-1,3-dioxolan-2-yl]-methyl]-1H-imidazol,
1-[(Diiodmethyl)-sulfonyl]-4-methyl-benzol,
2-Brom-2-(brommethyl)-pentandinitril,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
Methyl-1-(2,3-dihydro-2,2-dimethyl-1H-inden-1-yl)-1H-imidazol-5-carboxylat,
2-Chlor-N-(2,3-dihydro-1,1,3-trimethyl-1H-inden-4-yl)-3-pyridincarboxamid,
O,O-Diethyl-[2-(dipropylamino)-2-oxoethyl]-ethylphosphoramidothioat,
α-(2,4-Dichlorphenyl)-β-fluor-β-propyl-1H-1,2,4-triazol-1-ethanol,
3-(1,1-Dimethylpropyl-1-oxo-1H-inden-2-carbonitril,
2,6-Dichlor-5-(methylthio)-4-pyrimidinyl-thiocyanat.

Auch eine Mischung mit anderen bekannten Wirkstoffen, wie Herbiziden oder mit Düngemitteln und Wachstumsregulatoren ist möglich.

Die erfindungsgemäßen Wirkstoffe werden als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verschäumen, Bestreichen usw.. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.
Bei der Saatgutbehandlung werden im allgemeinen Wirkstoffmengen von 0,001 bis 50 g je Kilogramm Saatgut, vorzugsweise 0,01 bis 10 g benötigt.
Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Wirkstoffe eignen sich bei guter Pflanzenverträglichkeit und günstiger Warmblütertoxizität auch zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofinannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch ihre Wirkung gegen die Larven des Meerrettichblattkäfers (Phaedon cochleariae), die Raupen der Kohlschabe (Plutella maculipennis), die Raupen des Eulenfalters (spodoptera frugiperda), sowie gegen die grüne Reiszikade (Nephotettix cincticeps) aus.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Naturund synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoflkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufinilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus
Hautflügler wie
Sirex juvencus, Urocerus gigas, Urocerus gigas taignus, Urocerus augur
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Herstellung und die Verwendung der erfindungsgemäßen Wirkstoffe gehen aus den nachfolgenden Beispielen hervor.

### Herstellungsbeispiele

### Beispiel 1:

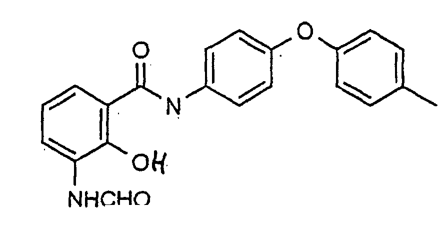

### Verfahren a)

1,67 g (0,005 Mol) 3-(Amino)-2-hydroxy-N-{4-[4-(methyl)-phenoxy]-phenyl}-benzamid werden in 100 ml Ameisensäure 24 Stunden unter Rückfluß zum Sieden erhitzt. Das Lösungsmittel wird bei vermindertem Druck vollständig abdestilliert. Man erhält 0,72 g (40 % der Theorie) 3-(Formylamino)-2-hydroxy-N-{4-[4-(-methyl)-phenoxy]-phenyl}-benzamid als Öl.
¹H-NMR:(CDCl₃/TMS): δ = 6,80 - 7,85 (m, 11H) ppm

### Herstellung der Ausgangsverbindung

### Beispiel (II-1):

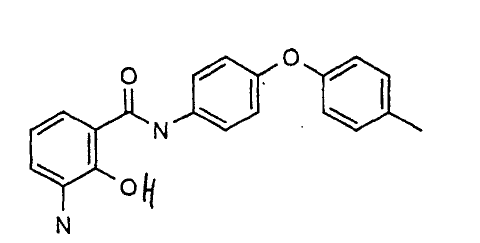

### Verfahren a-1a)

Eine Lösung von 1,3 g (0,0036 Mol) 3-Nitro-2-hydroxy-N-[4-(4-methylphenoxy)-phenyl]-benzamid in 8 ml Methanol wird mit 0,1 g Raney-Nickel versetzt und bei 20°C bei einem Druck von 3 bis 9 bar mit Wasserstoff in einem Autoklaven hydriert. Ungelöste Bestandteile der Mischung werden abfiltriert und das Filtrat bis zur Trockne bei vermindertem Druck eingeengt. Man erhält 0,67 g (56 % der Theorie) 3-Amino-2-hydroxy-N-[4-(4-methylphenoxy)-phenyl]-benzamid.
¹H-NMR:(CDCl₃/TMS): δ = 6,73 - 7,99 (m, 11H) ppm

### Beispiel 2:

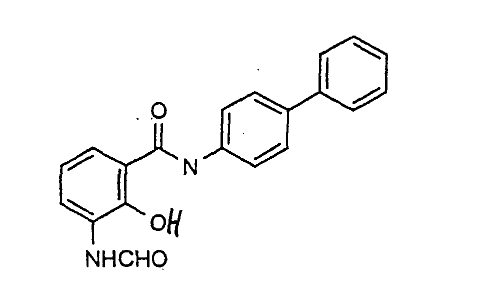

### Verfahren b)

3 g (0,0099 Mol) 3-Nitro-2-hydroxy-N-(4-phenylphenyl)-benzoesäureamid werden mit 0,4 g 10% Palladium auf Kohle und 10 ml Wasser versetzt. Anschließend tropft man 19 ml Ameisensäure zu und erwärmt das Gemisch 2 Stunden auf 110°C. Nach dem Abkühlen wird der Katalysator abgesaugt und der Rückstand mit Wasser nachgewaschen. Das Filtrat wird mit Dichlormethan extrahiert, die organische Phase abgetrennt, über Natriumsulfat getrocknet und das Lösungsmittel bei vermindertem Druck abdestilliert. Man erhält 1,4 g (48 % der Theorie) 3-Formamido-2-hydroxy-N-(4-phenylphenyl)-benzoesäureamid vom Schmelzpunkt 190°C.

### Herstellung der Ausgangsverbindung

### Beispiel IVa-1

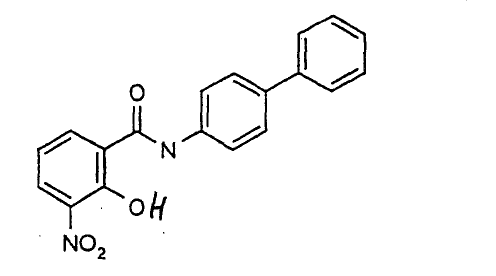

### Verfahren b-1)

15 g (0,08 Mol) 3-Nitrosalicylsäure, 13,8 g (0,08 Mol) 4-Aminobiphenyl und 18,5 g (0,08 Mol) Bicyclohexylcarbodiimid werden in 400 ml Pyridin vorgelegt und Stunden unter rühren auf 90°C erhitzt. Nach Abkühlen, Filtrieren und Einengen des Gemisches wird mit 200 ml 10 %iger Salzsäure versetzt und mit 200 ml Dichlormethan extrahiert. Die vereinigten organischen Phasen werden über Natriumsulfat getrocknet und im Vakuum vom Lösungsmittel befreit. Der Rückstand wird aus Toluol umkristallisiert.

Man erhält 16 g (60 % der Theorie) 3-Nitro-2-hydroxy-N-(4-phenyl-phenyl)-benzoesäureamid als gelbe Kristalle mit einem Schmelzpunkt von 152°C.

### Beispiel 3:

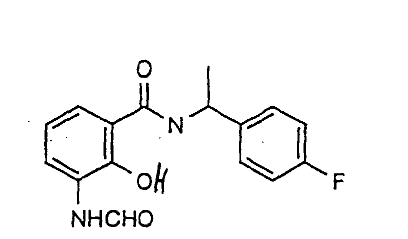

### Verfahren c)

2,4 g (0,006 Mol) 3-Nitro-2-benzyloxy-N-[1-(4-fluorphenyl)-ethyl)-benzoesäureamid werden mit 0,6 g 10% Palladium auf Kohle und 8 ml Wasser versetzt. Zu dieser Mischung gibt man nacheinender 15 ml Ameisensäure und 0,9 g Zinn-Pulver und erhitzt anschließend 5 Stunden unter Rückfluß zum Sieden. Danach werden die festen Bestandteile abgesaugt, und das Filtrat mit Dichlormethan extrahiert. Die organische Phase wird über Natriumsulfat getrocknet und bei vermindertem Druck eingeengt. Man erhält 1,1 g (61 % der Theorie) 3-Formamido-2-hydroxy-N-[1-(4-fluorphenyl)-ethyl)-benzoesäureamid als Öl.
¹H-NMR:(CDCl₃/TMS): δ = 1,62 (d, 3H) ppm

### Herstellung der Ausgangsverbindung

### Beispiel (V-1):

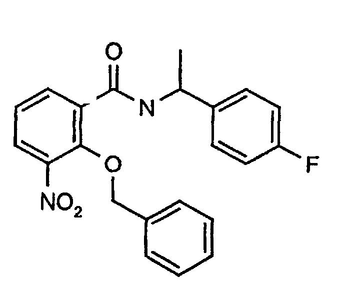

### Verfahren a-2)

Zu einer Lösung von 3 g (0,011 Mol) 3-Nitro-2-benzyloxybenzoesäure in 40 ml Dichlormethan gibt man bei -10°C 2,4 g (0,011 Mol) Triethylamin. Nach 5 Minuten Rühren werden bei -10°C 2,8 g (0,011 Mol) Chlorameisensäureisobutylester zugetropft und weitere 30 Minuten gerührt. Dann gibt man bei -10°C eine Lösung von 1,53 g (0,011 Mol) 1-(4-Fluorphenyl)-ethylamin in 10 ml Dichlormethan zu und rührt ohne weitere Kühlung 12 Stunden nach. Die Mischung wird mit Natriumhydrogencarbonat-Lösung versetzt, die organische Phase abgetrennt, mit Waser gewaschen und über Natriumsulfat getrocknet. Nach dem Abdestillieren des Lösungsmittels erhält man 2,5 g (56% der Theorie) 3-Nitro-2-benzyloxy-N-[1-(4-fluorphenyl)-ethyl)-benzoesäureamid als Öl, ¹H-NMR:(CDCl₃/TMS): δ = 1,34 (d, 3H) ppm

Analog den Herstellungsbeispielen 1 bis 3 sowie entsprechend der allgemeinen Beschreibung der erfindungsgemäßen Verfahren werden die in der nachstehenden Tabelle 1 aufgeführten Verbindungen der allgemeinen Formel (I) erhalten:

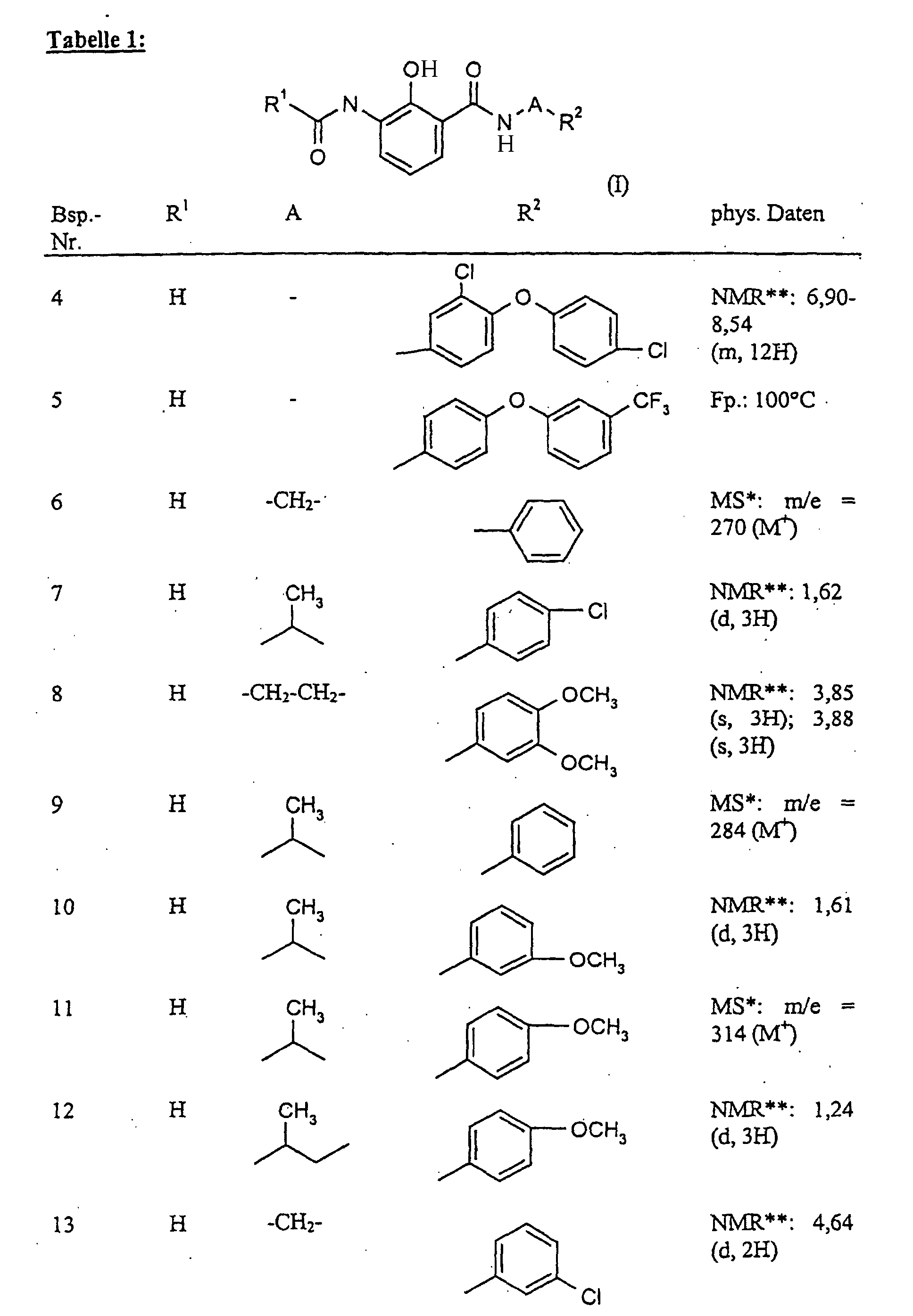

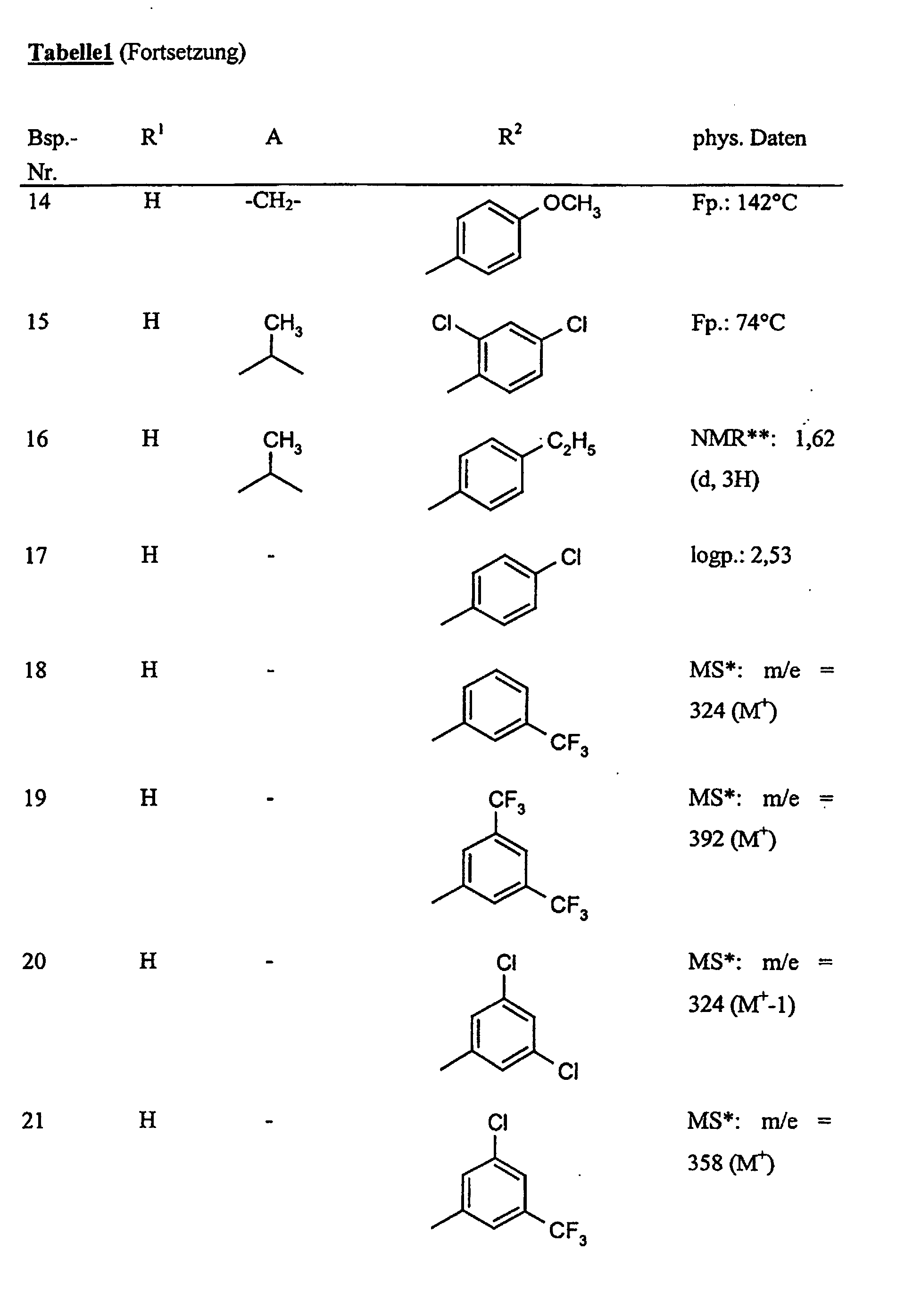

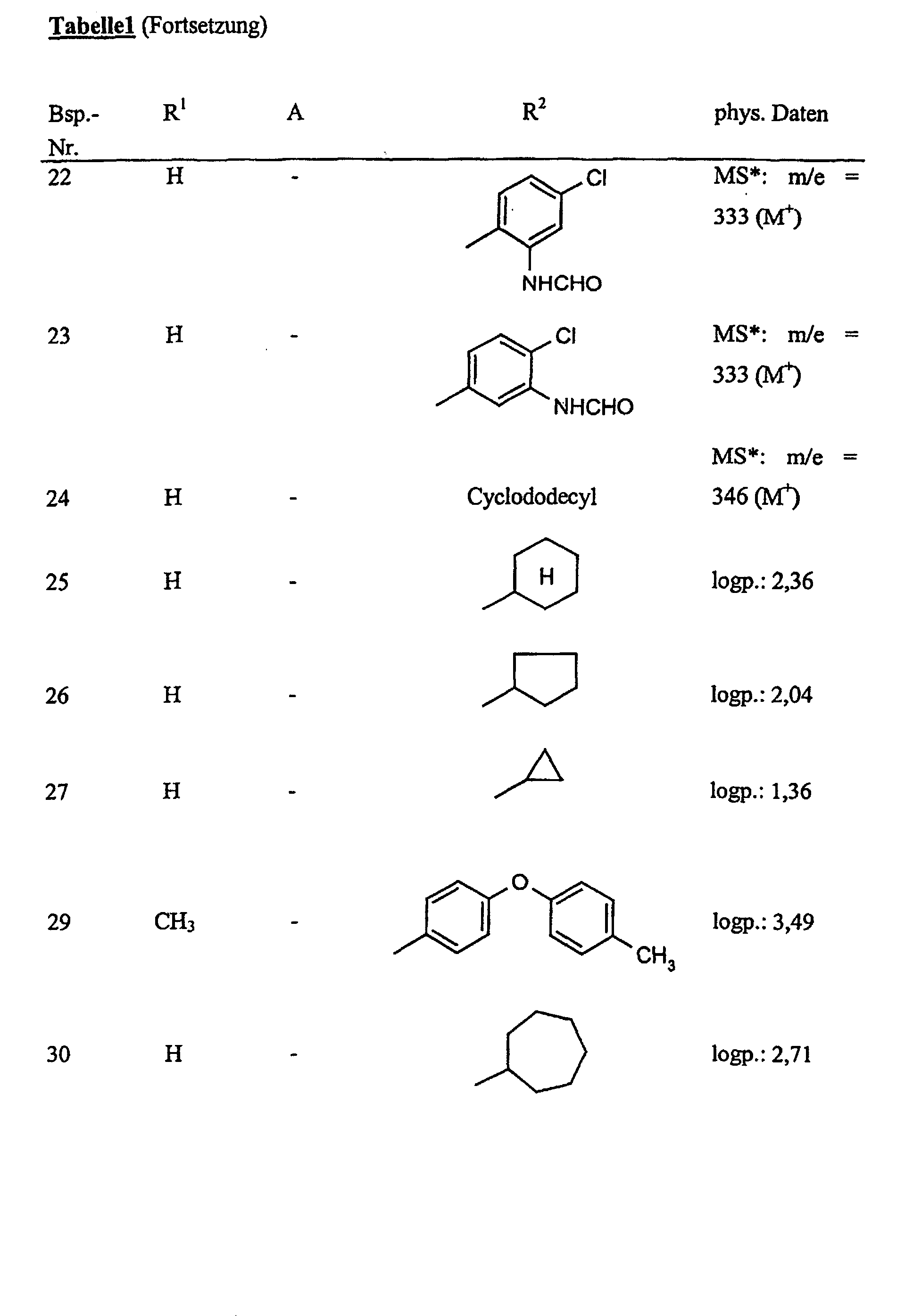

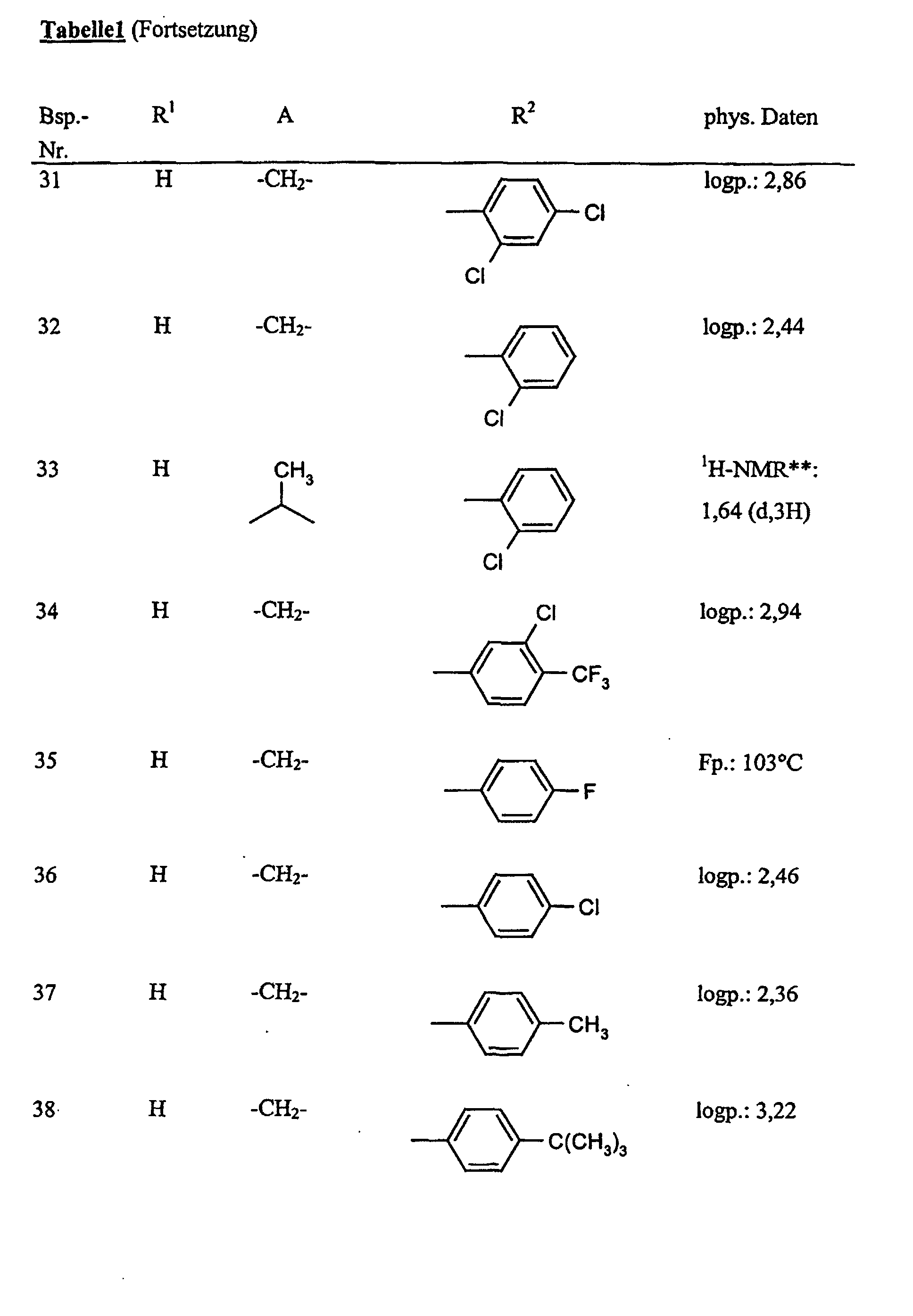

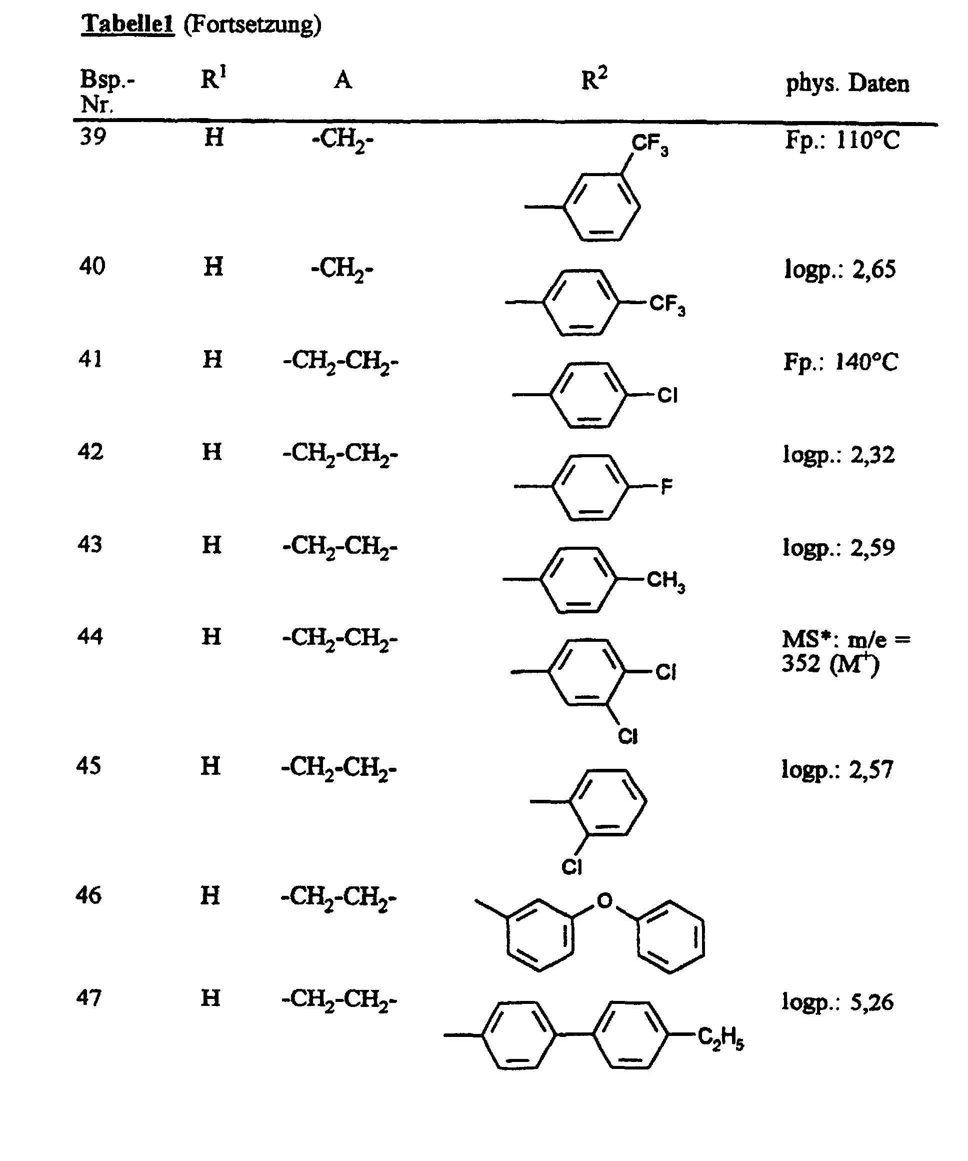

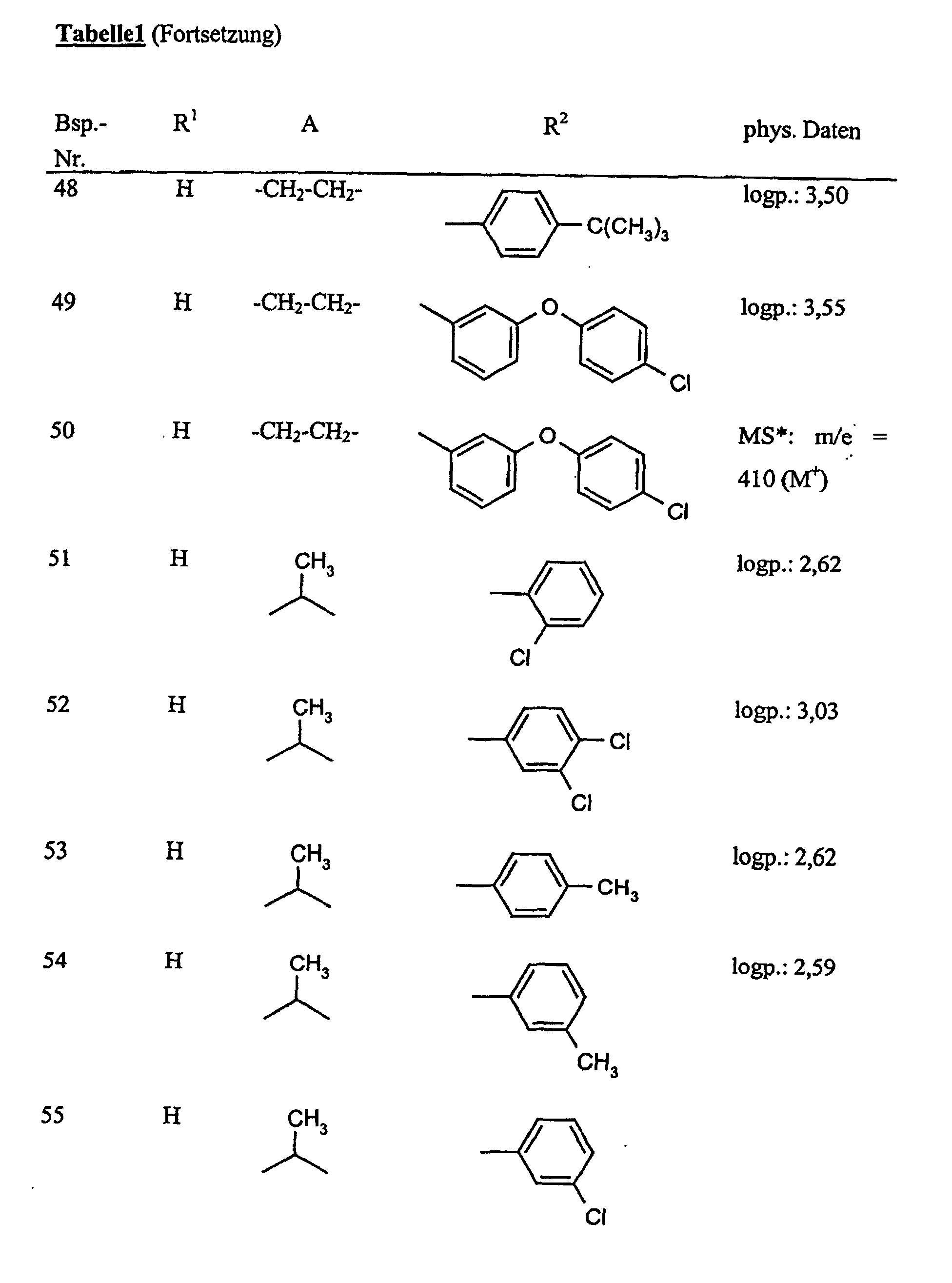

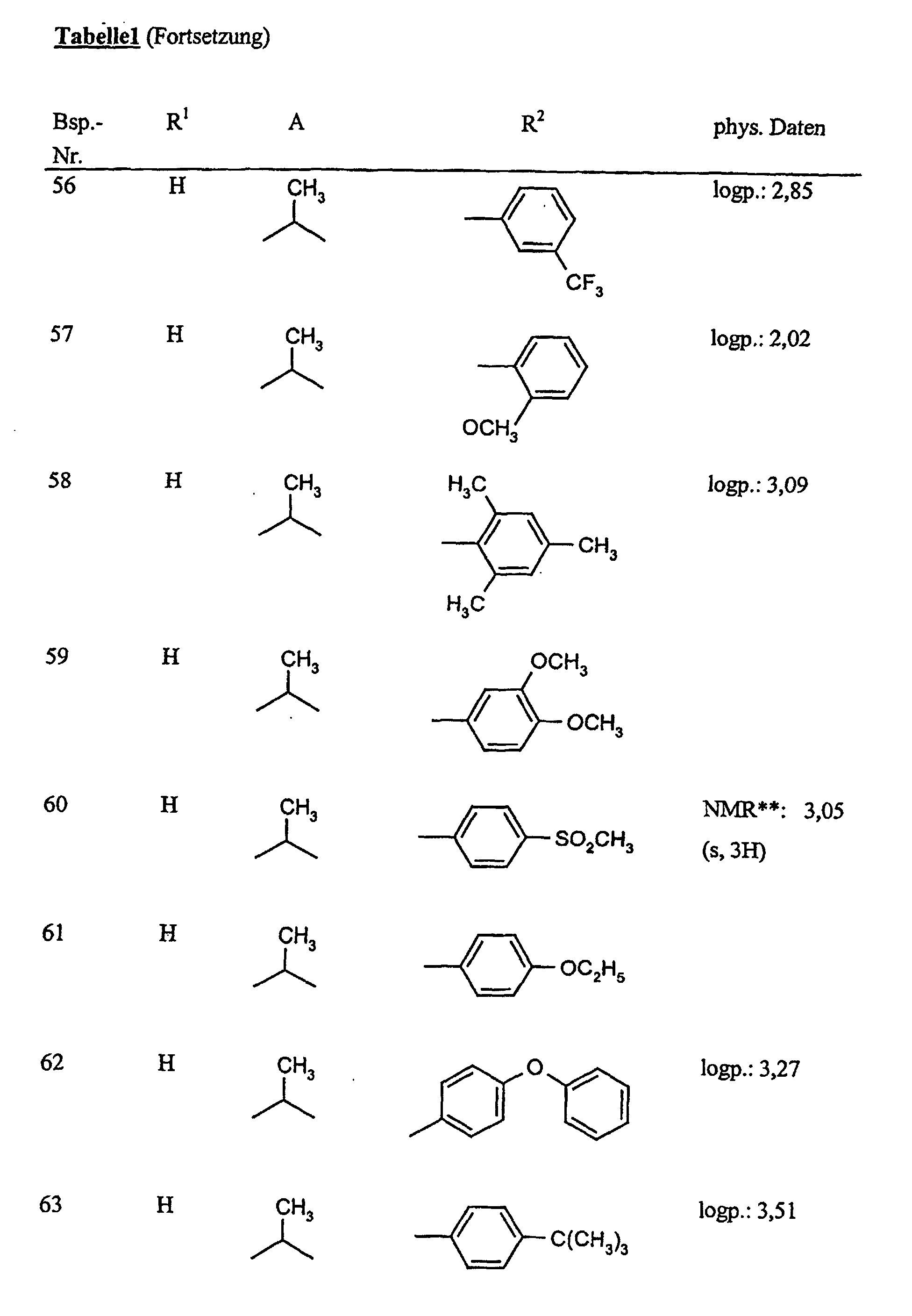

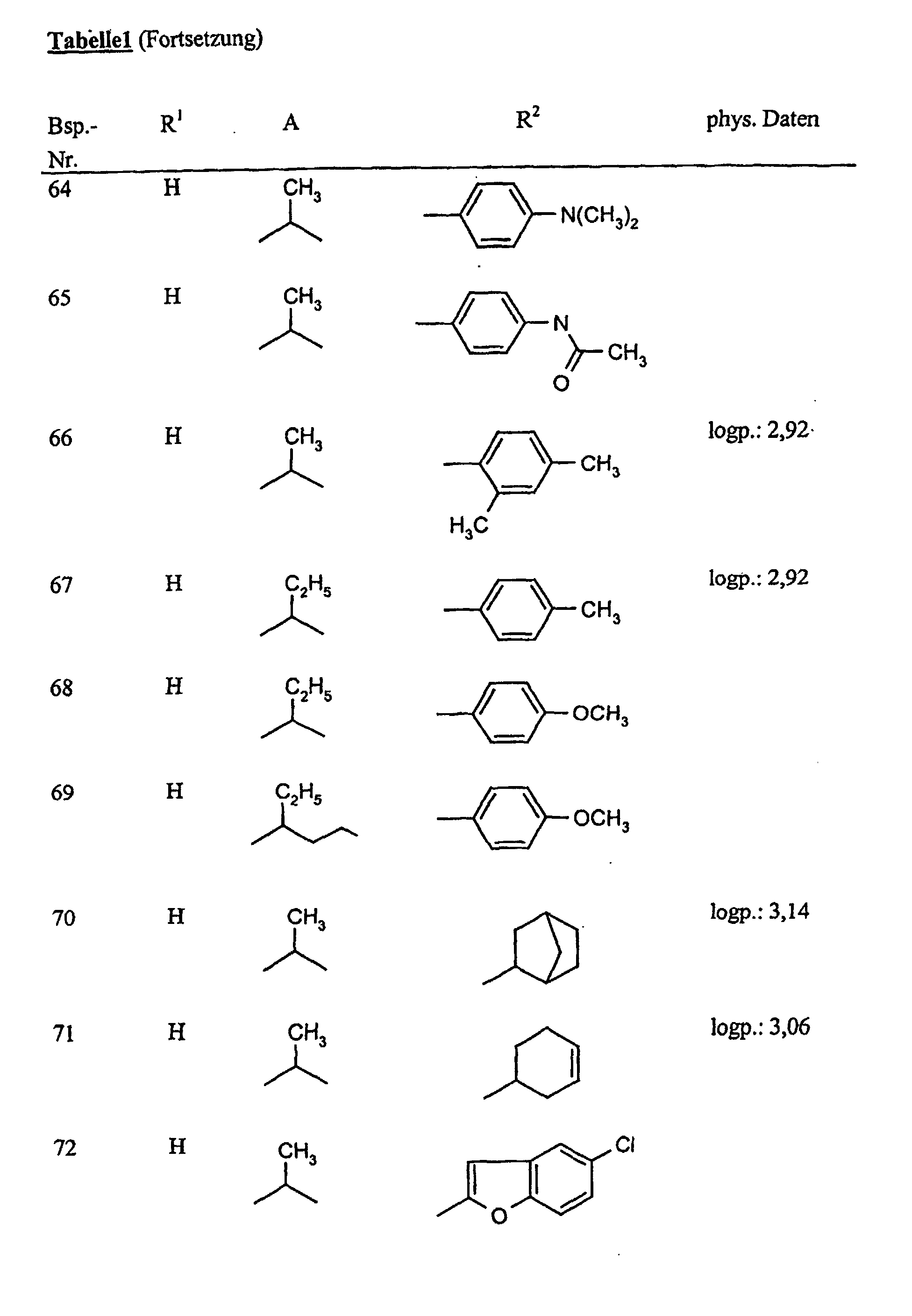

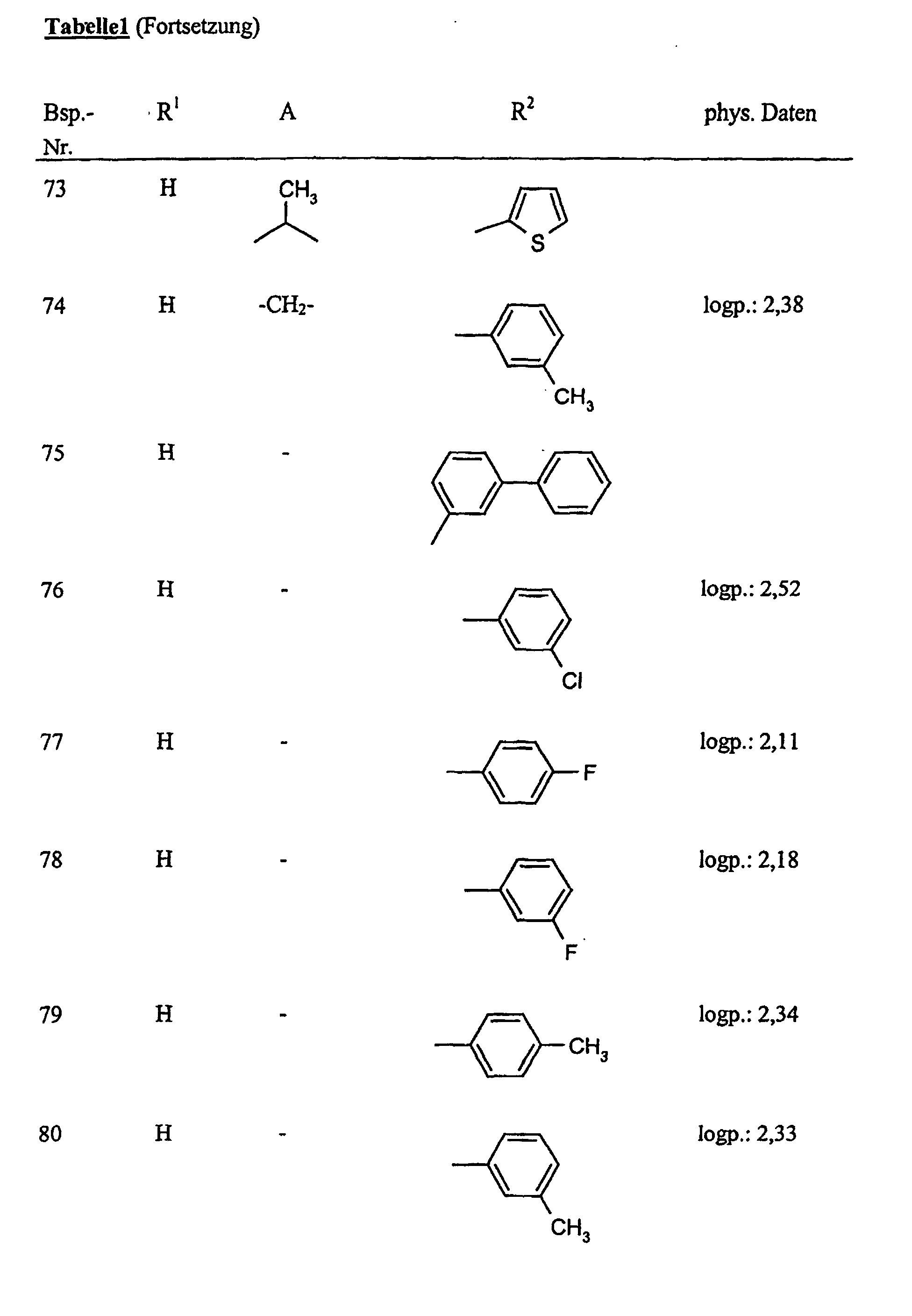

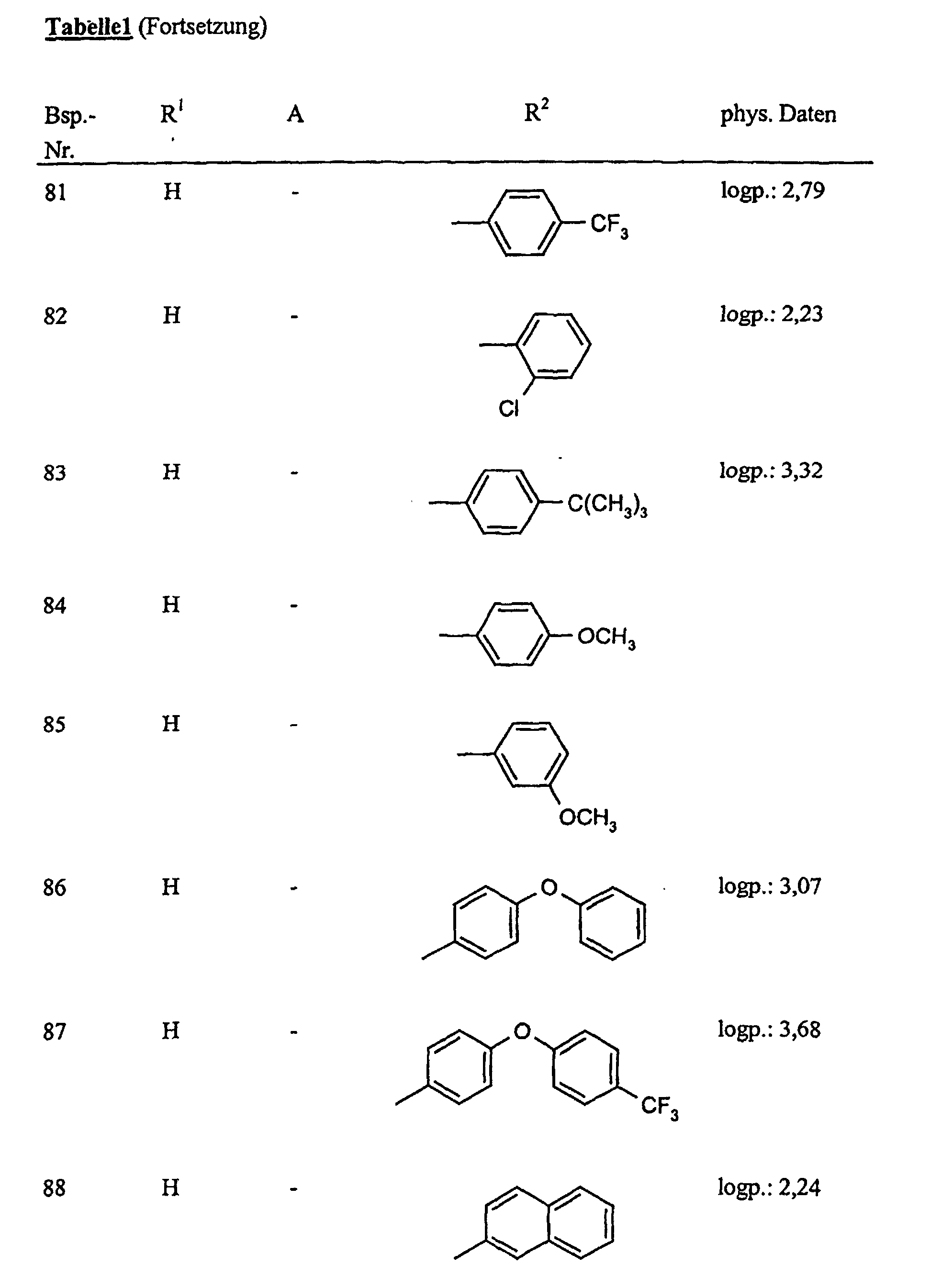

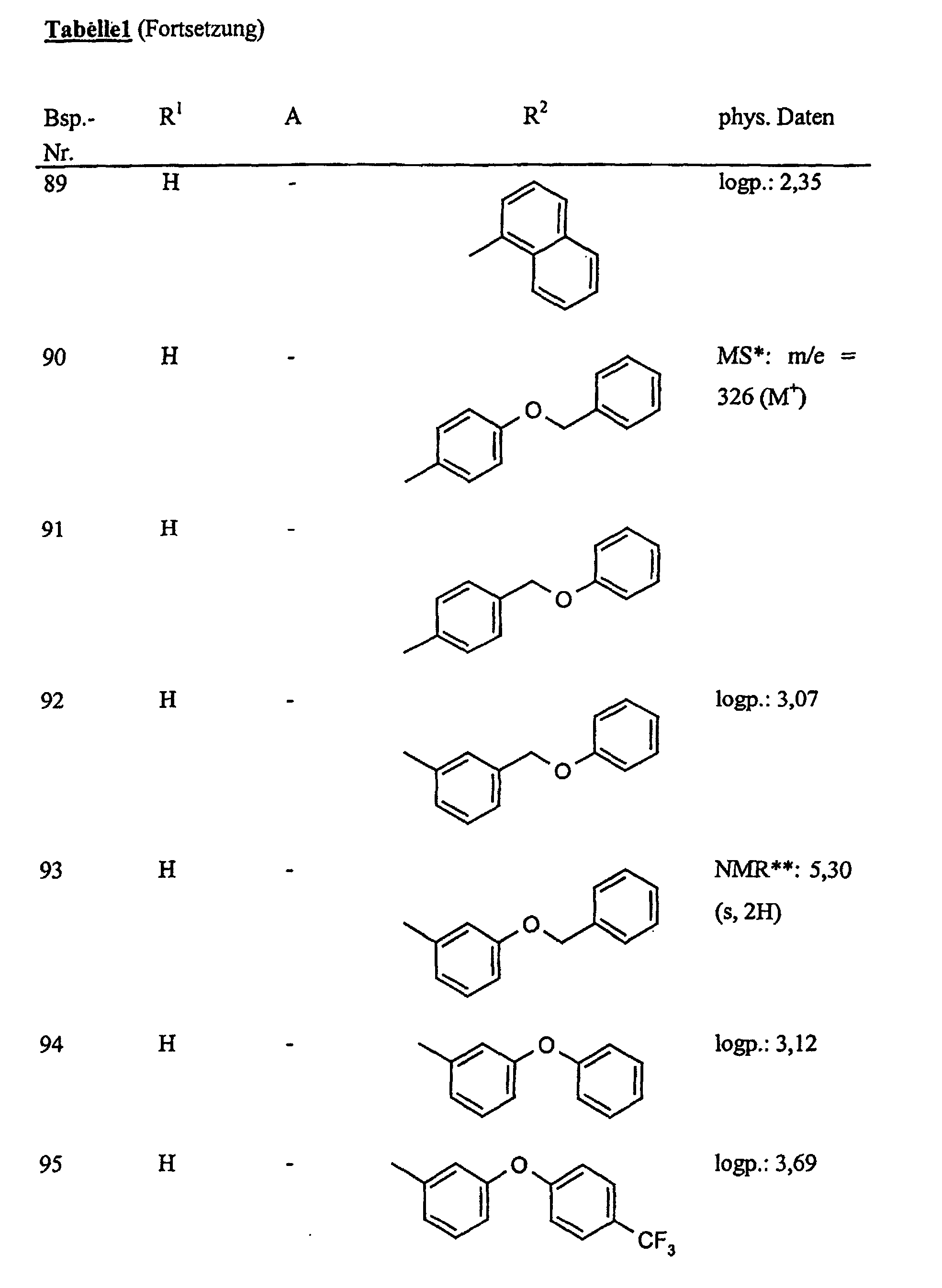

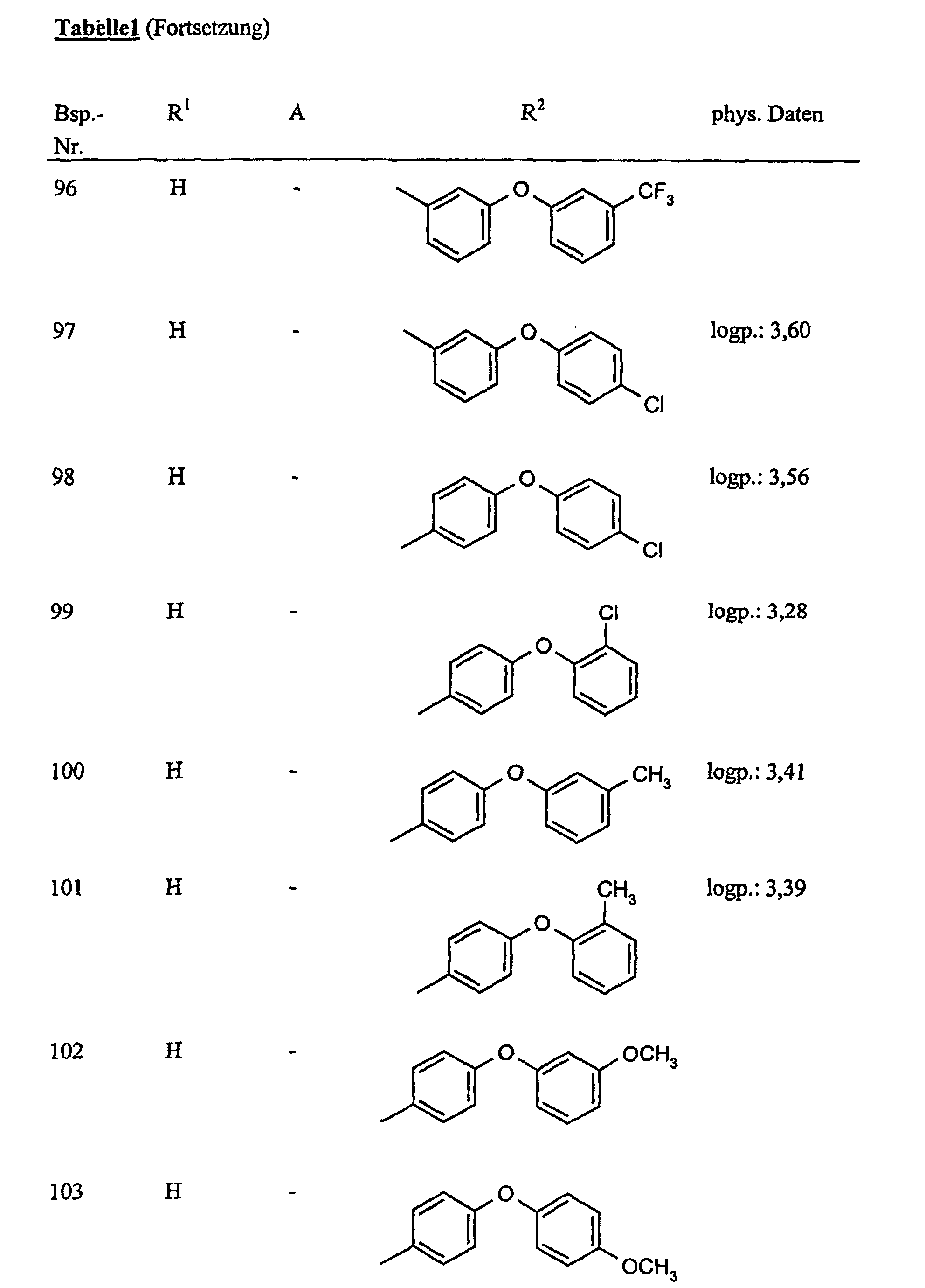

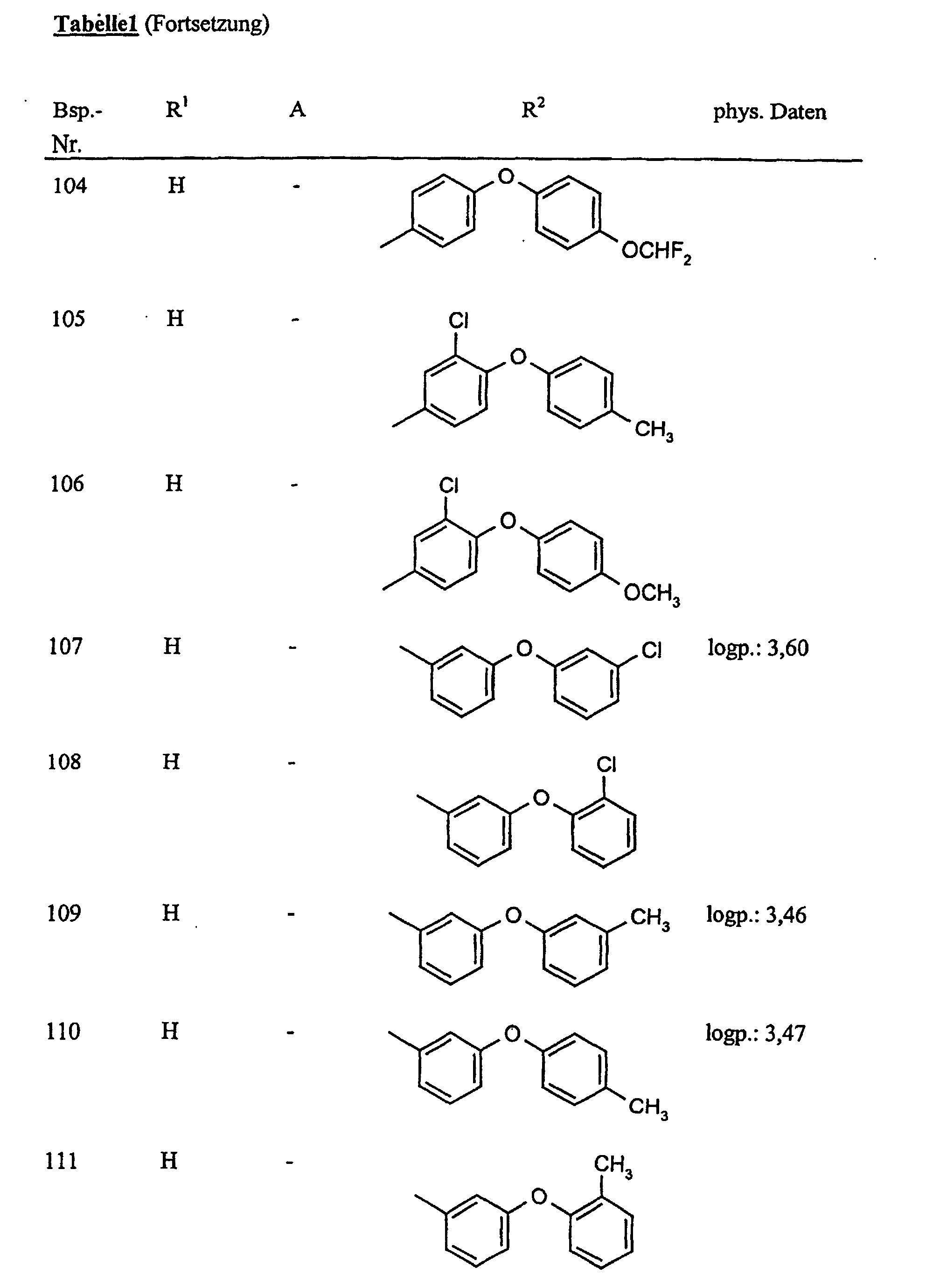

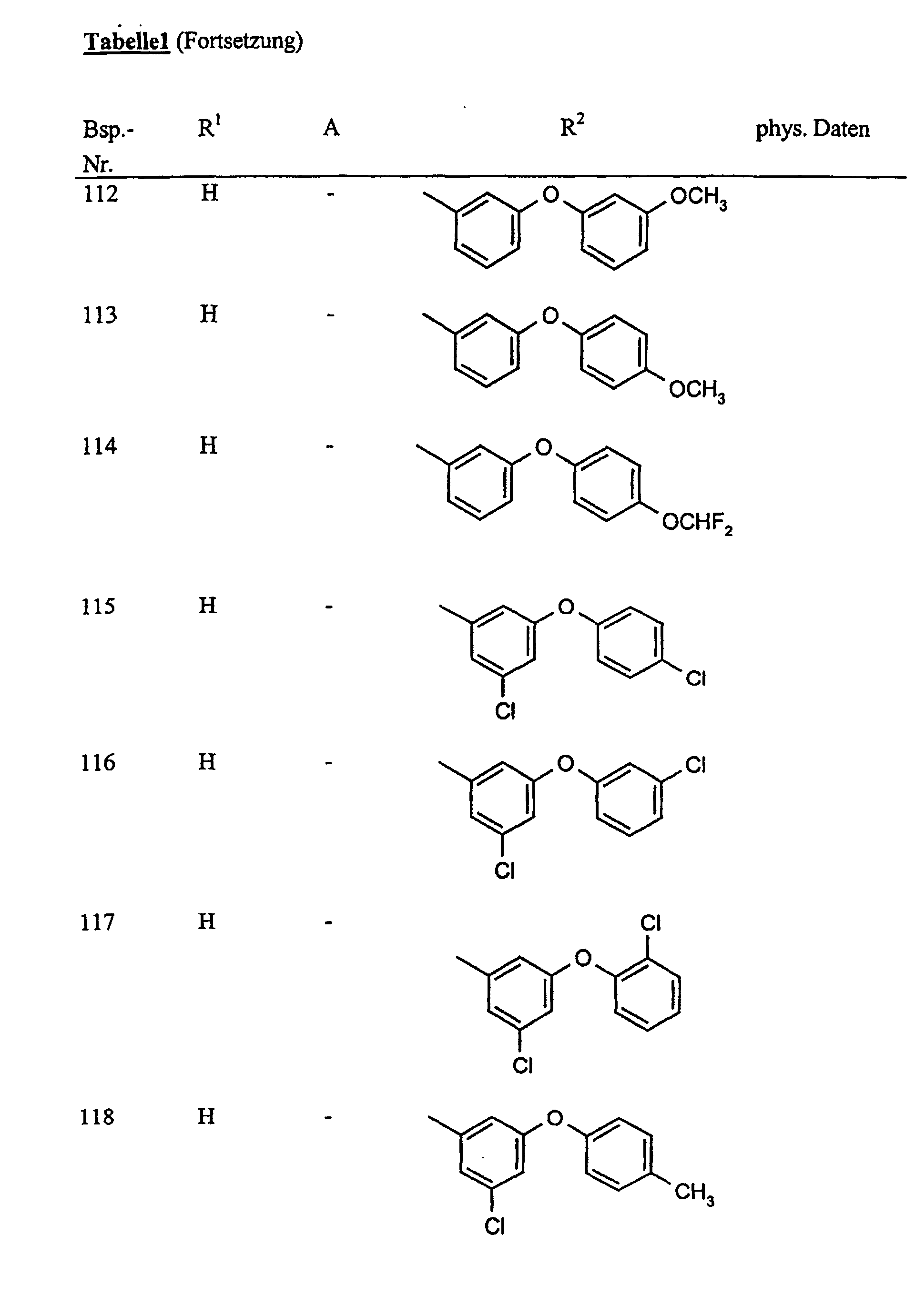

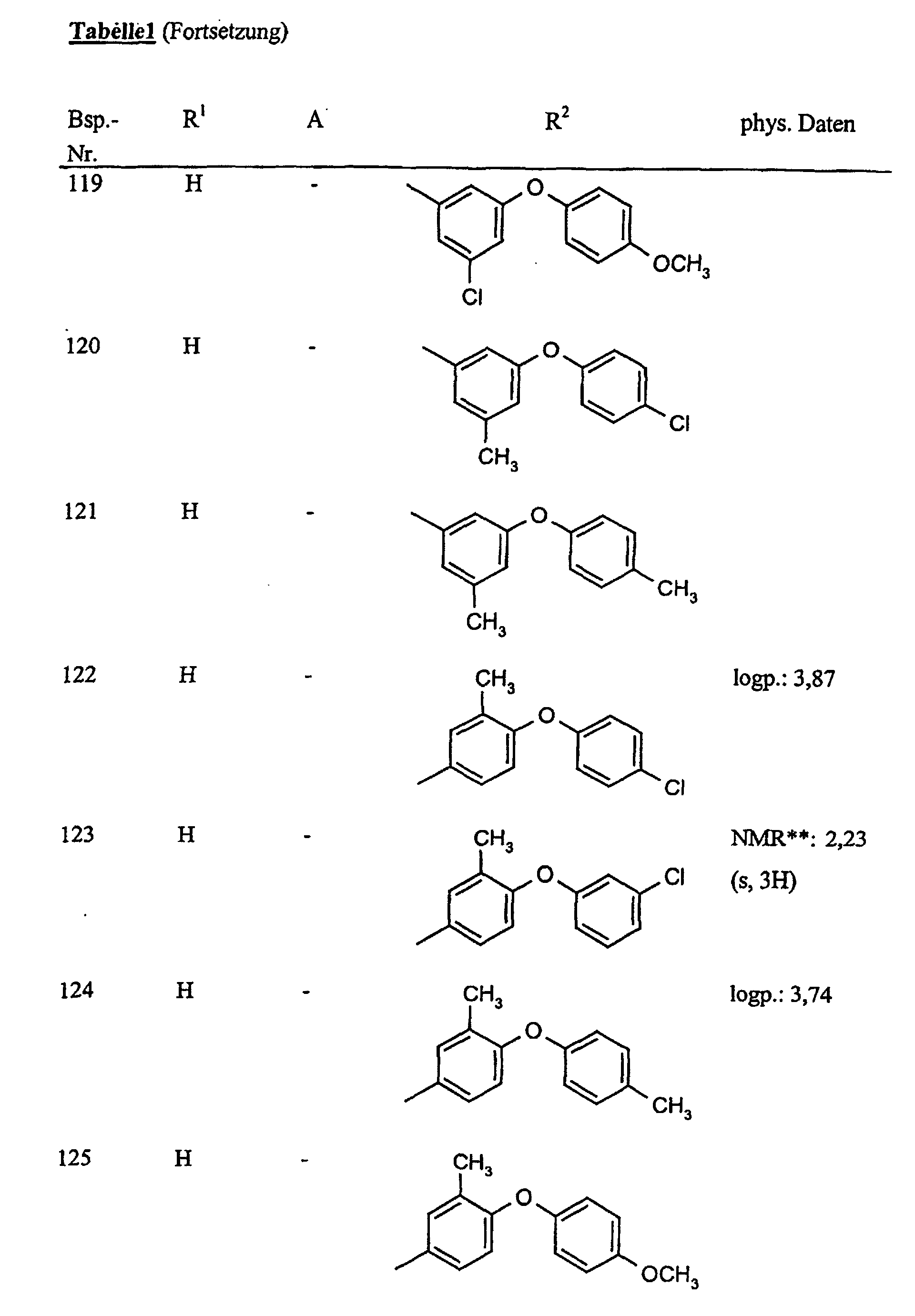

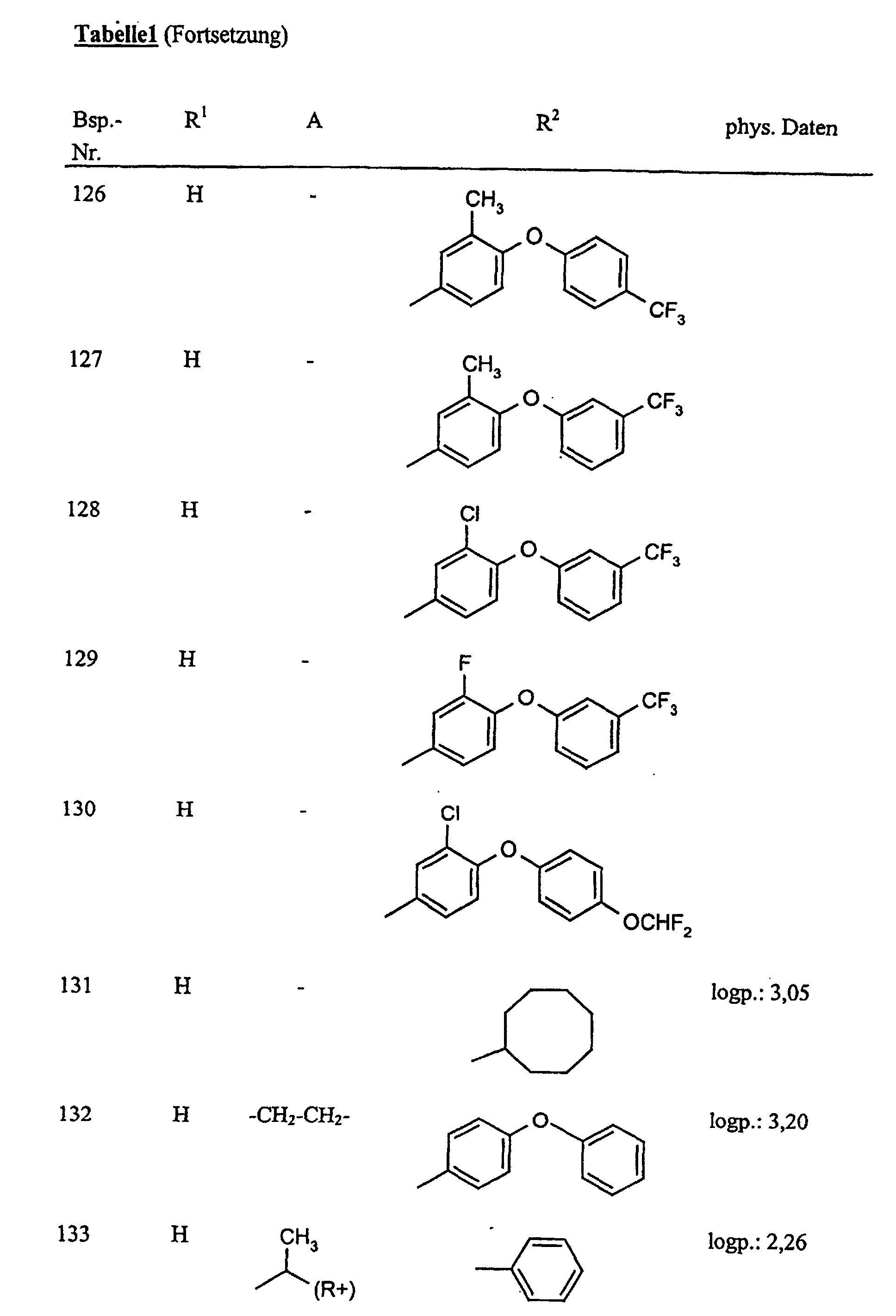

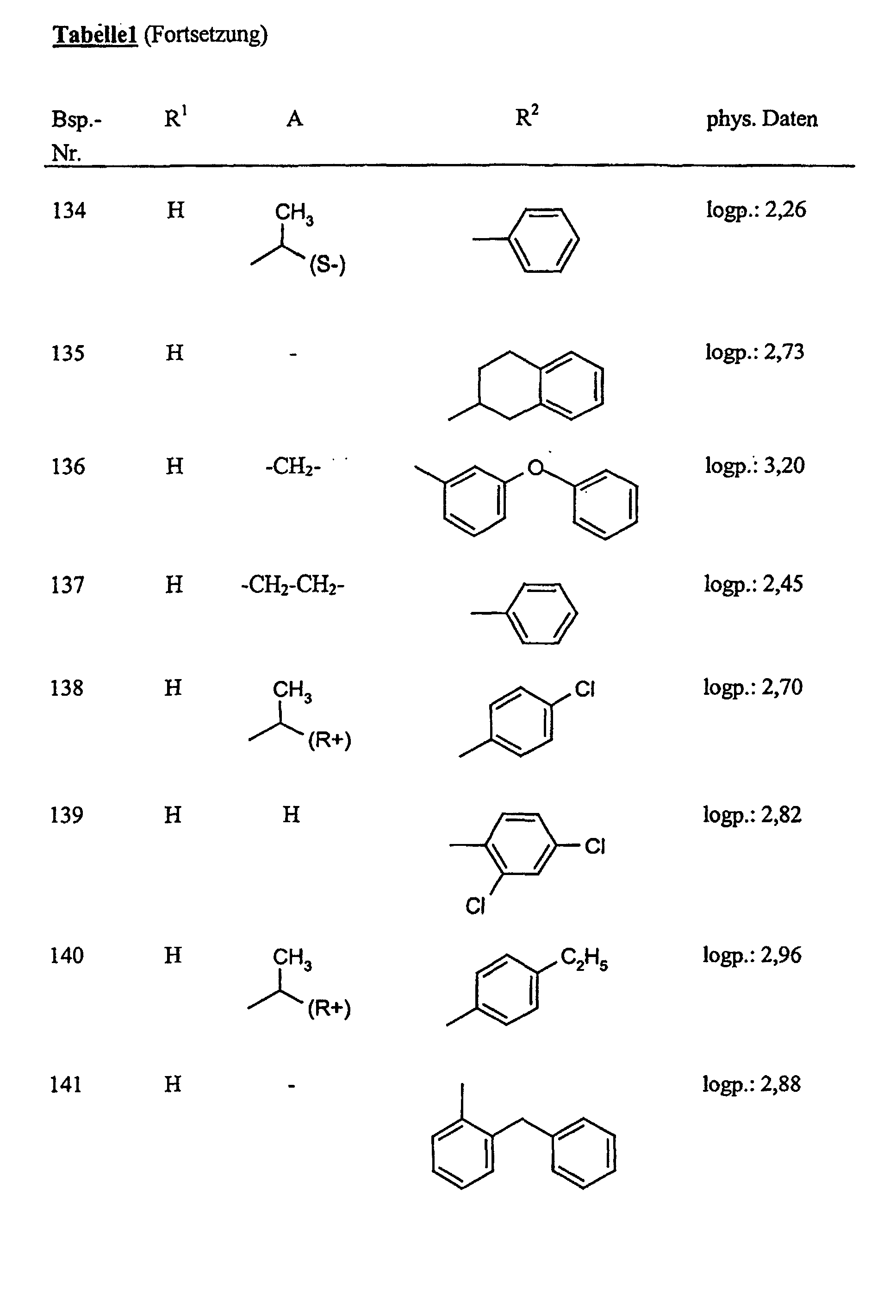

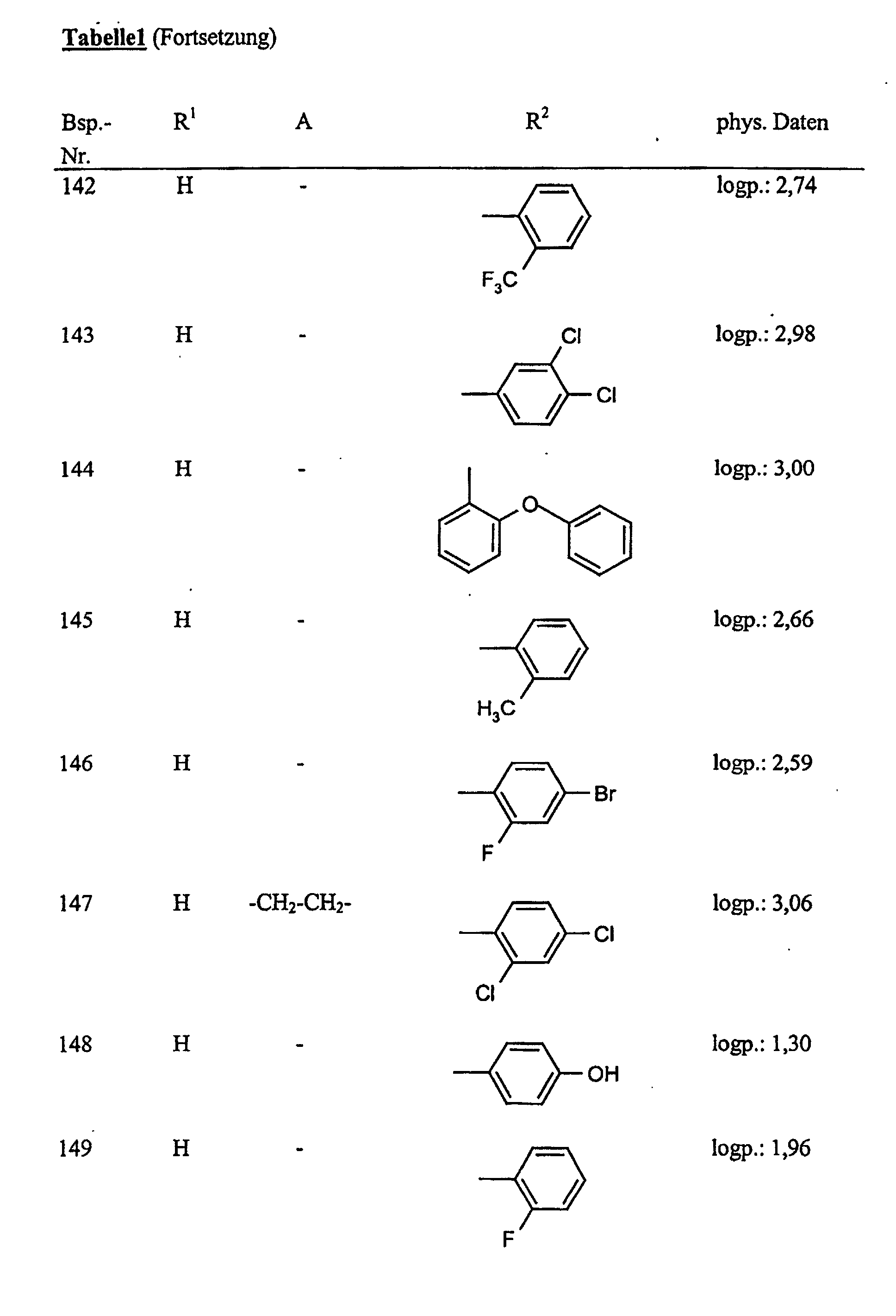

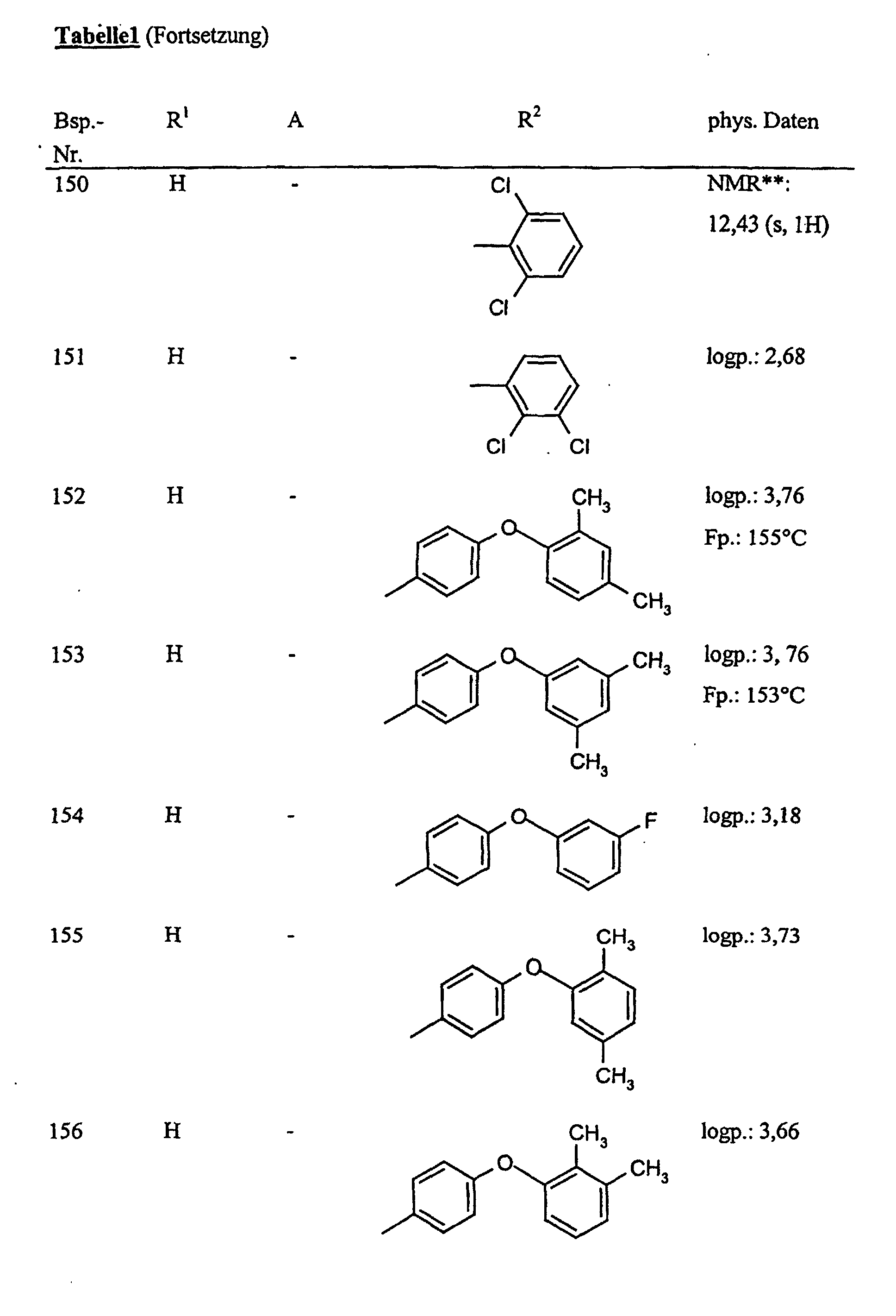

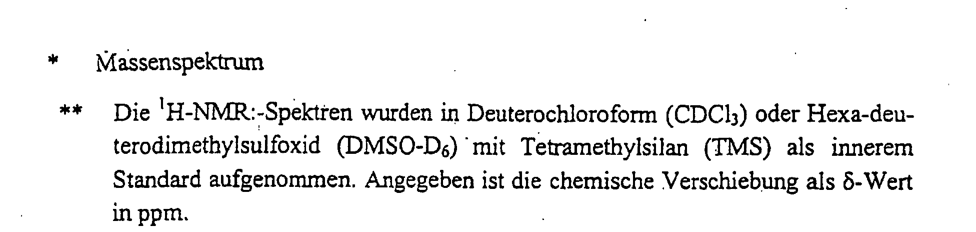

Analog des Herstellungsbeispieles (II-1) sowie entsprechend der allgemeinen Beschreibung der erfindungsgemäßen Verfahren a-1a) und a-1b) werden die in der nachstehenden Tabelle 2 aufgeführten Verbindungen der allgemeinen Formel (II) erhalten:

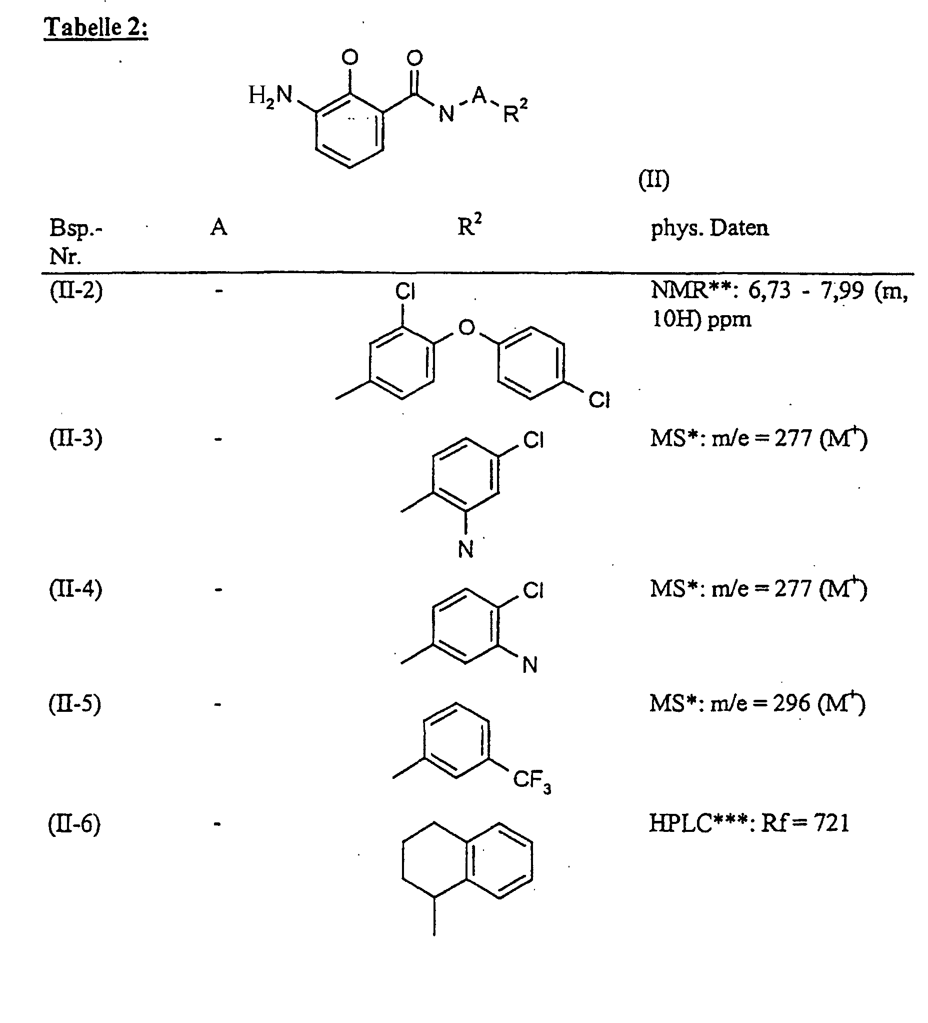

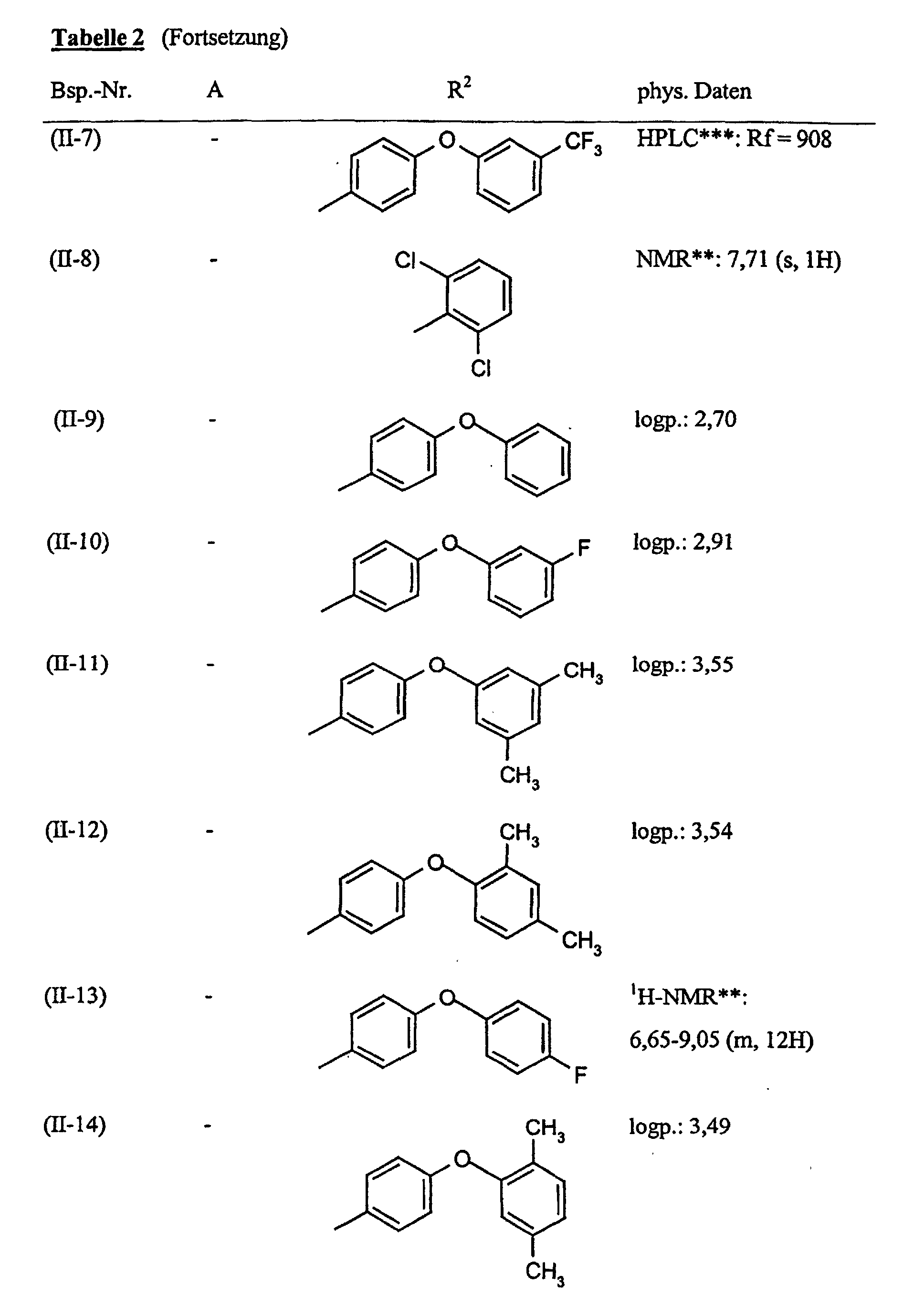

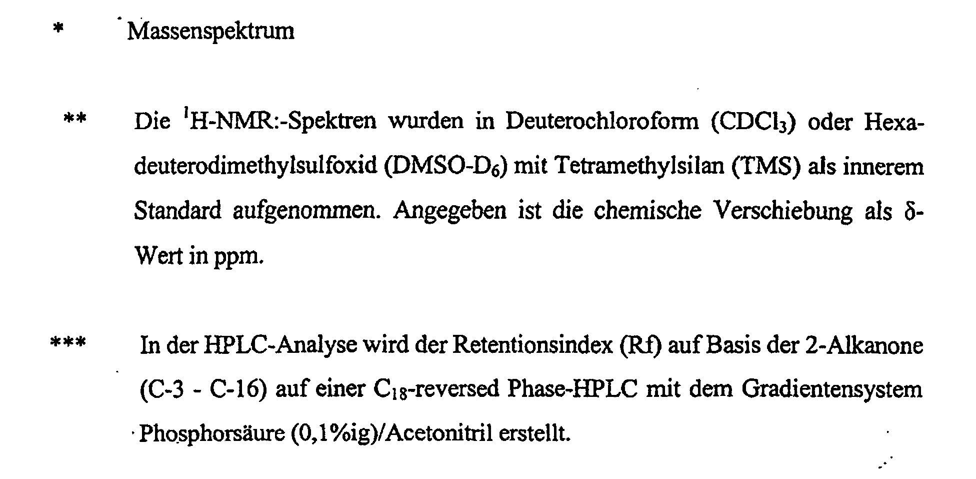

Analog des Herstellungsbeispieles (V-1) sowie entsprechend der allgemeinen Beschreibung des erfindungsgemäßen Verfahrens a-2) werden die in der nachstehenden Tabelle 3 aufgeführten Verbindungen der allgemeinen Formel (V) erhalten:

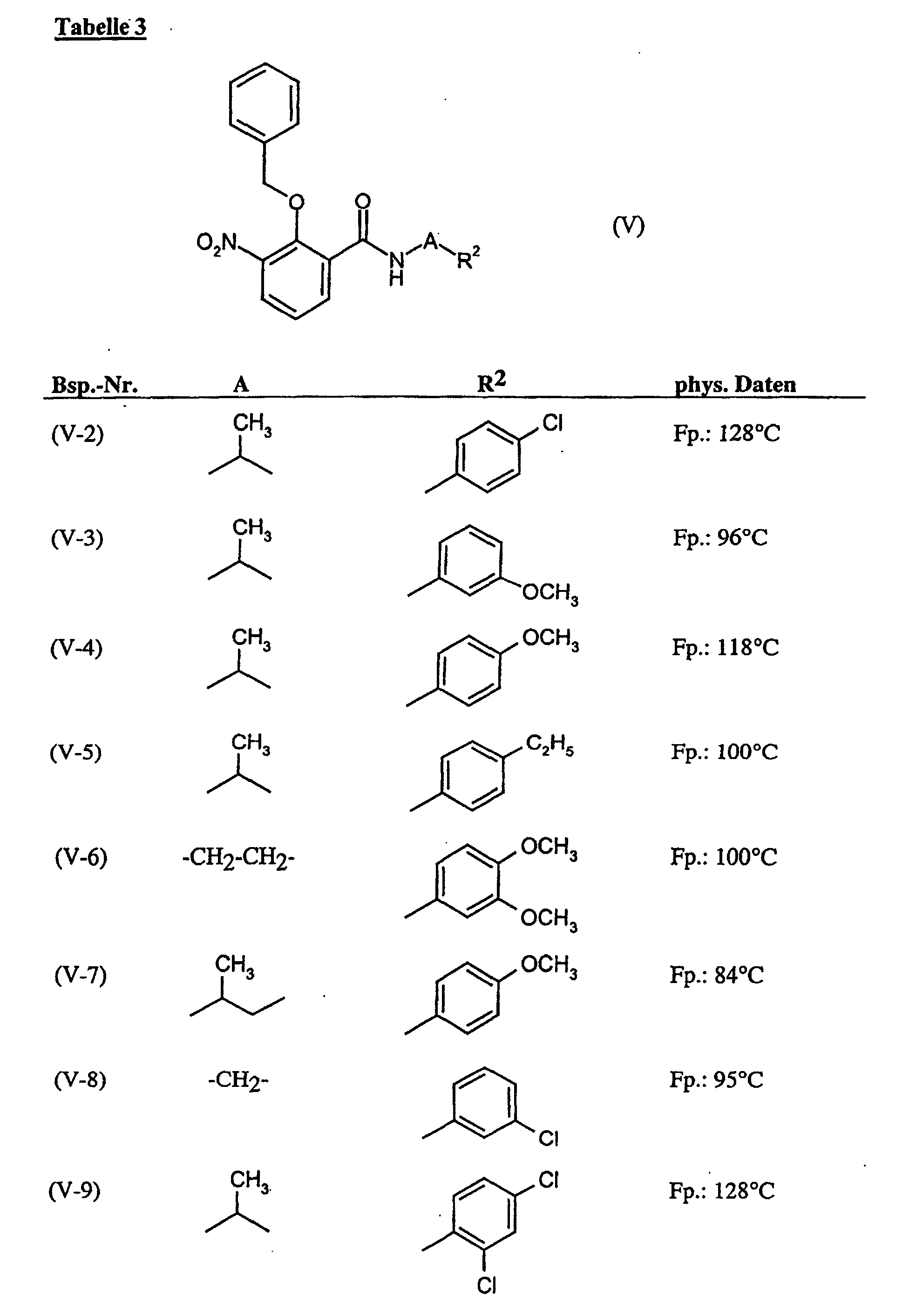

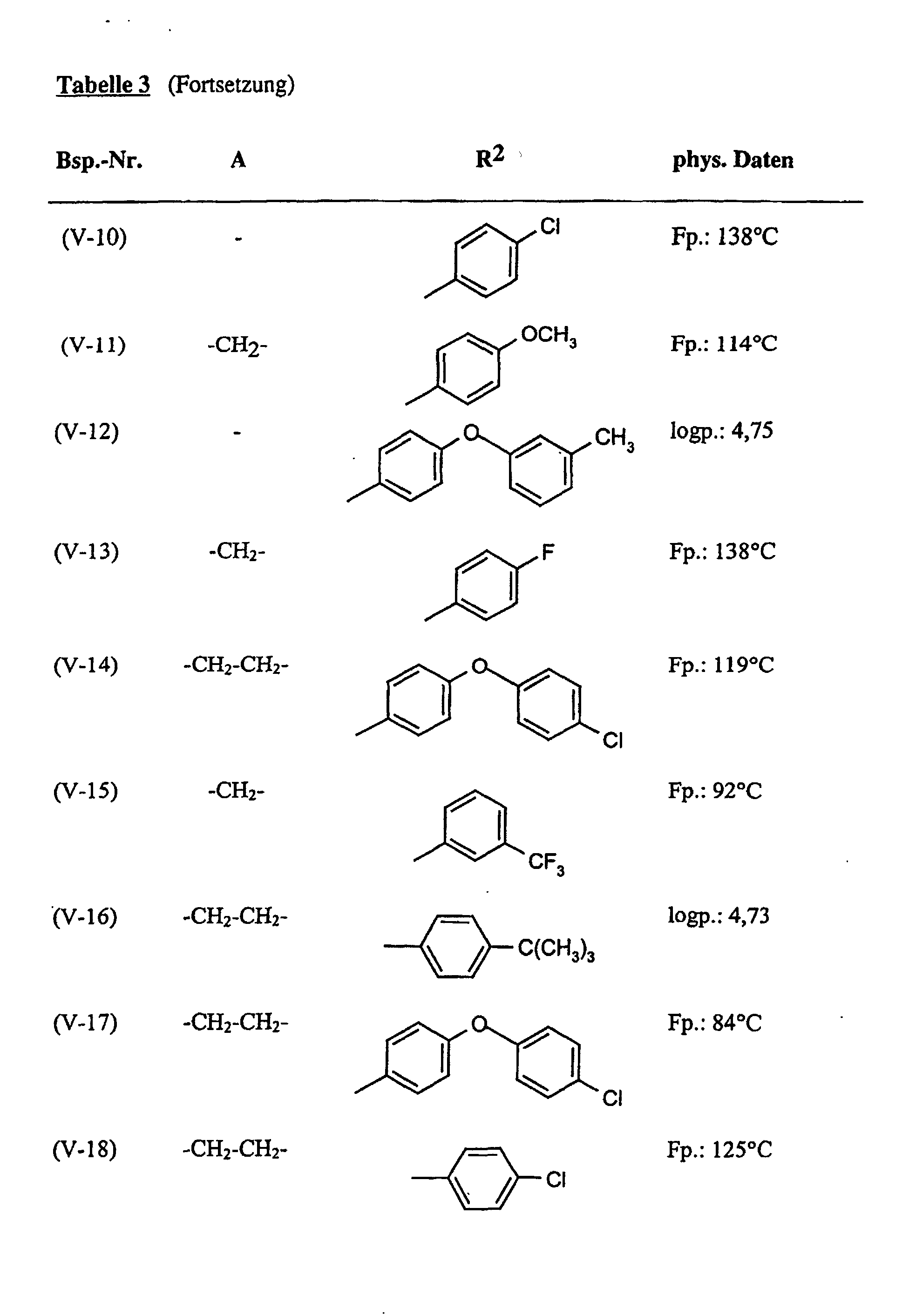

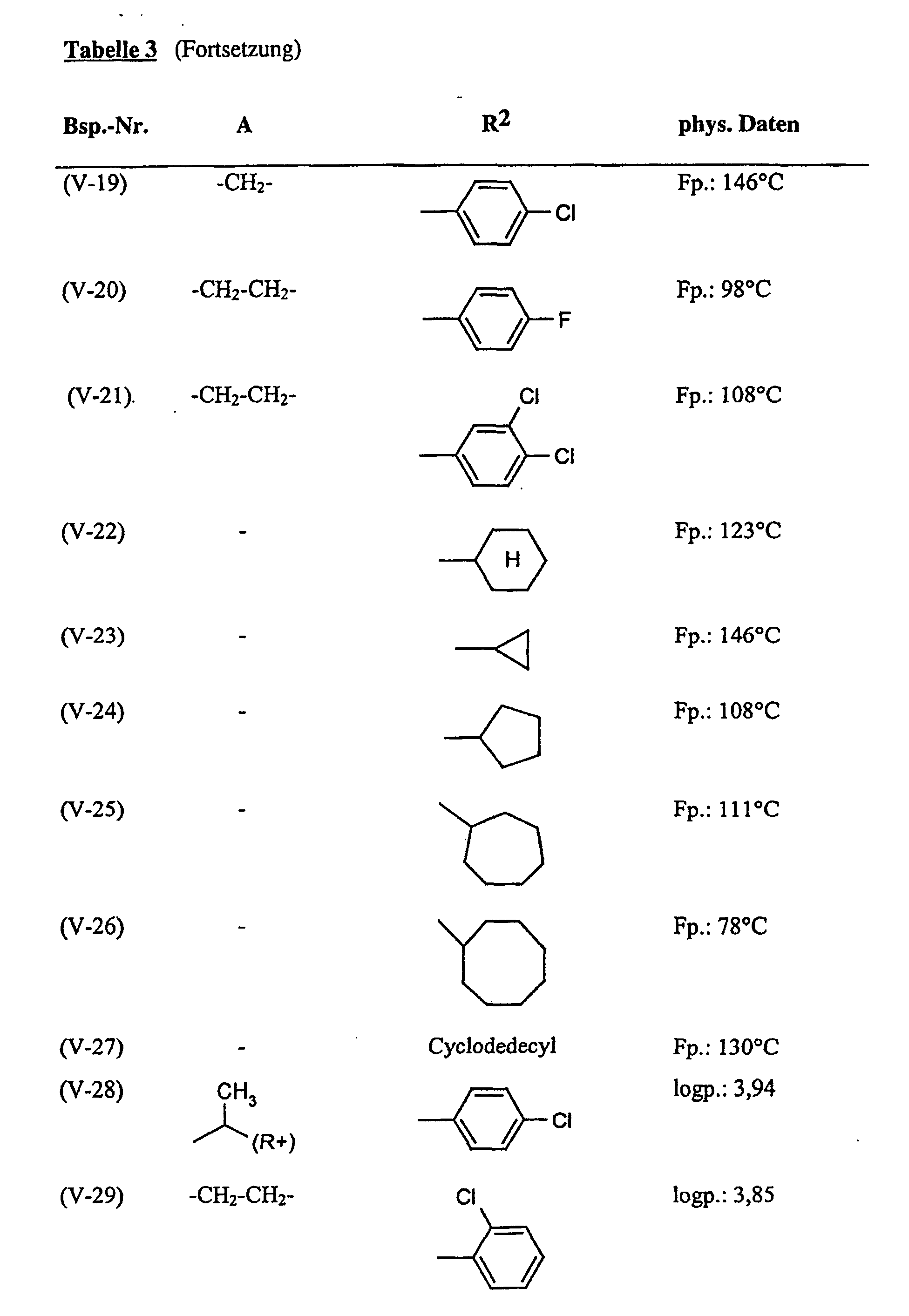

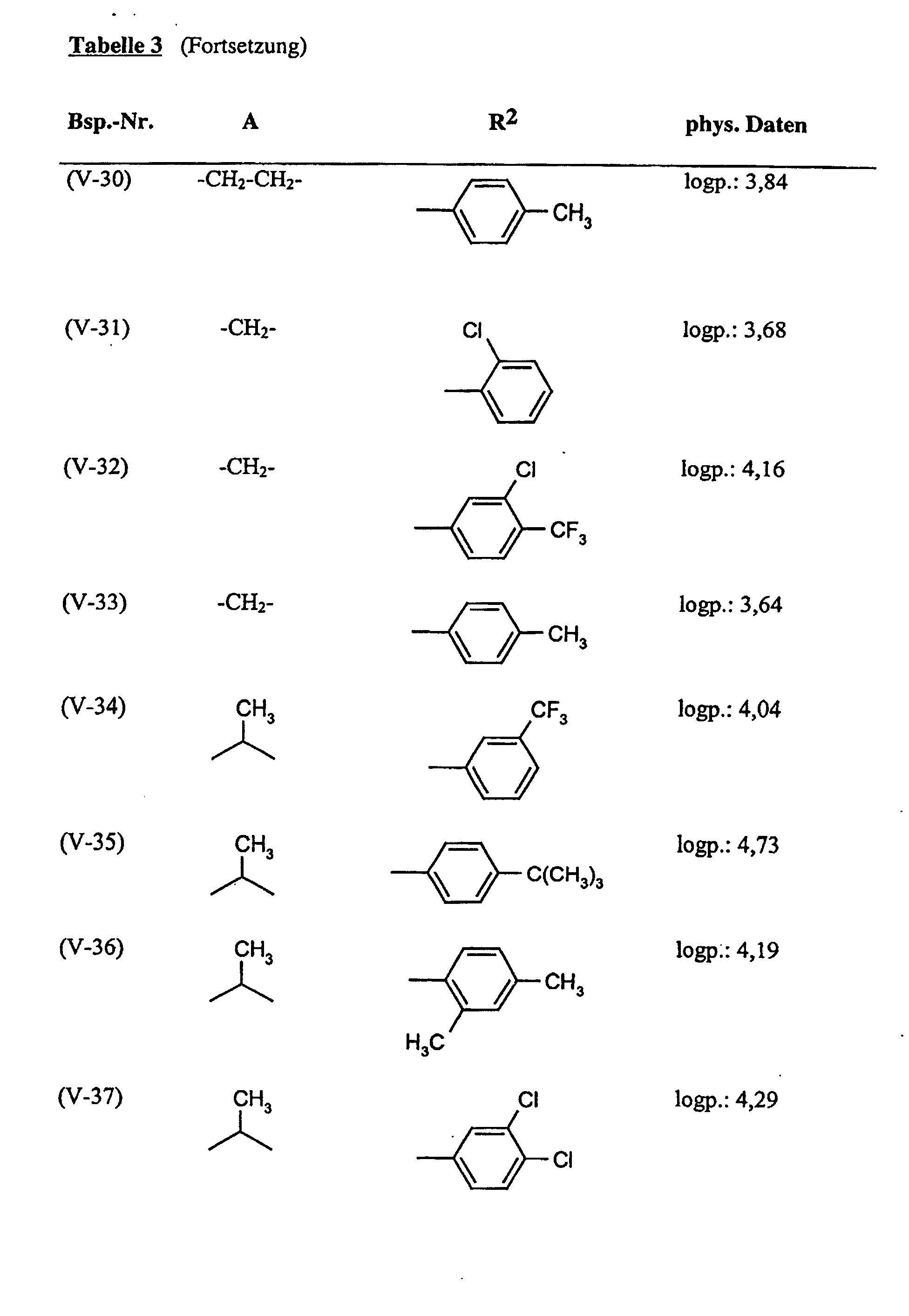

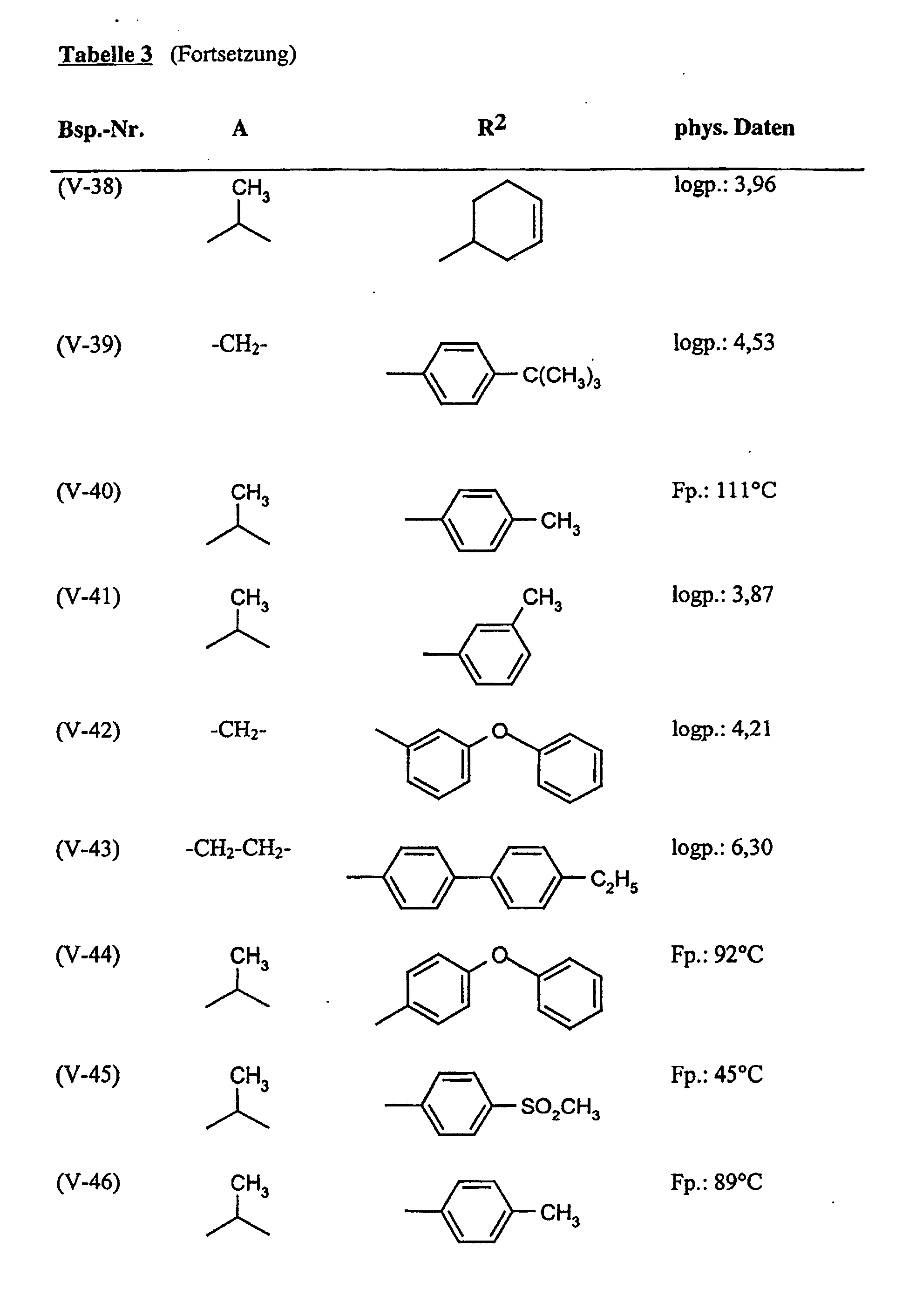

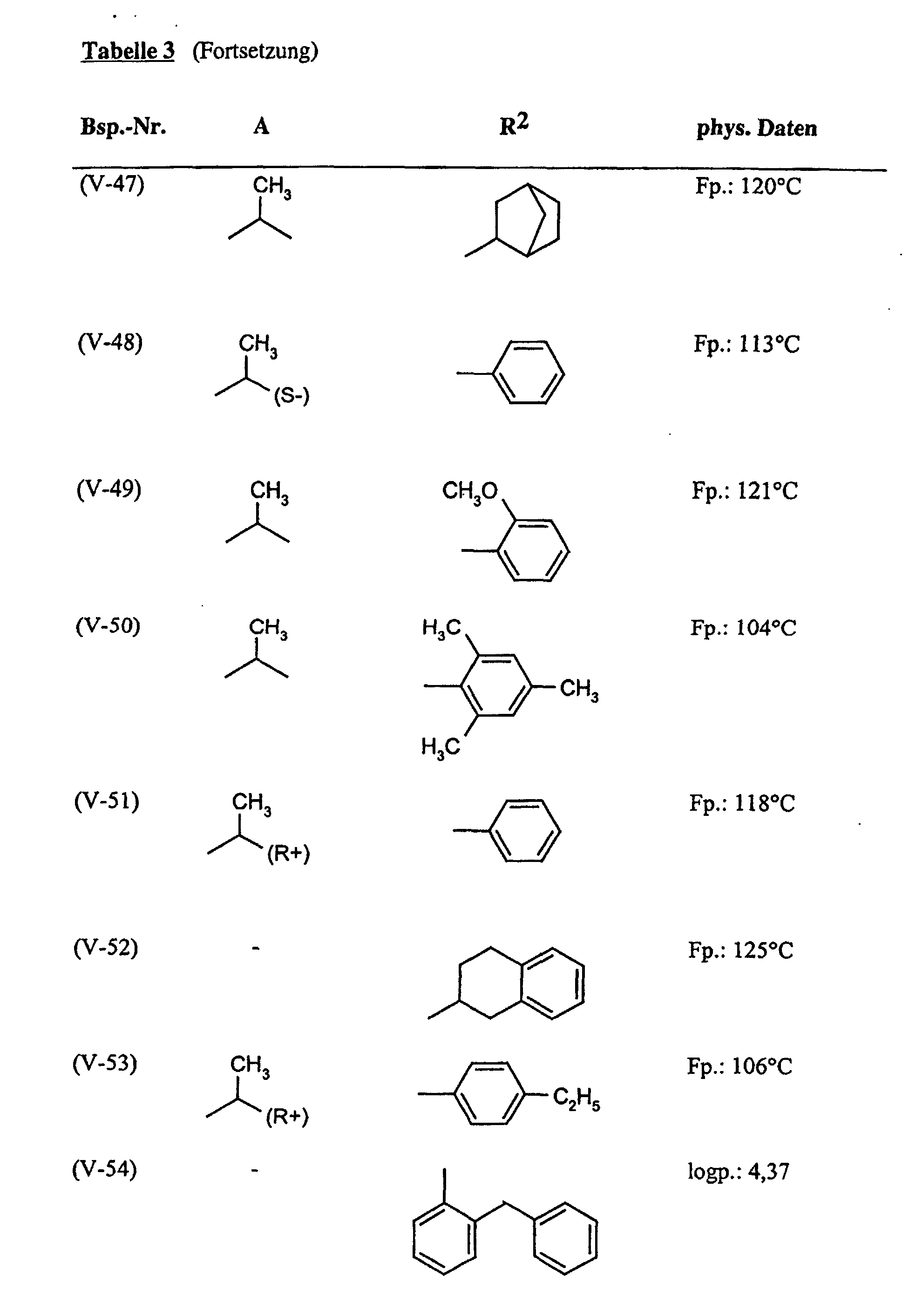

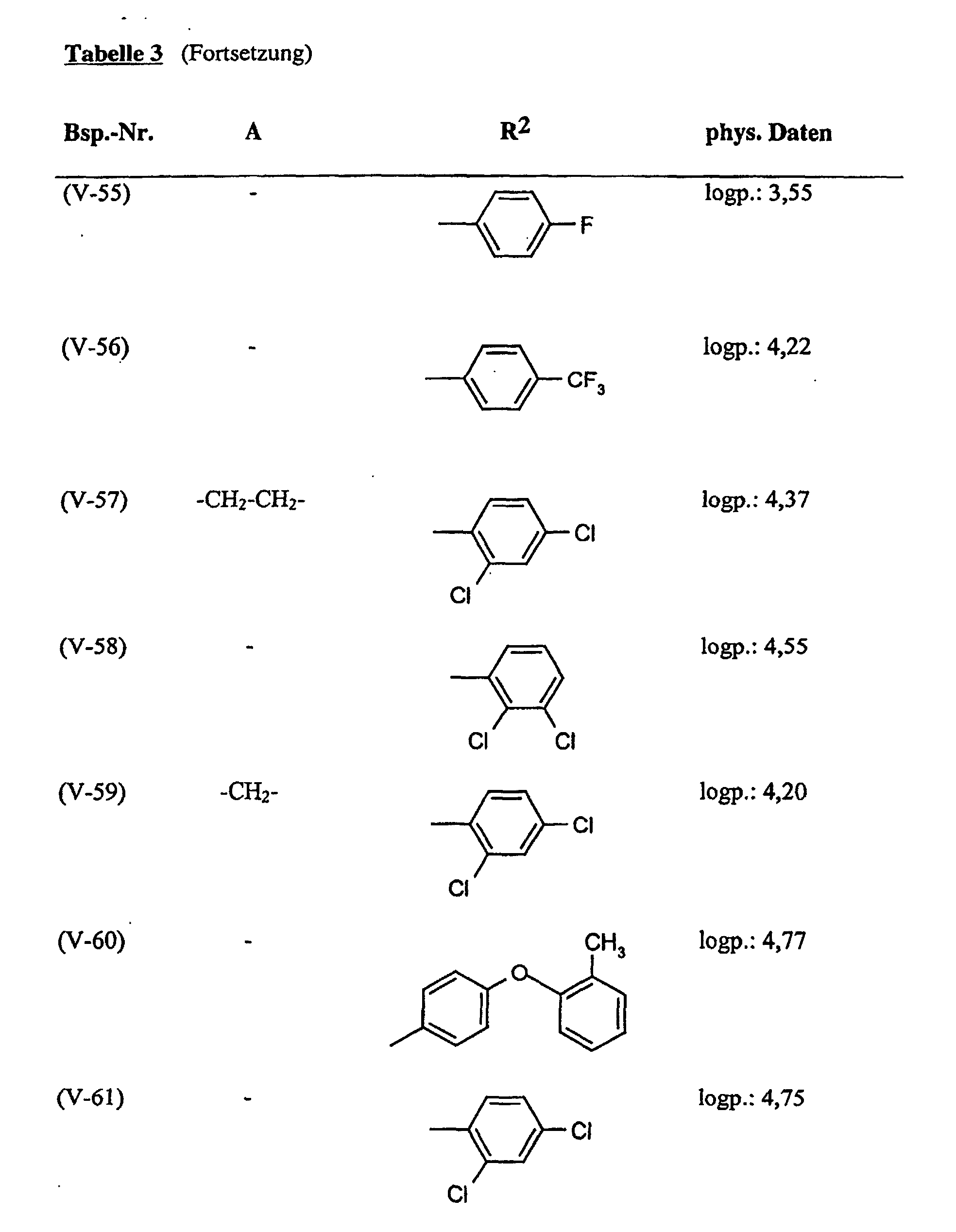

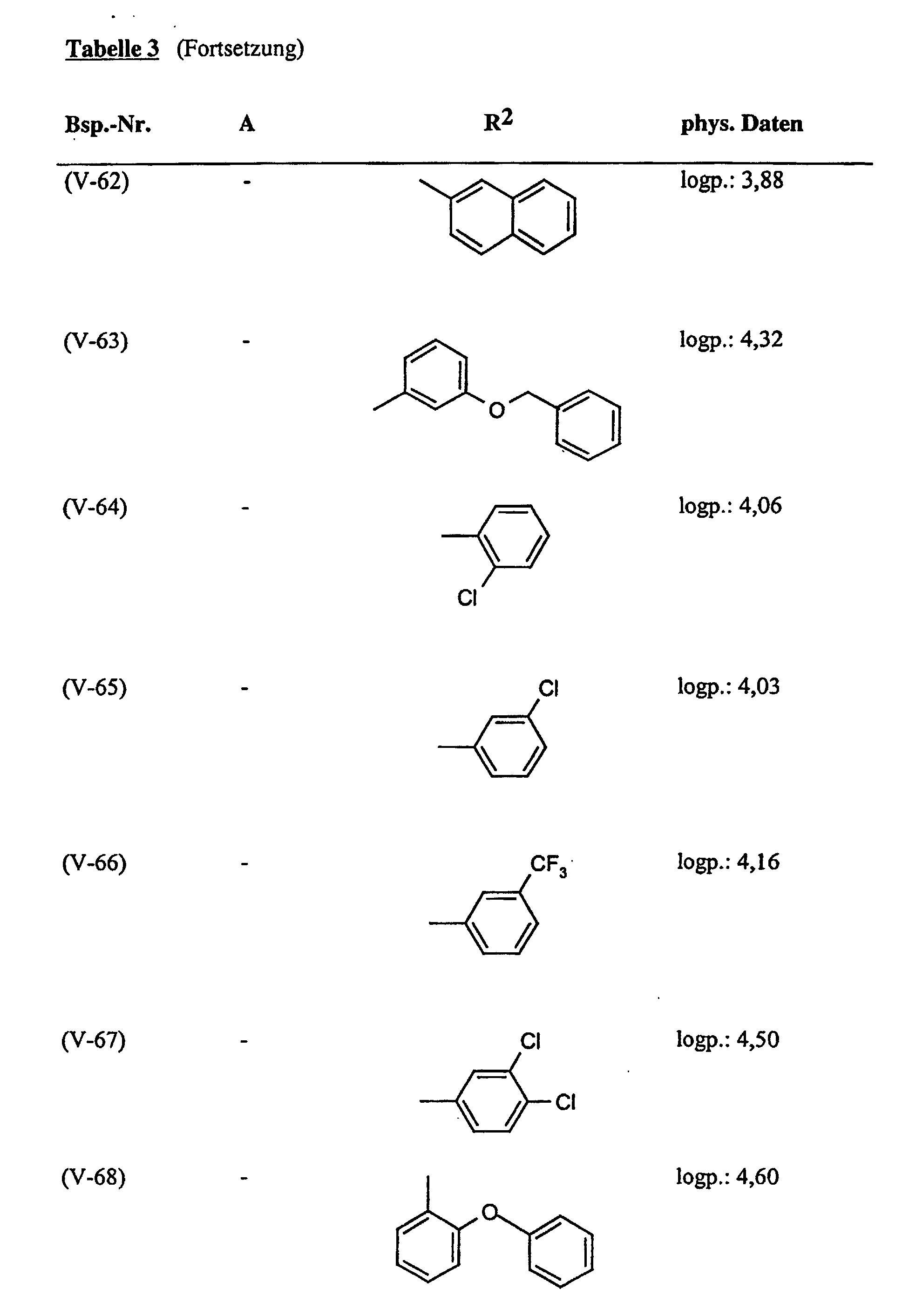

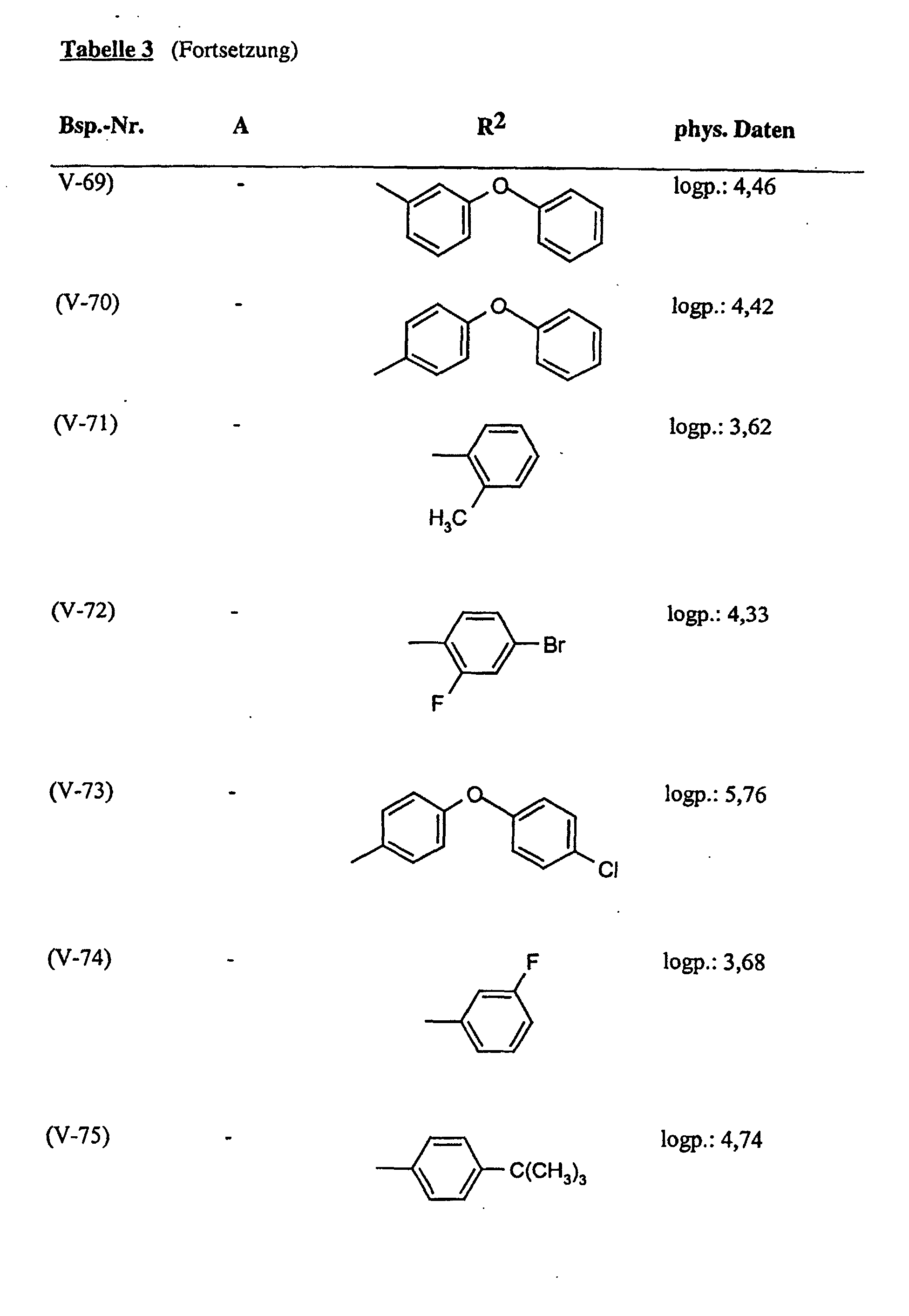

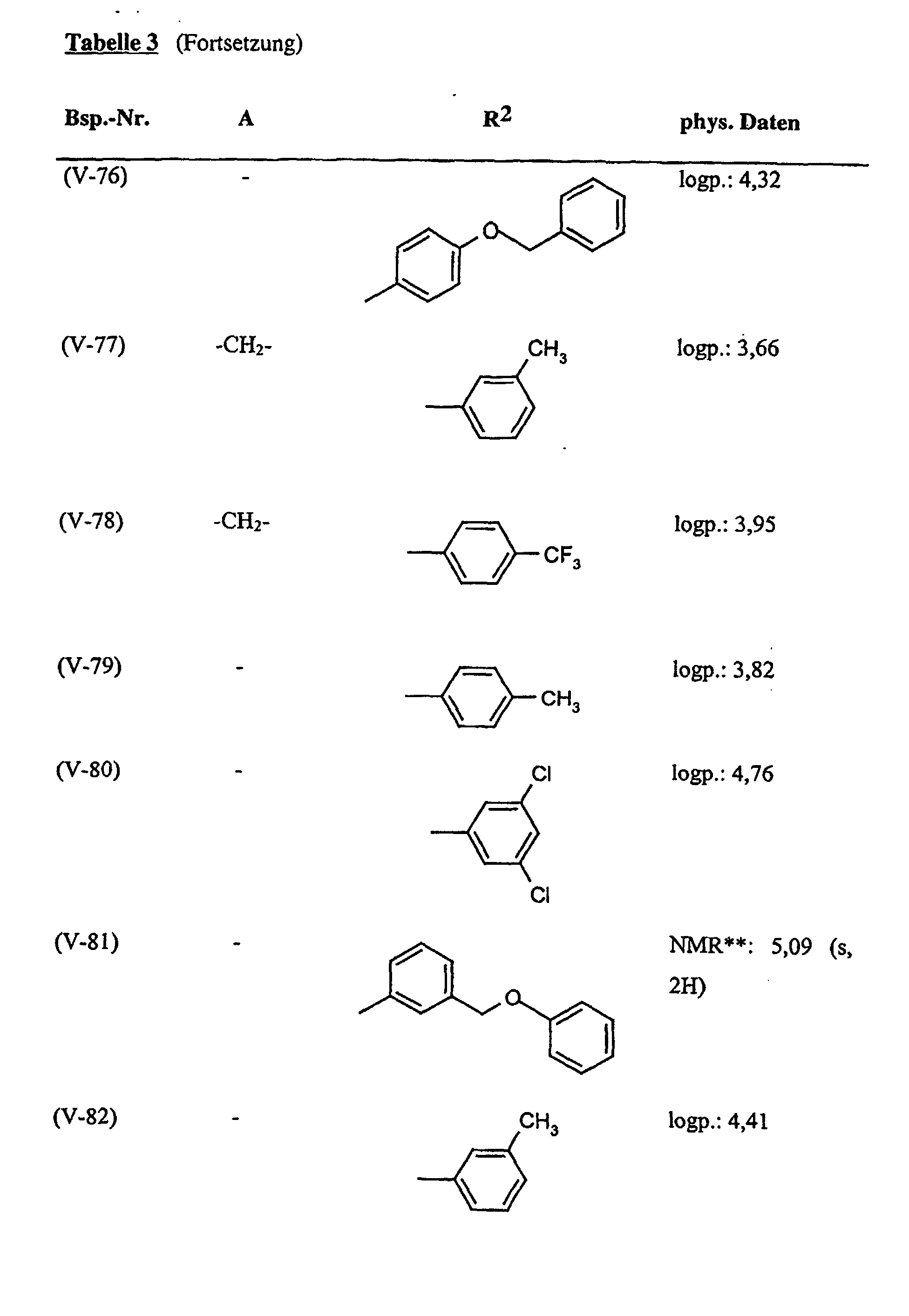

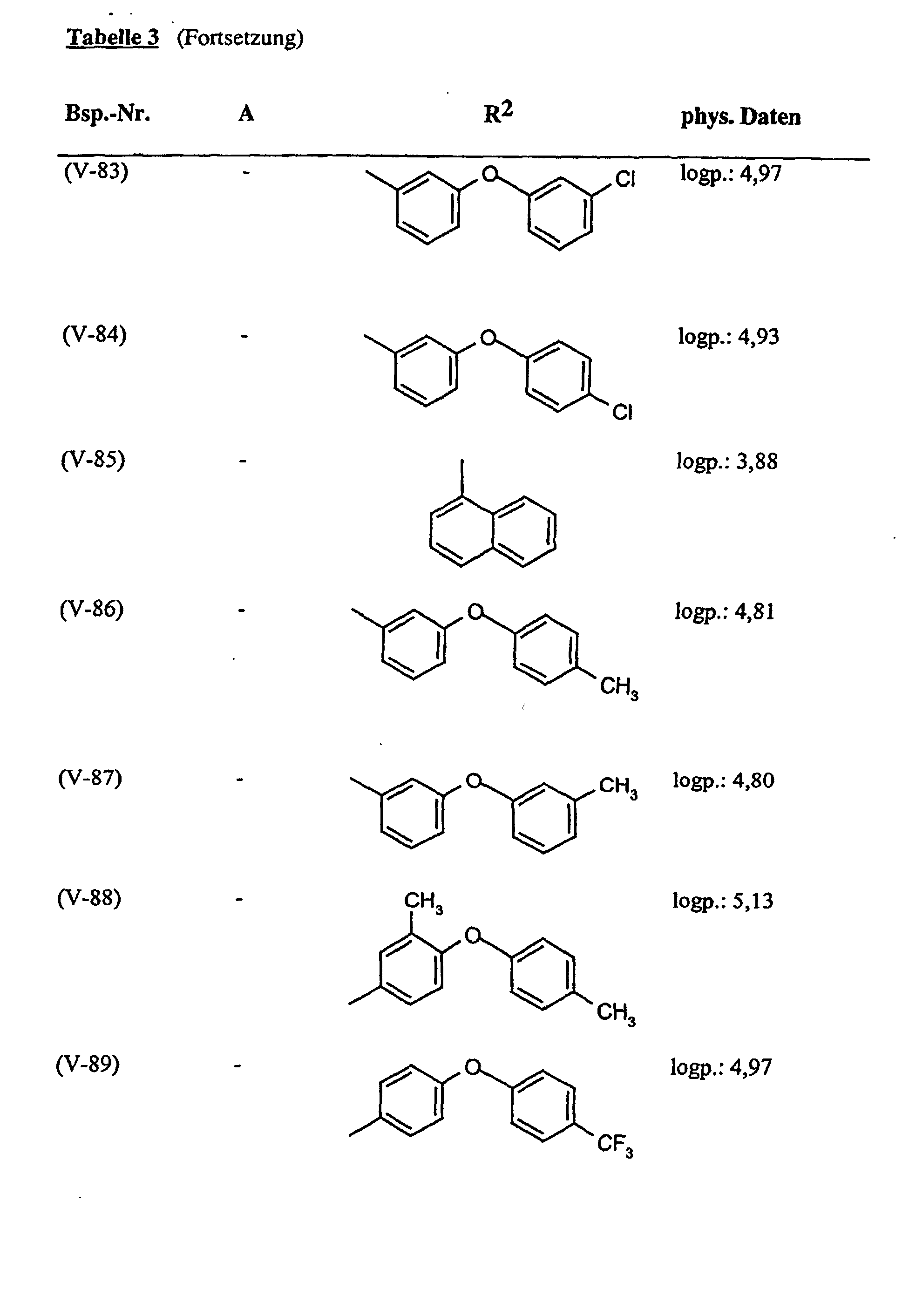

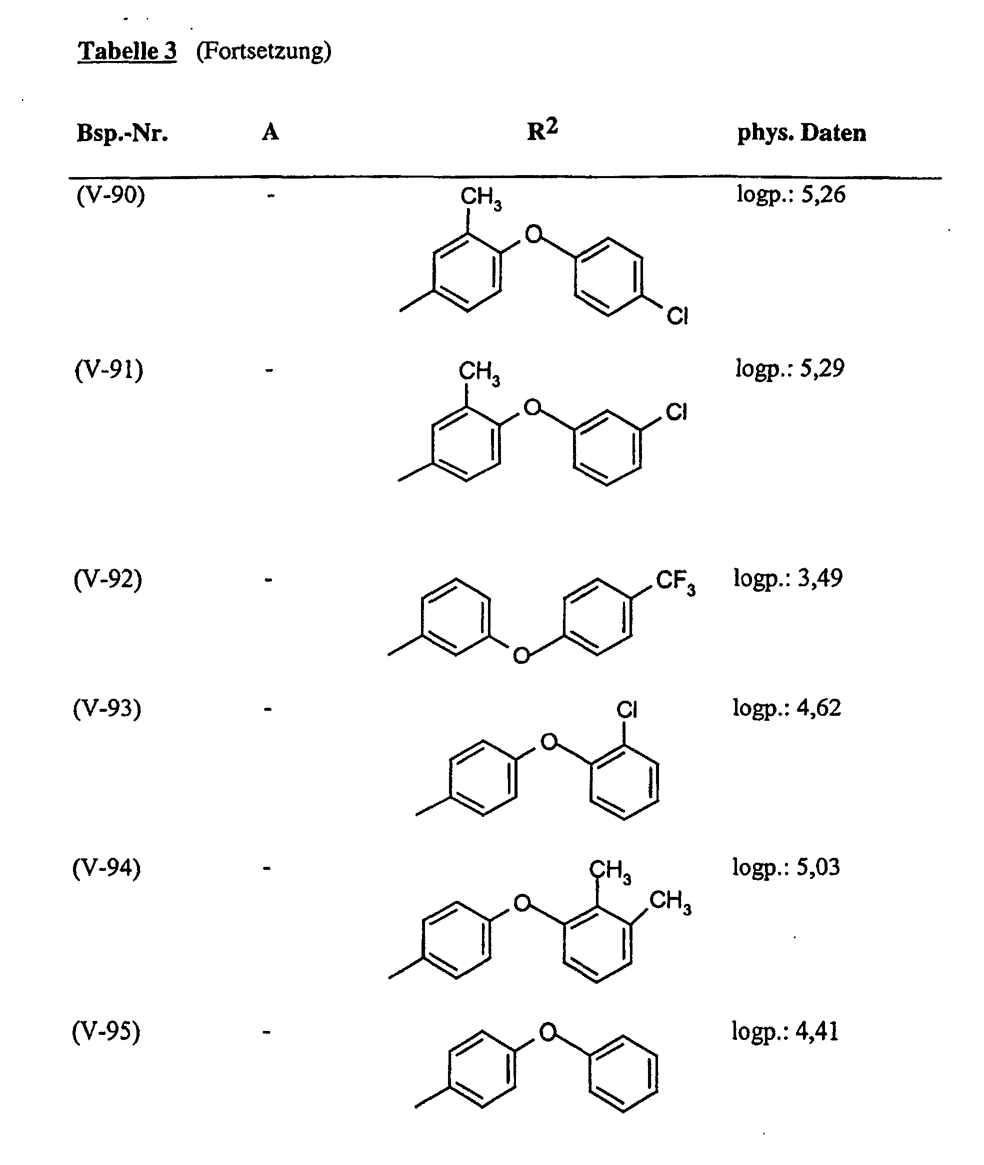

Analog des Herstellungsbeispieles (IV-a-1) sowie entsprechend der allgemeinen Beschreibung des erfindungsgemäßen Verfahrens b-1) werden die in der nachstehenden Tabelle 4 aufgeführten Verbindungen der allgemeinen Formel (IV-a) erhalten:

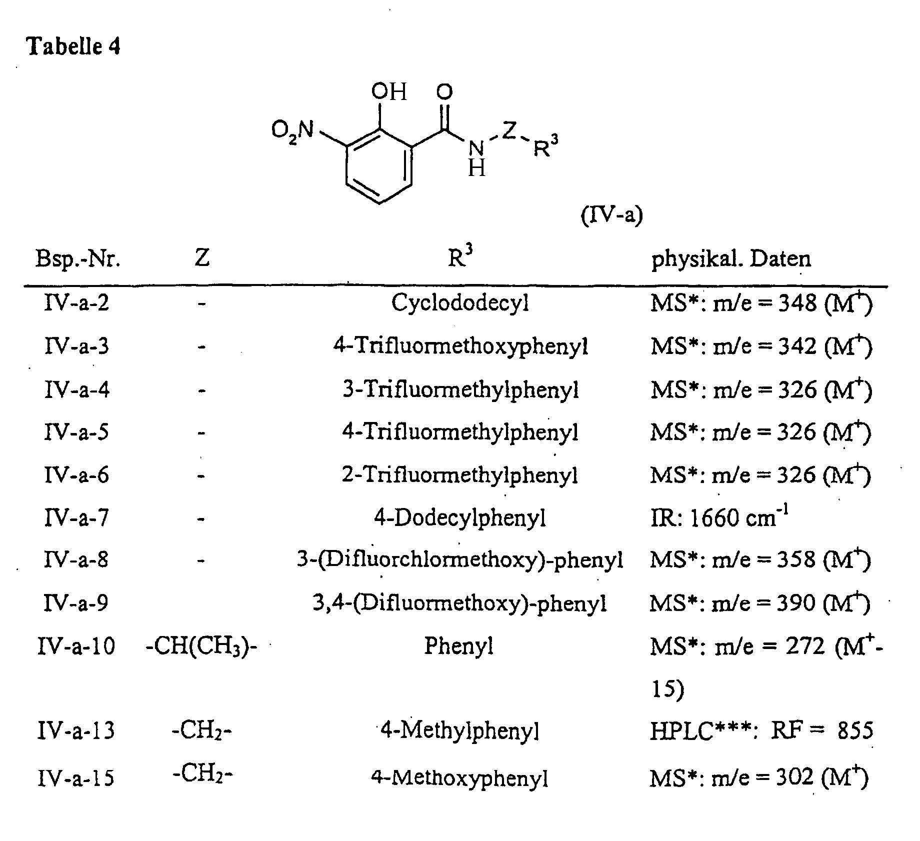

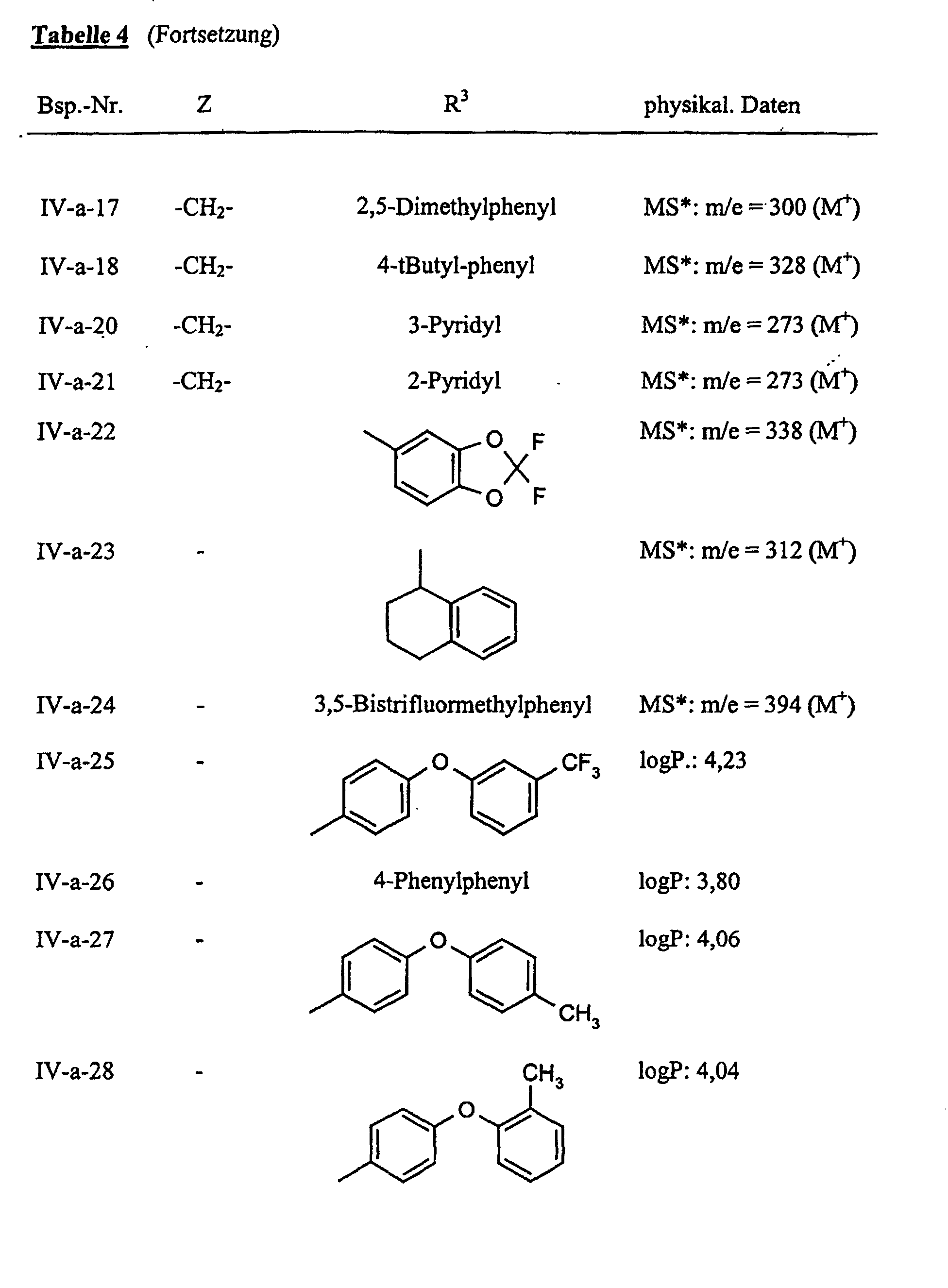

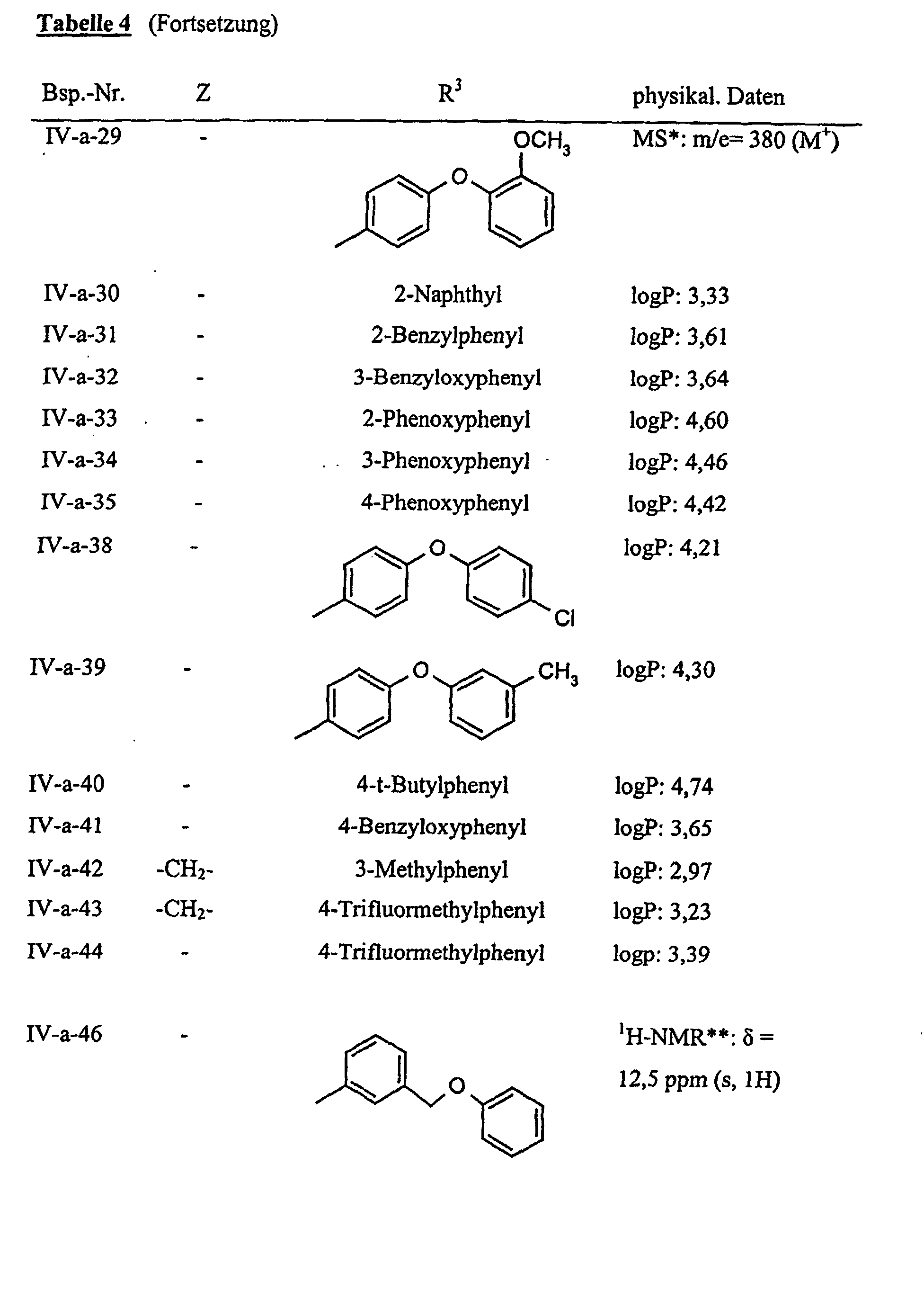

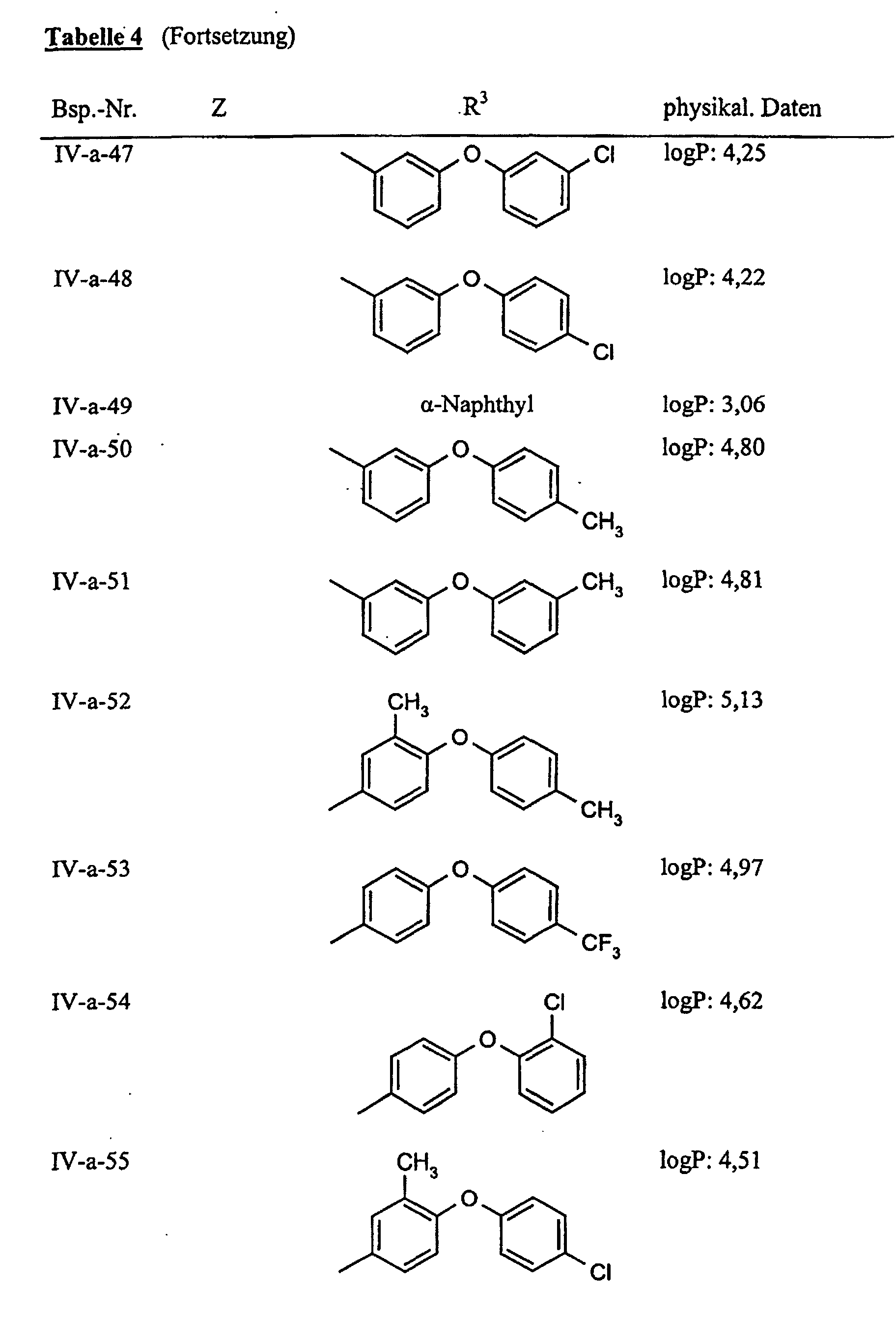

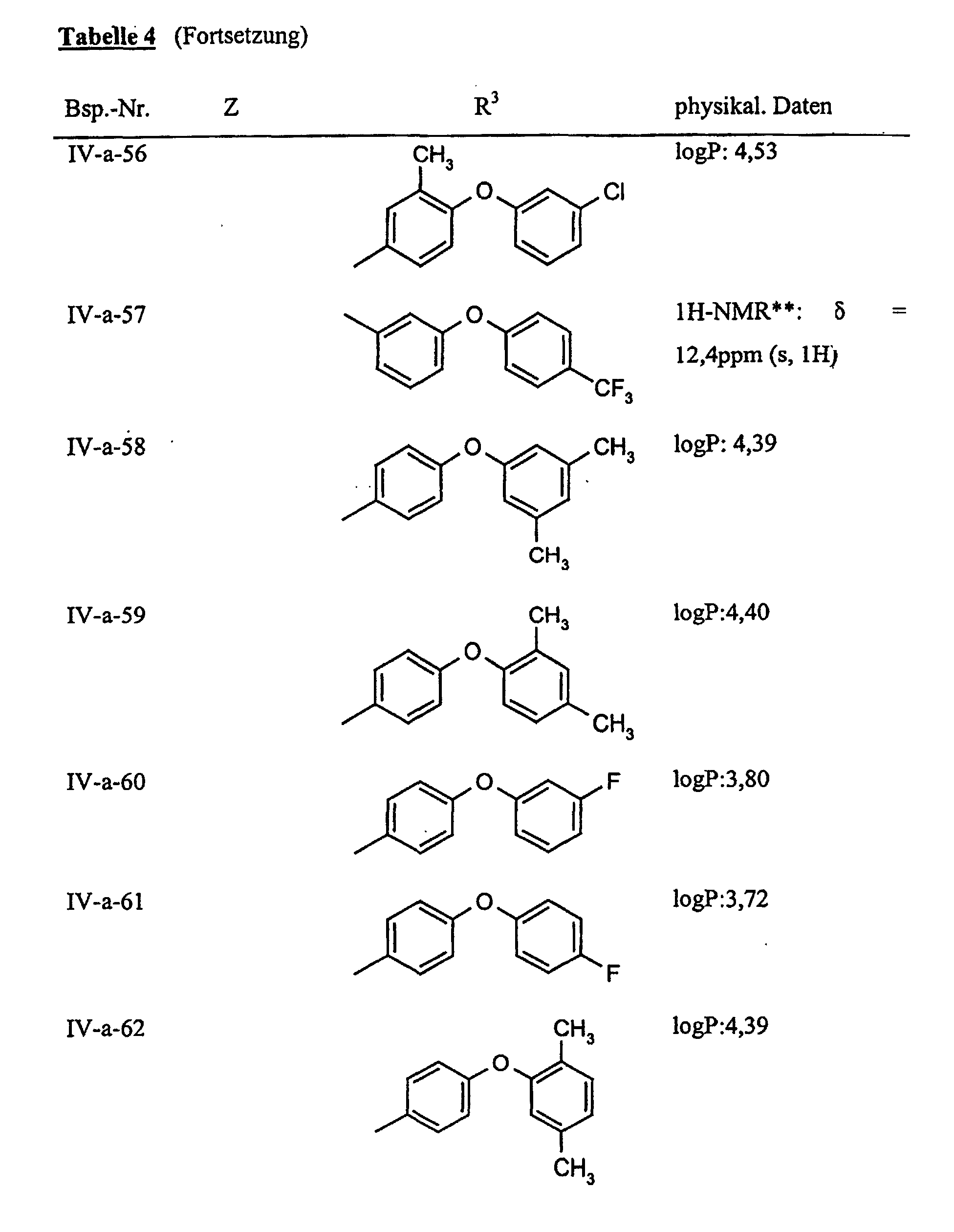

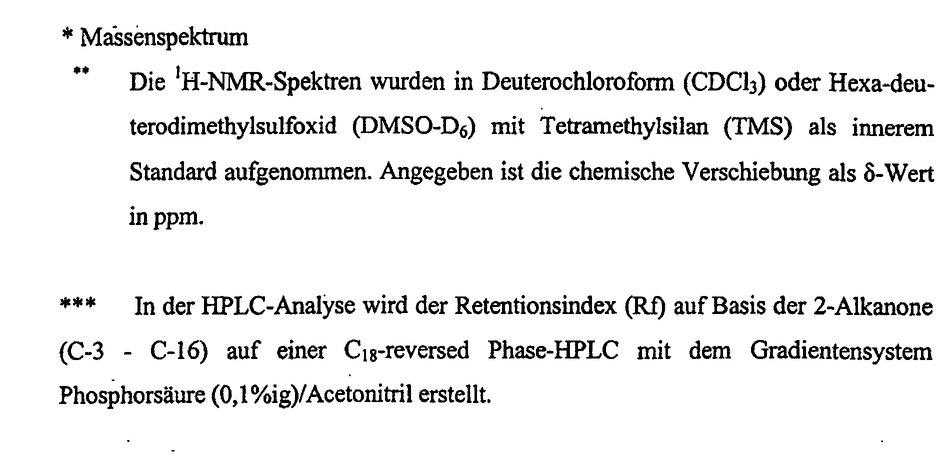

### Anwendungsbeispiele:

### Beispiel: A

### Sphaerotheca-Test (Gurke) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Bei diesem Test zeigt z.B. die folgende Verbindung (5) der Herstellungsbeispiele bei einer Wirkstoffkonzentration von 100 ppm einen Wirkungsgrad von mehr als 80%.

### Beispiel B

### Venturia-Test (Apfel) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung bis zur Tropfnässe besprüht. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Bei diesem Test zeigen z.B. die folgenden Verbindungen der Herstellungsbeispiele (2), (4), (5), (12) und (15) bei einer Wirkstoffkonzentration von 100 ppm einen Wirkungsgrad von mehr als 80 %.

### Beispiel C

### Pyricularia-Test (Reis) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit werden junge Reispflanzen mit der Wirkstoffzubereitung bis zur Tropfnässe besprüht. 4 Tage nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Bei diesem Test zeigt z.B. die folgende Verbindung (5) der Herstellungsbeispiele bei einer Wirkstoffkonzentration von 0,05 % einen Wirkungsgrad von mehr als 80 %.

### Beispiel: D

### Pseudocercosporella herpotrichoides-Test (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

Bei diesem Test zeigt z.B. die folgende Verbindung (5) der Herstellungsbeispiele bei einer Wirkstoffaufwandmenge von 250 g/ha einen Wirkungsgrad von mehr als 80 %.

### Beispiel E

### Phytophthora-Test (Tomate) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Bei diesem Test zeigen z.B. die folgenden Verbindungen 2, 4, 5, 10, 12 und 15 bei einer Wirkstoffkonzentration von 100 ppm einen Wirkungsgrad von über 80 %.

### Beispiel F

### Plasmopara-Test (Reben) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

Bei diesem Test zeigen z.B. die Verbindungen 5, 15 und 16 bei einer Wirkstoffkonzentration von 100 ppm einen Wirkungsgrad von über 90 %.

### Beispiel G

### Botrytis-Test (Bohne) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet

Bei diesem Test zeigen die Verbindungen 2, 3, 4, 5, 10, 12, 15 und 16 bei einer Wirkstoffkonzentration von 500 ppm einen Wirkungsgrad von 90%.

### Beispiel : H

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

In diesem Test bewirkten z.B. die Verbindungen der Herstellungsbeispiele (V-34) und (47) bei einer beispielhaften Wirkstoffkonzentration von 0,01 % eine Abtötung von 100 % nach 7 Tagen, während die bekannte Verbindung (A) keine Abtötung zeigte.

### Beispiel: I

### Plutella-Test

- Lösungsmittel:: 7 Gewichtsteile Dimethylfomamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen der Herstellungsbeispiele (41), (39), (V-34), (14), (V-40), (V-51), (47), (131) und (37) bei einer beispielhaften Wirkstofkonzentration von 0,01 % eine Abtötung von 80 bis100 % nach 7 Tagen.

### Beispiel J

### Spodoptera-Test

- Lösungsmittel:: 7 Gewichtsteile
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda besetzt, solange die Blätter noch feucht sind.

Nach der gewünschten Zeit wird die Wirkung in % bestimmt. Dabei bedeutet 100%, daß alle Raupen abgetötet wurden; 0% bedeutet, daß keine Raupen abgetötet wurden.

Bei diesem Test zeigt z.B. die folgende Verbindung (47) bei einer beispielhaften Konzentration von 0,1 % nach 7 Tagen einen Abtötungsgrad von 85 %.

### Beispiel K

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen (39), (35) und (71) bei einer beispielhaften Wirkstoffkonzentration von 0,001 % eine Abtötung von 100 % nach 7 Tagen.

## Patentansprüche

1. Verwendung von Verbindungen der Formel (I), 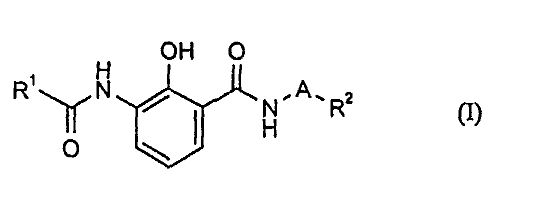 in welcher
A für eine Einfachbindung oder für eine Alkylenkette mit 1 bis 6 Kohlenstoffatomen steht,
R¹ für Wasserstoff, Alkyl oder Alkoxy mit jeweils 1 bis 4 Kohlenstoffatomen steht,
R² für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl oder Cycloalkenyl mit 3 bis 12 Kohlenstoffatomen;
für jeweils gegebenenfalls einfach oder mehrfach, gleich oder verschieden substituiertes Aryl mit bis zu 12 Ringgliedern oder für Heterocyclyl mit 3 bis 8 Ringgliedern steht, wobei die möglichen Substituenten aus der nachstehenden Aufzählung ausgewählt sind:
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und I bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
substituiertes Aryl, Aryloxy, Arylthio, Arylalkyl, Arylalkyloxy, Arylalkylthio, Aryloxyalkyl, Arylthioalkyl, Heterocyclyl, Heterocyclyloxy, Heterocyclylthio, Heterocyclylalkyl, Heterocyclylalkyloxy oder Heterocyclylalkylthio.
zur Bekämpfung von Schädlingen an Pflanzen und technischen Materialien.

2. Verbindungen der Formel (I), 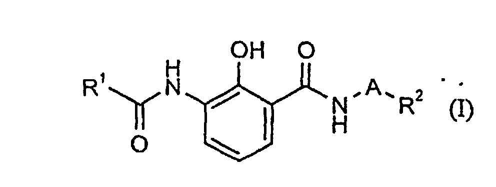 in welcher
A für eine Einfachbindung oder für eine Alkylenkette mit 1 bis 6 Kohlenstoffatomen steht,
R¹ für Wasserstoff, Alkyl oder Alkoxy mit jeweils 1 bis 4 Kohlenstoffatomen steht,
R² für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substituiertes Cycloalkyl oder Cycloalkenyl mit 3 bis 12 Kohlenstoffatomen;
für jeweils gegebenenfalls einfach oder mehrfach, gleich oder verschieden substituiertes Aryl mit bis zu 12 Ringgliedern oder für Heterocyclyl mit 3 bis 8 Ringgliedern steht, wobei die möglichen Substituenten aus der nachstehenden Aufzählung ausgewählt sind:
Halogen, Cyano, Amino, Hydroxy, Formyl, Carboxy, Carbamoyl, Thiocarbarnoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Acylamino, N-Acyl-N-alkylamino, Alkylamino, Dialkylamino, Alkylcarbonyl, Alkylcarbonyloxy, Alkoxycarbonyl, Allylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
substituiertes Aryl, Aryloxy, Arylthio, Arylalkyl, Arylalkyloxy, Arylalkylthio, Aryloxyalkyl, Arylthioalkyl, Heterocyclyl, Heterocyclyloxy, Heterocyclylthio, Heterocyclylalkyl, Heterocyclylalkyloxy oder Heterocyclylalkylthio.
und die Verbindungen 3-(Formylamino)-2-hydroxy-N-{4-[2,4,6-tris-(1-methylpropyl)-phenoxy]-phenyl}-benzamid, N-{4-[3,5-Bis-(1,1-dimethylethyl)-phenoxy]-phenyl}-3-(formylamino)-2-hydroxy-benzamid, N-{4-[2,4-Bis-(1,1-dimethylethyl)-phenoxy]-phenyl}-3-(formylamino)-2-hydroxy-benzamid, N-{4-[2,6-Bis-(1-methylpropyl)-phenoxy]-phenyl}-3-(formylamino)-2-hydroxybenzamid, 3-(Formylamino)-2-hydroxy-N-{4-[3-(trifluoromethyl)-phenoxy]-phenyl}-benzamid, N-{4-[4-(1,1-dimethylethyl)-phenoxy]-phenyl}-3-(formylamino)-2-hydroxy-benzamid, 3-(Formylamino)-2-hydroxy-N-(4-phenoxyphenyl)-benzamid, N-(4-Butylphenyl)-3-(formylamino)-2-hydroxy-benzamid und N-{3-chloro-4-(4-chlorophenoxy)phenyl}-3-(formylamino)-2-hydroxy-benzamid, 3-(Formylamino)-2-hydroxy-N-(phenylmethyl)-benzamid, 3-Formamidosalicylanilid, 3-(Formylamino)-2-hydroxy-N-(2-phenylethyl)-benzamid und 4'-Chlor-3-acetylaminosalicylanilid ausgenommen sind.

3. Verbindungen der Formel (I) gemäß Anspruch 2, in welcher
A für eine Einfachbindung oder für Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
R¹ für Wasserstoff, Methyl, Ethyl, n- oder i-Propyl, n-, i-, s- oder t-Butyl, Methoxy, Ethoxy, n- oder i-Propoxy steht,
R² für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
oder fiir jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxy-carbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy, Cyclopropyl, Cyclobutyl, Cyclopentyl oder Cyclohexyl,
substituiertes Phenyl, Phenoxy, Phenylalkyl, Phenylthio, Phenoxyalkyl, Phenylthioalkyl, Phenylalkyloxy oder Phenylalkylthio, mit jeweils 1 bis 4 Kohlenstoffatomen in den jeweiligen Alkylketten.

4. Verbindungen der Formel (I) gemäß Anspruch 2, in welcher
A für eine Einfachbindung oder für Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
R¹ für Wasserstoff, Methyl, Ethyl, Methoxy oder Ethoxy steht,
R² für jeweils gegebenenfalls einfach bis sechsfach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis virrfach, gleich oder verschieden durch Fluor, Chlor, Methyl, Trifluormethyl, Ethyl, n- oder i-Propyl substituiertes, jeweils zweifach verknüpftes Trimethylen (propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (propan-1,3-diyl), Tetramethylen (Butan-1,4-diyl), Methylendioxy oder Ethylendioxy,
Cyclopropyl, Cyclobutyl, Cyclopentyl oder Cyclohexyl,
substituiertes Phenyl, Phenoxy, Phenylthio, Benzyl, Phenylethyl, Phenylpropyl, Benzyloxy, Benzylthio, Phenoxymethyl, Phenoxyethyl, Phenylthiomethyl oder Phenylthioethyl.

5. Verbindungen der Formel (I) gemäß Anspruch 2, in welcher
A für eine Einfachbindung oder für Methylen, 1,1-Ethylen, 1,2-Ethylen, 1,1-Propylen, 1,2-Propylen oder 2,2-Propylen steht,
R¹ für Wasserstoff steht,
R² für jeweils gegebenenfalls einfach oder zweifach durch Methyl, Ethyl, Methoxy oder Ethoxy substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils gegebenenfalls einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten aus der nachstehenden Aufzählung ausgewählt sind:
Fluor, Chlor, Brom, Cyano, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Fonnyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
substituiertes Phenyl, Phenoxy, Phenylthio, Benzyl, Phenyl-1-ethyl, Phenyl-2-ethyl, Benzyloxy, Benzylthio, Phenoxymethyl oder Phenylthiomethyl.

6. Schädlingsbekämpfungsmittel, **gekennzeichnet durch** einen Gehalt an mindestens einer Verbindung der Formeln (I) nach den Ansprüchen 2 bis 5.

7. Verfahren zur Bekämpfung von Schädlingen, **dadurch gekennzeichnet, daß** man Verbindungen der Formel (I) nach den Ansprüchen 2 bis 5 auf Schädlinge und/oder ihren Lebensraum einwirken läßt.

8. Verfahren zur Herstellung von Schädlingsbekämpfungsmitteln, **dadurch gekennzeichnet, daß** man Verbindungen der Formel (I) nach den Ansprüchen 1 bis 5 mit Streckmitteln und/oder oberflächenaktiven Mitteln vermischt.

9. Verfahren zur Herstellung von Verbindungen der Formel (I), 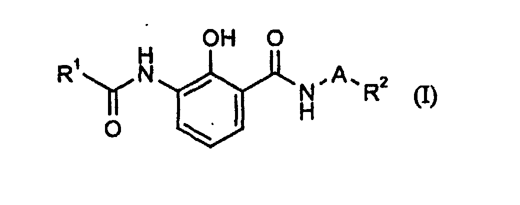 worin
R¹, A und R² die in Anspruch 2 angegebenen Bedeutungen haben,
**dadurch gekennzeichnet, daß** man
a) Aminosalicylsäureamide der allgemeinen Formel (II), 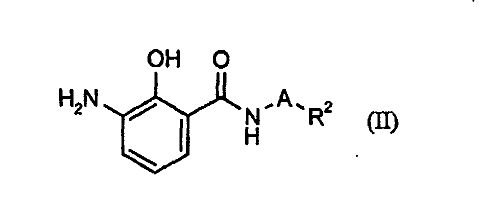 in welcher
A und R² die in Anspruch 11 angegebenen Bedeutungen haben,
mit Acylierungsmitteln der allgemeinen Formel (III), 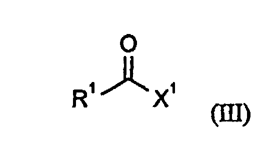 in welcher
R¹ die in Anspruch 2 angegebene Bedeutung hat und
X¹ für Halogen, Hydroxy, Alkoxy oder Alkylcarbonyloxy steht,
gegebenenfalls in Gegenwart eines Verdünnungsmittels, gegebenenfalls in Gegenwart eines Säureakzeptors, und gegebenenfalls in Gegenwart eines weiteren Reaktionshilfsmittels, umsetzt, oder wenn man
b) Nitrosalicylsäureamide der allgemeinen Formel (IV), 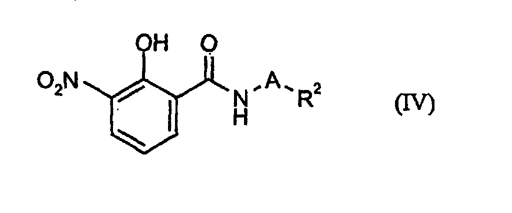 in welcher
A und R² die in Anspruch 13 angegebenen Bedeutungen haben,
mit Ameisensäure, gegebenenfalls in Gegenwart eines Katalysators und gegebenenfalls in Gegenwart eines weiteren Reaktionshilfsmittels, umsetzt, oder wenn man
c) O-Benzyl-nitrosalicylsäureamide der allgemeinen Formel (V), 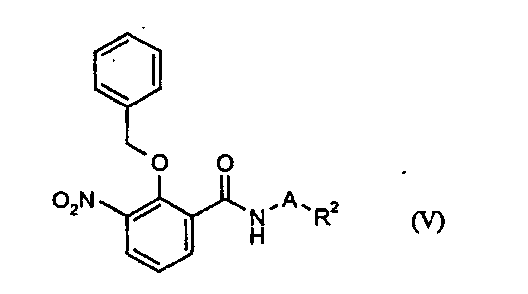 in welcher
A und R² die in Anspruch 2 angegebenen Bedeutungen haben,
mit Ameisensäure, gegebenenfalls in Gegenwart von Wasserstoff oder eines unedlen Metalls, gegebenenfalls in Gegenwart eines Katalysators und gegebenenfalls in Gegenwart eines weiteren Reaktionshilfsmittels, umsetzt.

10. Verbindungen der Formel (II), 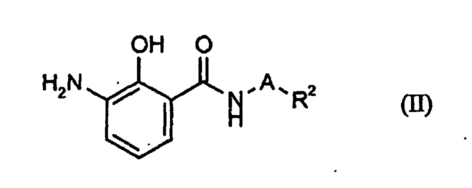 in welcher
A und R² die in Anspruch 2 angegebenen Bedeutungen haben mit Ausnahme von 4-{4-[(3-Amino-2-hydroxybenzoyl)-amino]-3-hydroxy-1-piperidyl}-N,N,4-trimethyl-2,2-diphenyl-butanamid und 3-Aminosalicylanilid.

11. Verbindungen der Formel (V), 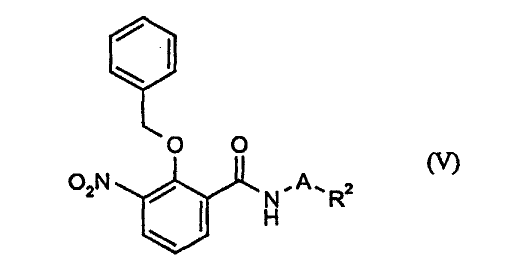 in welcher A und R² die in Anspruch 2 angegebenen Bedeutungen haben.

12. Verbindungen der Formel (IV-a), 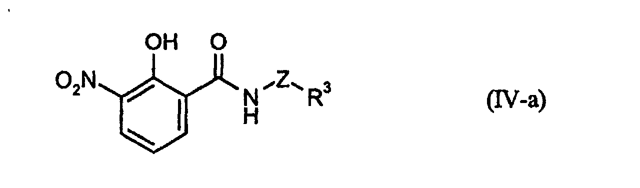 in welcher
Z für eine Einfachbindung oder für 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
R³ für jeweils gegebenenfalls einfach bis dreifach durch Fluor, Chlor, Brom, Cyano, Carboxy, Methyl, Ethyl, Methoxy, Ethoxy, n- oder i-Propyl, Methoxy-carbonyl oder Ethoxy-carbonyl substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
oder für jeweils einfach bis dreifach substituiertes Phenyl, Naphthyl, Benzyl, Phenethyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Cyano, Amino, Carboxy, Carbamoyl, Thiocarbamoyl, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl,
substituiertes Phenyl, Phenoxy, Phenylalkyl, Phenylthio, Phenoxyalkyl, Phenylthioalkyl, Phenylalkyloxy oder Phenylalkylthio, mit jeweils 1 bis 4 Kohlenstoffatomen in den jeweiligen Alkylketten.

13. Verbindungen der Formel (IV-a) gemäß Anspruch 12, in welcher
Z für eine Einfachbindung oder für 1,1-Ethylen, 1,2-Ethylen, 1,1-Propylen, 1,2-Propylen oder 2,2-Propylen steht,
R³ für jeweils einfach oder zweifach durch Methyl, Ethyl, Methoxy oder Ethoxy substituiertes Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Tetralinyl, Decalinyl, Cyclododecatrienyl, Indanyl, Norbornyl oder Adamantyl;
für jeweils einfach bis dreifach substituiertes Phenyl, Naphthyl, Furyl, Benzofuranyl, Pyrrolyl, Indolyl, Thienyl, Benzothienyl, Pyridyl, Chinolyl, Pyrimidyl, Pyridazinyl, Pyrazinyl steht, wobei die möglichen Substituenten aus der nachstehenden Aufzählung ausgewählt sind:
Cyano, Formyl, Carboxy, Carbamoyl, Thiocarbamoyl, Methyl, Ethyl, n-, oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
und/oder gegebenenfalls einfach bis vierfach, gleich oder verschieden durch Fluor, Chlor, Brom, Cyano, Formyl, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetylamino, Formylamino, N-Formyl-N-methylamino, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
substituiertes Phenyl, Phenoxy, Phenylthio, Benzyl, Phenyl-1-ethyl, Phenyl-2-ethyl, Benzyloxy, Benzylthio, Phenoxymethyl oder Phenylthiomethyl.

14. Verwendung von Verbindungen der Formel (V) wie in Anspruch 11 definiert zur Bekämpfung von Schädlingen.

## Claims

1. Use of compounds of the formula (I) 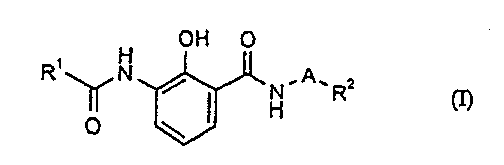 in which
A represents a single bond or an alkylene chain having 1 to 6 carbon atoms,
R¹ represents hydrogen, alkyl or alkoxy, each of which has 1 to 4 carbon atoms,
R² represents cycloalkyl or cycloalkenyl, each of which has 3 to 12 carbon atoms and each of which is optionally monosubstituted or polysubstituted by identical or different substituents from the series consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxy-carbonyl;
or represents aryl having up to 12 ring members or heterocyclyl having 3 to 8 ring members, it being possible for each of these aryl or heterocyclyl substituents to be optionally monosubstituted or polysubstituted by identical or different substituents, the possible substituents being selected from the enumeration which follows:
halogen, cyano, amino, hydroxyl, formyl, carboxyl, carbamoyl, thiocarbamoyl;
in each case straight-chain or branched alkyl, alkoxy, alkylthio, alkylsulphinyl or alkylsulphonyl, each of which has 1 to 6 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy, each of which has 2 to 6 carbon atoms and 1 to 13 identical or different halogen atoms;
in each case straight-chain or branched acylamino, N-acyl-N-alkylamino, alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties;
in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms; or aryl, aryloxy, arylthio, arylalkyl, arylalkyloxy, arylalkylthio, aryloxyalkyl, arylthioalkyl, heterocyclyl, heterocyclyloxy, heterocyclylthio, heterocyclylalkyl, heterocyclylalkyloxy or heterocyclylalkylthio, each of which is optionally monosubsituted or polysubstituted by identical or different substituents from the series consisting of
halogen, cyano, amino, hydroxyl, formyl, carboxyl, carbamoyl, thiocarbamoyl;
in each case straight-chain or branched alkyl, alkoxy, alkylthio, alkylsulphinyl or alkylsulphonyl, each of which has 1 to 6 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy, each of which has 2 to 6 carbon atoms and 1 to 13 identical or different halogen atoms;
in each case straight-chain or branched acylamino, N-acyl-N-alkylamino, alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties;
in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
for controlling pests on plants and industrial materials.

2. Compounds of the formula (I) 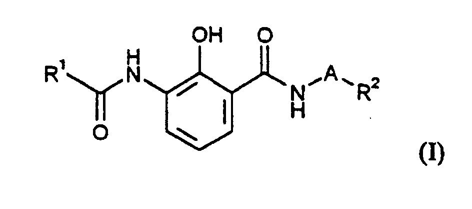 in which
A represents a single bond or an alkylene chain having 1 to 6 carbon atoms,
R¹ represents hydrogen, alkyl or alkoxy, each of which has 1 to 4 carbon atoms,
R² represents cycloalkyl or cycloalkenyl, each of which has 3 to 12 carbon atoms and each of which is optionally monosubstituted or polysubstituted by identical or different substituents from the series consisting of halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxy-carbonyl;
or represents aryl having up to 12 ring members or heterocycyl having 3 to 8 ring members, it being possible for each of these aryl or heterocyclyl substituents to be optionally monosubstituted or polysubstituted by identical or different substituents, the possible substituents being selected from the enumeration which follows: halogen, cyano, amino, hydroxyl, formyl, carboxyl, carbamoyl, thiocarbamoyl;
in each case straight-chain or branched alkyl, alkoxy, alkylthio, alkylsulphinyl or alkylsulphonyl, each of which has 1 to 6 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy, each of which has 2 to 6 carbon atoms and 1 to 13 identical or different halogen atoms;
in each case straight-chain or branched acylamino, N-acyl-N-alkylamino, alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties;
in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
or aryl, aryloxy, arylthio, arylalkyl, arylalkyloxy, arylalklthio, aryloxyalkyl, arylthioalkyl, heterocyclyl, heterocyclyloxy, hetercyclylthio, heterocycylakyl, heterocycylalkyloxy or heterocycylalkylthio, each of which is optionally monosubstituted or polysubstituted by identical or different substituents from the series consisting of:
halogen, cyano, amino, hydroxyl, formyl, carboxyl, carbamoyl, thiocarbamoyl;
in each case straight-chain or branched alkyl, alkoxy, alkylthio, alkylsulphinyl or alkylsulphonyl, each of which has 1 to 6 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy, each of which has 2 to 6 carbon atoms and 1 to 13 identical or different halogen atoms;
in each case straight-chain or branched acylamino, N-acyl-N-alkylamino, alkylamino, dialkylamino, alkylcarbonyl, alkylcarbonyloxy, alkoxycarbonyl, alkylsulphonyloxy, hydroximinoalkyl or alkoximinolkyl, each of which has 1 to 6 carbon atoms in the individual alkyl moieties;
in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms; cycloalkyl having 3 to 6 carbon atoms,
with the exception of the compounds 3-(formylamino)-2-hydroxy-N-{4-[2,4,6-tris-(1-methylpropyl)-phenoxy]-phenyl}-benzamide, N-{4-[3,5-bis-(1,1-dimethylethyl)-phenoxy]-phenyl}-3-(formylamino)-2-hydroxy-benzamide, N-{4-[2,4-bis-(1,1-dimethylethyl)-phenoxy]-phenyl}-3-(formylamino)-2-hydroxy-benzamide, N-{4-[2,6-bis-(1-methylpropyl)-phenoxy]-phenyl}-3-(formylamino)-2-hydroxy-benzamide, 3-(formylmino)-2-hydroxy-N-{4-[3-trifluoromethyl)-phenoxy]-phenyl}-benzamide, N-{4-[4-(1,1-dimethylethyl)-phenoxy]-phenyl}-3-(formylamino)-2-hydroxy-benzamide, 3-(formylamino)-2-hydroxy-N-(4-phenoxyphenyl)-benzamide, N-(4-butylphenyl)-3-(formylamino)-2-hydroxy-benzamide and N-{3-chloro-4-(4-chlorophenoxy)phenyl}-3-(formylamino)-2-hydroxy-benzamide, 3-(formylamino)-2-hydroxy-N-(phenylmethyl)-benzamide, 3-formamido-salicylanilide, 3-(formylamino)-2-hydroxy-N-(2-phenylethyl)-benzamide and 4'-chloro-3-acetylaminosalicylanilide.

3. Compounds of the formula (I) according to Claim 2 in which
A represents a single bond, or methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene),
R¹ represents hydrogen, methyl, ethyl, n- or i-propyl, n-, i-, s- or t-butyl, methoxy, ethoxy, n- or i-propoxy,
R2 represents cyclopropyl, cyclobutyl, cyclopentyl, cyclohexyl, cycloheptyl, cyclooctyl, cyclononyl, cyclodecyl, cycloundecyl, cyclododecyl, tetralinyl, decalinyl, cyclododecatrienyl, indanyl, norbornyl or adamantyl, each of which is optionally monosubstituted to hexasubstituted by fluorine, chlorine, bromine, cyano, carboxyl, methyl, ethyl, methoxy, ethoxy, n- or i-propyl, methoxy-carbonyl or ethoxy-carbonyl;
or represents in each case optionally monosubstituted to trisubstituted phenyl, naphthyl, benzyl, phenethyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the enumeration which follows:
fluorine, chlorine, bromine, cyano, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted to tetrasubstituted by identical or different substituents from the series consisting of fluorine, chlorine, methyl, trifluoromethyl, ethyl and n- or i-propyl,
cyclopropyl, cyclobutyl, cyclopentyl or cyclohexyl,
and/or phenyl, phenoxy, phenylalkyl, phenylthio, phenoxyalkyl, phenylthioalkyl, phenylalkyloxy or phenylalkylthio, having in each case 1 to 4 carbon atoms in the respective alkyl chains, which, in turn, are optionally monosubstituted to tetrasubstituted by identical or different substituents from the series consisting of fluorine, chlorine, bromine, cyano, formyl, carbamoyl, thiocarbamoyl, methyl, ethyl, n- or i-propyl, n-, i-, s- or t-butyl, methoxy, ethoxy, nor i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted or polysubstituted by identical or different substituents from the series consisting of fluorine, chlorine, methyl, trifluoromethyl, ethyl and n- or i-propyl,
cyclopropyl, cyclobutyl, cyclopentyl or cyclohexyl.

4. Compounds of the formula (I) according to Claim 2 in which
A represents a single bond or methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene),
R¹ represents hydrogen, methyl, ethyl, methoxy or ethoxy,
R² represents cyclopropyl, cyclobutyl, cyclopentyl, cyclohexyl, cycloheptyl, cyclooctyl, cyclononyl, cyclodecyl, cycloundecyl, cyclododecyl, tetralinyl, decalinyl, cyclododecatrienyl, indanyl, norbornyl or adamantyl, each of which is optionally monosubstituted to hexasubstituted by fluorine, chlorine, bromine, cyano, carboxyl, methyl, ethyl, methoxy, ethoxy, n- or i-propyl, methoxy-carbonyl or ethoxy-carbonyl;
or represents in each case optionally monosubstituted to trisubstituted phenyl, naphthyl, benzyl, phenethyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the enumeration which follows:
fluorine, chlorine, bromine, cyano, formyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted to tetrasubstituted by identical or different substituents from the series consisting of fluorine, chlorine, methyl, trifluoromethyl, ethyl and n- or i-propyl,
cyclopropyl, cyclobutyl, cyclopentyl or cyclohexyl,
and/or phenyl, phenoxy, phenylthio, benzyl, phenylethyl, phenylpropyl, benzyloxy, benzylthio, phenoxymethyl, phenoxyethyl, phenylthiomethyl or phenylthioethyl which, in turn, are optionally monosubstituted to tetrasubstituted by identical or different substituents from the series consisting of fluorine, chlorine, bromine, cyano, formyl, carbamoyl, thiocarbamoyl, methyl, ethyl, n- or i-propyl, n-, i-, s- or t-butyl, methoxy, ethoxy, nor i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted or polysubstituted by identical or different substituents from the series consisting of fluorine, chlorine, methyl, trifluoromethyl, ethyl and n- or i-propyl,
cyclopropyl, cyclobutyl, cyclopentyl or cyclohexyl.

5. Compounds of the formula (I) according to Claim 2 in which
A represents a single bond or methylene, 1,1-ethylene, 1,2-ethylene, 1,1-propylene, 1,2-propylene or 2,2-propylene,
R¹ represents hydrogen,
R2 represents cyclopropyl, cyclobutyl, cyclopentyl, cyclohexyl, cycloheptyl, cyclooctyl, cyclononyl, cyclodecyl, cycloundecyl, cyclododecyl, tetralinyl, decalinyl, cyclododecatrienyl, indanyl, norbomyl or adamantyl, each of which is optionally monosubstituted or disubstituted by methyl, ethyl, methoxy or ethoxy;
or represents in each case optionally monosubstituted to trisubstituted phenyl, naphthyl, furyl, benzofuranyl, pyrrolyl, indolyl, thienyl, benzothienyl, pyridyl, quinolyl, pyrimidyl, pyridazinyl or pyrazinyl, and the possible substituents being selected from the enumeration which follows:
fluorine, chlorine, bromine, cyano, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted to tetrasubstituted by identical or different substituents from the series consisting of fluorine, chlorine, methyl, trifluoromethyl, ethyl and n- or i-propyl,
and/or phenyl, phenoxy, phenylthio, benzyl, phenyl-1-ethyl, phenyl-2-ethyl, benzyloxy, benzylthio, phenoxymethyl or phenylthiomethyl, which, in turn, are each case optionally monosubstituted to tetrasubstituted by identical or different substituents from the series consisting of fluorine, chlorine, bromine, cyano, formyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene(propane-1,3-diyl), tetramethylene(butane-1,4-diyl), methylenedioxy or ethylenedioxy, each of which is optionally monosubstituted or polysubstituted by identical or different substituents from the series consisting of fluorine, chlorine, methyl, trifluoromethyl, ethyl, n- or -i-propyl.

6. Pesticides, **characterized in that** they comprise at least one compound of the formulae (I) according to Claims 2 to 5.

7. Method of controlling pests, **characterized in that** compounds of the formula (I) according to Claims 2 to 5 are allowed to act on pests and/or their environment.

8. Process for the preparation of pesticides, **characterized in that** compounds of the formula (I) according to Claims 1 to 5 are mixed with extenders and/or surface-active agents.

9. Process for the preparation of compounds of the formula (I) 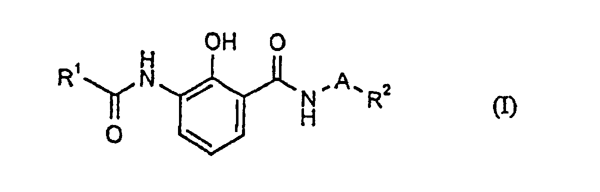 in which
R¹, A and R² have the meanings given in Claim 2
**characterized in that**
a) aminosalicylamides of the general formula (II) 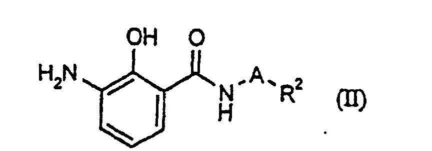 in which
A and R² have the meanings given in Claim 11
are reacted with acylating agents of the general formula (III) 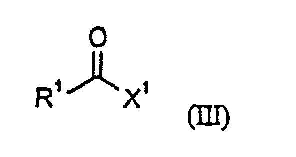 in which
R¹ has the meaning given in Claim 2 and
X¹ represents halogen, hydroxyl, alkoxy or alkylcarbonyloxy,
if appropriate in the presence of a diluent, if appropriate in the presence of an acid acceptor and if appropriate in the presence of a further reaction auxiliary, or when
b) nitrosalicylamides of the general formula (IV) 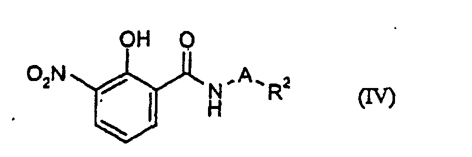 in which
A and R² have the meanings given in Claim 13,
are reacted with formic acid, if appropriate in the presence of a catalyst and if appropriate in the presence of a further reaction auxiliary, or when
c) O-benzyl-nitrosalicylamides of the general formula (V) 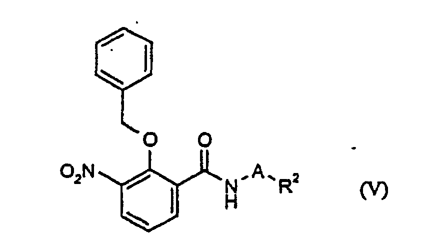 in which
A and R² have the meanings given in Claim 2,
are reacted with formic acid, if appropriate in the presence of hydrogen or a non-noble metal, if appropriate in the presence of a catalyst and if appropriate in the presence of a further reaction auxiliary.

10. Compounds of the formula (II) 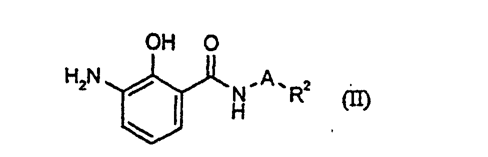 in which
A and R² have the meanings given in Claim 2 with the exception of 4-{4-[(3-amino-2-hydroxybenzoyl)-amino]-3-hydroxy-1-piperidyl}-N,N,4-trimethyl-2,2-diphenyl-butanamide and 3-aminosalicylanilide.

11. Compounds of the formula (V) 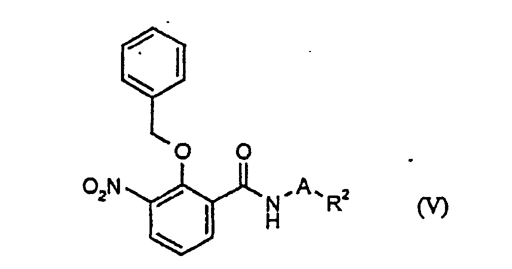 in which A and R² have the meanings given in Claim 2.

12. Compounds of the formula (IV-a) 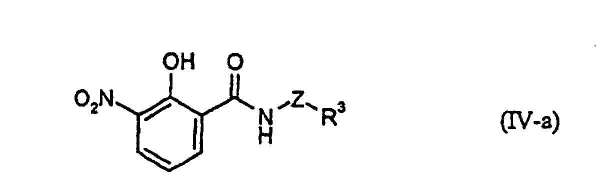 in which
Z represents a single bond, or represents 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene),
R3 represents cyclopropyl, cyclobutyl, cyclopentyl, cyclohexyl, cycloheptyl, cyclooctyl, cyclononyl, cyclodecyl, cycloundecyl, cyclododecyl, tetralinyl, decalinyl, cyclododecatrienyl, indanyl, norbornyl or adamantyl, each of which is optionally monosubstituted to trisubstituted by fluorine, chlorine, bromine, cyano, carboxyl, methyl, ethyl, methoxy, ethoxy, n- or i-propyl, methoxy-carbonyl or ethoxy-carbonyl;
or represents in each case monosubstituted to trisubstituted phenyl, naphthyl, benzyl, phenethyl, furyl, bezofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents being selected from the enumeration which follows:
cyano, amino, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsufonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsufonyloxy, ethylsulphonyloxy, hydroximinomethyl, hydroximinoethyl, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, each of which is optionally monosubstituted to tetrasubstituted by identical or different substituents from the series consisting of fluorine, chlorine, methyl, trifluoromethyl, ethyl or n- or i-propyl,
cyclopropyl, cyclobutyl, cyclopentyl or cyclohexyl,
and/or phenyl, phenoxy, phenylalkyl, phenylthio, phenoxyalkyl, phenylthioalkyl, phenylalkyloxy or phenylalkylthio, each of which has 1 to 4 carbon atoms in the respective alkyl chains, and each of which is optionally monosubstituted to tetrasubstituted by identical or different substituents from the series consisting of fluorine, chlorine, bromine, cyano, formyl, carbamoyl, thiocarbamoyl, methyl, ethyl, n- or i-propyl, n-, i-, s- or t-butyl, methoxy, ethoxy, nor i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamono, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene (propane-1,3-diyl), tetramethylene (butane-1,4-diyl) methylenedioxy or ethylenedioxy, each of which is optionally monosubstituted or polysubstituted by identical or different substituents from the series consisting of fluorine, chlorine, methyl, trifluoromethyl, ethyl or n- or i-propyl,
cyclopropyl, cyclobutyl, cyclopentyl or cyclohexyl.

13. Compounds of the formula (IV-a) according to Claim 12, in which
Z represents a single bond, or represents 1,1-ethylene, 1,2-ethylene, 1,1-propylene, 1,2-propylene or 2,2-propylene,
R3 represents cyclopropyl, cyclobutyl, cyclopentyl, cyclohexyl, cycloheptyl, cyclooctyl, cyclononyl, cyclodecyl, cycloundecyl, cyclododecyl, tetralinyl, decalinyl, cyclododecatrienyl, indanyl, norbornyl or adamantyl, each of which is optionally monsubstituted or disubstituted or methyl, ethyl, methoxy or ethoxy;
in each case monosubstituted to trisubstituted phenyl, naphthyl, furyl, benzofuranyl, pyrrolyl, indolyl, thienyl, benzothienyl, pyridyl, chinolyl, pyrimidyl, pyridazinyl, pyrazinyl, the possible substituents being selected from the enumeration which follows:
cyano, formyl, carboxyl, carbamoyl, thiocarbamoyl, methyl, ethyl, n- or i-propyl, n-, i, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene (propane-1,3-diyl), tetramethylene (butane-1,4,-diyl), methylenedioxy or ethylenedioxy, each of which is optionally monosubstituted to tetrasubstituted by identical or different substitutents from the series consisting of fluorine, chlorine, methyl, trifluormethyl, ethyl or nor i-propyl,
and/or phenyl, phenoxy, phenylthio, benzyl, phenyl-1-ethyl, phenyl-2-ethyl, benzyloxy, benzylthio, phenoxymethyl or phenylthiomethyl, each of which is optionally monosubstituted to tetrasubstituted by identical or different substitutents from the series consisting of fluorine, chlorine, bromine, cyano, formyl, carbamoyl, thiocarbamoyl, methyl, ethyl, n- or i-propyl, n-, i-, s-, or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, acetylamino, formylamino, N-formyl-N-methylamino, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, each of which is optionally monosubstituted or polysubstituted by identical or different substituents from the series consisting of fluorine, chlorine, methyl, trifluromethyl, ethyl or n- or i-propyl.

14. Use of compounds of the formula (V) as defined in Claim 11 for controlling pests.

## Revendications

1. Utilisation de composés de formule (I) 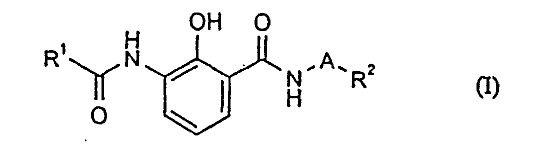 dans laquelle
A est une liaison simple ou une chaîne alkylénique ayant 1 à 6 atomes de carbone,
R¹ représente l'hydrogène, un reste alkyle ou un reste alkoxy ayant chacun 1 à 4 atomes de carbone,
R² est un reste cycloalkyle ou un reste cycloalcényle de 3 à 12 atomes de carbone, portant chacun, le cas échéant, un ou plusieurs substituants, identiques ou différents, halogéno, cyano, carboxy, phényle (qui porte éventuellement un substituant halogéno, cyano, alkyle en C₁ à C₄, halogénalkyle en C₁ à C₄, alkoxy en C₁ à C₄, ou halogénalkoxy en C₁ à C₄), alkyle en C₁ à C₄ ou (alkoxy en C₁ à C₄)-carbonyle ;
un reste aryle ayant jusqu'à 12 chaînons dans le noyau ou un reste hétérocyclyle ayant 3 à 8 chaînons dans le noyau, portant chacun, le cas échéant, un ou plusieurs substituants identiques ou différents, les substituants possibles étant choisis parmi ceux qui sont énumérés ci-après :
halogéno, cyano, amino, hydroxy, formyle, carboxy, carbamoyle, thiocarbamoyle ;
alkyle, alkoxy, alkylthio, alkylsulfinyle ou alkylsulfonyle, chacun linéaire ou ramifié ayant chacun 1 à 6 atomes de carbone ; alcényle ou alcényloxy, chacun linéaire ou ramifié ayant chacun 2 à 6 atomes de carbone ;
halogénalkyle, halogénalkoxy, halogénalkylthio, halogénalkylsulfinyle ou halogénalkylsulfonyle, chacun linéaire ou ramifié ayant chacun 1 à 6 atomes de carbone et 1 à 13 atomes d'halogènes identiques ou différents ;
halogénalcényle ou halogénalcényloxy, chacun linéaire ou ramifié ayant chacun 2 à 6 atomes de carbone et 1 à 13 atomes d'halogène identiques ou différents ;
acylamino, N-acyl-N-alkylamino, alkylamino, dialkylamino, alkylcarbonyle, alkylcarbonyloxy, alkoxycarbonyle, alkylsulfonyloxy, hydroximinoalkyle ou alkoximinoalkyle, chacun linéaire ou ramifié ayant chacun 1 à 6 atomes de carbone dans les parties alkyle individuelles ;
alkylène ou dioxyalkylène ayant chacun 1 à 6 atomes de carbone et chacun deux liaisons, chacun portant, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'halogéno, cyano, amino, hydroxy, formyle, carboxy, carbamoyle, thiocarbamoyle portant chacun, le cas échéant, un ou plusieurs substituants, identiques ou différents ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
acylamino, N-acyl-N-alkylamino, alkylamino, dialkylamino, alkylcarbonyle, alkylcarbonyloxy, alkoxycarbonyle, alkylsulfonyloxy, hydroximinoalkyle ou alkoximinoalkyle chacun linéaire ou ramifié ayant chacun 1 à 6 atomes de carbone dans les parties alkyle individuelles ;
alkylène ou dioxyalkylène ayant chacun 1 à 6 atomes de carbone et chacun deux liaisons, portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
aryle, aryloxy, arylthio, arylalkyle, arylalkyloxy, arylalkylthio, aryloxyalkyle, arylthioalkyle, hétérocyclyle, hétérocyclyloxy, hétérocyclylthio, hétérocyclylalkyle, hétérocyclylalkyloxy ou hétérocyclylalkylthio portant chacun un ou plusieurs substituants, identiques ou différents,
pour combattre des parasites sur des plantes et sur des matériaux techniques.

2. Composés de formule (I) 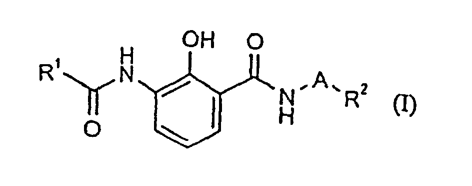 dans laquelle
A est une liaison simple ou une chaîne alkylénique ayant 1 à 6 atomes de carbone,
R¹ représente l'hydrogène, un reste alkyle ou un reste alkoxy ayant chacun 1 à 4 atomes de carbone,
R² représente un reste cycloalkyle ou cycloalcényle ayant chacun 3 à 12 atomes de carbone et portant chacun, le cas échéant, un ou plusieurs substituants, identiques ou différents, halogéno, cyano, carboxy, phényle (qui est éventuellement substitué par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle ;
un reste aryle à noyau ayant jusqu'à 12 chaînons ou un reste hétérocyclyle à noyau de 3 à 8 chaînons portant chacun, le cas échéant, un ou plusieurs substituants identiques ou différents, les substituants possibles étant choisis parmi ceux qui sont énumérés ci-après :
halogéno, cyano, amino, hydroxy, formyle, carboxy, carbamoyle, thiocarbamoyle ;
alkyle, alkoxy, alkylthio, alkylsulfinyle ou alkylsulfonyle, chacun linéaire ou ramifié ayant chacun 1 à 6 atomes de carbone ;
alcényle ou alcényloxy, chacun linéaire ou ramifié ayant chacun 2 à 6 atomes de carbone ;
halogénalkyle, halogénalkoxy, halogénalkylthio, halogénalkylsulfinyle ou halogénalkylsulfonyle, chacun linéaire ou ramifié ayant chacun 1 à 6 atomes de carbone et 1 à 13 atomes d'halogènes identiques ou différents ;
halogénalcényle ou halogénalcényloxy, chacun linéaire ou ramifié ayant chacun 2 à 6 atomes de carbone et 1 à 13 atomes d'halogènes identiques ou différents ;
acylamino, N-acyl-N-alkylamino, alkylamino, dialkylamino, alkylcarbonyle, alkylcarbonyloxy, alkoxycarbonyle, alkylsulfonyloxy, hydroximinoalkyle ou alkoximinoalkyle, chacun linéaire ou ramifié ayant chacun 1 à 6 atomes de carbone dans les parties alkyle individuelles ;
alkylène ou dioxyalkylène ayant chacun 1 à 6 atomes de carbone et chacun deux liaisons, portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'un ou plusieurs substituants, identiques ou différents
halogéno, cyano, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
acylamino, N-acyl-N-alkylamino, alkylamino, dialkylamino, alkylcarbonyle, alkylcarbonyloxy, alkoxycarbonyle, alkylsulfonyloxy, hydroximinoalkyle ou alkoximinoalkyle chacun linéaire ou ramifié ayant chacun 1 à 6 atomes de carbone dans les parties alkyle individuelles ;
alkylène ou dioxyalkylène ayant chacun 1 à 6 atomes de carbone et chacun deux liaisons, portant chacun, le cas échéant, un ou plusieurs substituants, identiques
ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
aryle, aryloxy, arylthio, arylalkyle, arylalkyloxy, arylalkylthio, aryloxyalkyle, arylthioalkyle, hétérocyclyle, hétérocyclyloxy, hétérocyclylthio, hétérocyclylalkyle, hétérocyclylalkyloxy ou hétérocyclylalkylthio portant chacun, le cas échéant, un ou plusieurs substituants, identiques ou différents ;
et les composés 3-(formylamino)-2-hydroxy-N-{4-[2,4,6-tris-(1-méthylpropyl)-phénoxy]-phényl}-benzamide, N-{4-[3,5-bis-(1,1-diméthyléthyl)-phénoxy]-phényl}-3-(formylamino)-2-hydroxy-benzamide, N-{4-[2,4-bis-(1,1-diméthyléthyl)-phénoxy]-phényl}-3-(formylamino)-2-hydroxy-benzamide, N-{4-[2,6-bis-(1-méthylpropyl)-phénoxy]-phényl}-3-(formylamino)-2-hydroxy-benzamide, 3-(formylamino)-2-hydroxy-N-{4-[3-(trifluorométhyl)-phénoxy]-phényl}-benzamide, N-{4-[4-(1,1-diméthyléthyl)-phénoxy-phényl}-3-(formylamino)-2-hydroxybenzamide, 3-(formylamino)-2-hydroxy-N-(4-phénoxyphényl)-benzamide, N-(4-butylphényl)-3-(formylamino)-2-hydroxybenzamide et N-{3-chloro-4-(4-chlorophénoxy)phényl}-3-(formylamino)-2-hydroxy-benzamide, 3-(formylamino)-2-hydroxy-N-(phénylméthyl)-benzamide, 3-formamidosalicylanilide, 3-(formylamino)-2-hydroxy-N-(2-phényléthyl)-benzamide et 4'-chloro-3-acétylaminosalicylanilide étant exclus.

3. Composés de formule (I) suivant la revendication 2, formule dans laquelle
A est une liaison simple ou un reste méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène),
R¹ est l'hydrogène, un reste méthyle, éthyle, n-propyle, isopropyle, n-butyle, isobutyle, sec.-butyle, tertio-butyle, méthoxy, éthoxy, n-propoxy ou isopropoxy,
R² est un reste cyclopropyle, cyclobutyle, cyclopentyle, cyclohexyle, cycloheptyle, cyclo-octyle, cyclononyle, cyclodécyle, cyclo-undécyle, cyclododécyle, tétralinyle, décalinyle, cyclododécatriényle, indanyle, norbornyle ou adamantyle portant chacun, le cas échéant, un à six substituants fluoro, chloro, bromo, cyano, carboxy, méthyle, éthyle, méthoxy, éthoxy, n-propoxy, isopropoxy, méthoxycarbonyle ou éthoxycarbonyle ;
ou bien un reste phényle, naphtyle, benzyle, phénéthyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun, le cas échéant, un à trois substituants, les substituants possibles étant choisis parmi ceux qui sont énumérés ci-après :
fluor, chlore, brome, cyano, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyle, éthylsulfonyloxy, hydroximinométhyle, hydroximino-éthyle, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy portant chacun, le cas échéant, un à quatre substituants fluoro, chloro, méthyle, trifluorométhyle, éthyle, n-propyle ou isopropyle identiques ou différents,
cyclopropyle, cyclobutyle, cyclopentyle ou cyclohexyle,
et/ou phényle, phénoxy, phénylalkyle, phénylthio, phénoxyalkyle, phénylthioalkyle, phénylalkyloxy ou phénylalkylthio ayant chacun 1 à 4 atomes de carbone dans les chaînes alkyle individuelles, portant éventuellement un à quatre substituants, identiques ou différents, fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy ayant chacun deux liaisons et portant chacun, le cas échéant, un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

4. Composés de formule (I) suivant la revendication 2, dans laquelle
A est une liaison simple ou un reste méthylène, 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène),
R¹ est l'hydrogène, un reste méthyle, éthyle, méthoxy ou éthoxy,
R² est un reste cyclopropyle, cyclobutyle, cyclopentyle, cyclohexyle, cycloheptyle, cyclo-octyle, cyclononyle, cyclodécyle, cyclo-undécyle, cyclododécyle, tétralinyle, décalinyle, cyclododécatriényle, indanyle, norbornyle ou adamantyle portant chacun, le cas échéant, un à six substituants fluoro, chloro, bromo, cyano, carboxy, méthyle, éthyle, méthoxy, éthoxy, n-propoxy, isopropoxy, méthoxycarbonyle ou éthoxycarbonyle ;
un reste phényle, naphtyle, benzyle, phénétyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun, le cas échéant, un à trois substituants, les substituants possibles étant choisis parmi ceux qui sont énumérés ci-après :
fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy portant chacun, le cas échéant, un à quatre substituants, identiques ou différents, fluoro, chloro, méthyle, trifluorométhyle, éthyle, n-propyle ou isopropyle,
cyclopropyle, cyclobutyle, cyclopentyle ou cyclohexyle,
et/ou phényle, phénoxy, phénylthio, benzyle, phényléthyle, phénylpropyle, benzyloxy, benzylthio, phénoxyméthyle, phénoxyéthyle, phénylthiométhyle ou phénylthioéthyle portant éventuellement un à quatre substituants, identiques ou différents, fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy portant chacun le cas échéant, un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

5. Composés de formule (I) suivant la revendication 2, formule dans laquelle
A est une liaison simple ou un reste méthylène, 1,1-éthylène, 1,2-éthylène, 1,1-propylène, 1,2-propylène ou 2,2-propylène,
R¹ est l'hydrogène,
R² est un reste cyclopropyle, cyclobutyle, cyclopentyle, cyclohexyle, cycloheptyle, cyclo-octyle, cyclononyle, cyclodécyle, cyclo-undécyle, cyclododécyle, tétralinyle, décalinyle, cyclododécatriényle, indanyle, norbornyle ou adamantyle portant chacun, le cas échéant, un ou deux substituants méthyle, éthyle, méthoxy ou éthoxy ;
un reste phényle, naphtyle, furyle, benzofurannyle, pyrrolyle, indolyle, thiényle, benzothiényle, pyridyle, quinolyle, pyrimidyle, pyridazinyle, pyrazinyle portant chacun, le cas échéant, un à trois substituants, les substituants possibles étant choisis parmi ceux qui sont énumérés ci-après :
fluor, chlore, brome, cyano, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy ayant chacun deux liaisons et portant chacun, le cas échéant, un ou quatre substituants, identiques ou différents, fluoro, chloro, méthyle, trifluorométhyle, éthyle, n-propyle ou isopropyle,
et/ou phényle, phénoxy, phénylthio, benzyle, phényl-1-éthyle, phényl-2-éthyle, benzyloxy, benzylthio, phénoxyméthyle ou phénylthiométhyle portant éventuellement un à quatre substituants, identiques ou différents, fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-((propane-1,3-diyle), tétraméthylène-(butane-1,4-diyle), méthylènedioxy ou éthylènedioxy ayant chacun deux liaisons et portant chacun, le cas échéant, un ou plusieurs substituants, identiques ou différents, fluoro, chloro, méthyle, trifluorométhyle, éthyle, n-propyle ou isopropyle.

6. Compositions pesticides, **caractérisées par** une teneur en au moins un composé de formule (I) suivant les revendications 2 à 5.

7. Procédé pour combattre des parasites, **caractérisé en ce qu'**on fait agir des composés de formule (I) suivant les revendications 2 à 5 sur les parasites et/ou sur leur milieu.

8. Procédé de préparation de compositions pesticides, **caractérisé en ce qu'**on mélange des composés de formule (I) suivant les revendications 1 à 5 avec des diluants et/ou des agents tensio-actifs.

9. Procédé de production de composés de formule (I) 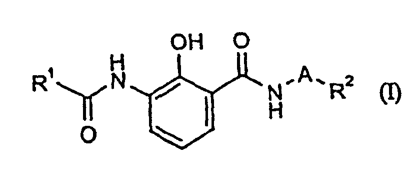 dans laquelle
R¹, A et R² ont les définitions indiquées dans la revendication 2,
**caractérisé en ce que** :
a) on fait réagir des aminosalicylamides de formule générale (II) 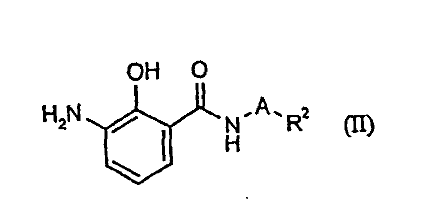 dans laquelle
A et R² ont les définitions indiquées dans la revendication 1,
avec des agents d'acylation de formule générale (III) 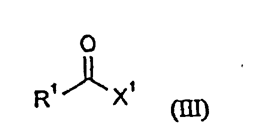 dans laquelle
R¹ a la définition indiquée dans la revendication 2 et
X¹ est un halogène, un groupe hydroxy, alkoxy ou alkylcarbonyloxy,
le cas échéant, en présence d'un diluant, éventuellement en présence d'un accepteur d'acide et, le cas échéant, en présence d'un autre auxiliaire de réaction, ou bien
b) on fait réagir des nitrosalicylamides de formule générale (IV) 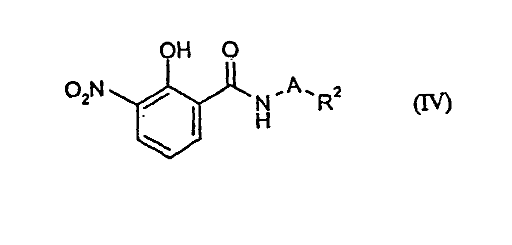 dans laquelle
A et R² ont les définitions indiquées dans la revendication 13,
avec l'acide formique, éventuellement en présence d'un catalyseur et, le cas échéant, en présence d'un autre auxiliaire de réaction,
ou bien
c) on fait réagir des O-benzyl-nitrosalicylamides de formule générale (V) 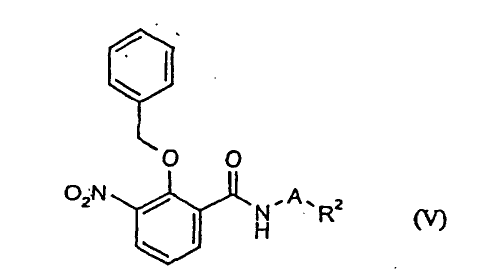 dans laquelle
A et R² ont les définitions indiquées dans la revendication 2,
avec l'acide formique, éventuellement en présence d'hydrogène ou d'un métal non noble, le cas échéant en présence d'un catalyseur et éventuellement en présence d'un autre auxiliaire de réaction.

10. Composés de formule (II) 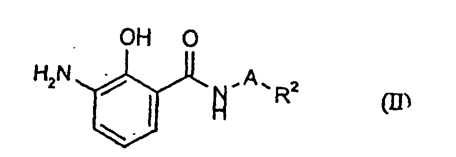 dans laquelle
A et R² ont les définitions indiquées dans la revendication 2, excepté le 4-{4-[(3-amino-2-hydroxybenzoyl)-amino]-3-hydroxy-1-pipéridyl}-N,N,4-triméthyl-2,2-diphényl-butanamide et le 3-aminosalicylanilide.

11. Composés de formule (V) 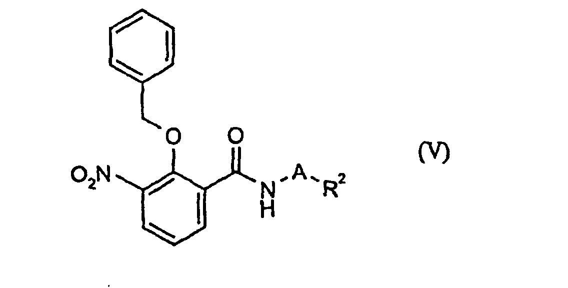 dans laquelle A et R² ont les définitions indiquées dans la revendication 2.

12. Composés de formule (IV-a) 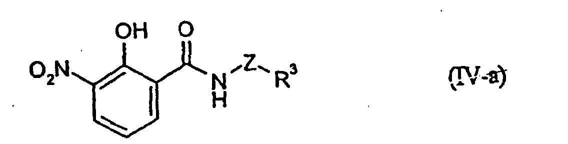 dans laquelle
Z est une liaison simple ou un reste 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2 ou 1,3-(2-méthylpropylène),
R³ est un reste cyclopropyle, cyclobutyle, cyclopentyle, cyclohexyle, cycloheptyle, cyclo-octyle, cyclononyle, cyclodécyle, cyclo-undécyle, cyclododécyle, tétralinyle, décalinyle, cyclododécatriényle, indanyle, norbornyle ou adamantyle portant chacun, le cas échéant, un à trois substituants fluoro, chloro, bromo, cyano, carboxy, méthyle, éthyle, méthoxy, éthoxy, n-propyle, isopropyle, méthoxycarbonyle ou éthoxycarbonyle ;
ou bien un reste phényle, naphtyle, benzyle, phénétyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétahydrofuryle, perhydropyrannyle, pyrrolidinyle, pipéridinyle ou morpholinyle portant chacun, le cas échéant, un à trois substituants, les substituants possibles étant choisis parmi ceux qui sont énumérés ci-après :
cyano, amino, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy ayant chacun deux liaisons et portant chacun, le cas échéant, un à quatre substituants fluoro, chloro, méthyle, trifluorométhyle, éthyle, n-propyle ou isopropyle identiques ou différents,
cyclopropyle, cyclobutyle, cyclopentyle ou cyclohexyle,
et/ou phényle, phénoxy, phénylalkyle, phénylthio, phénoxyalkyle, phénylthioalkyle, phénylalkyloxy ou phénylalkylthio ayant chacun 1 à 4 atomes de carbone dans les chaînes alkyle individuelles et portant chacun, le cas échéant, un à quatre substituants, identiques ou différents, fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy portant chacun, le cas échéant, un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

13. Composés de formule (IV-a) suivant la revendication 12, formule dans laquelle
Z est une liaison simple ou un reste 1,1-éthylène, 1,2-éthylène, 1,1-propylène, 1,2-propylène ou 2,2-propylène,
R³ est un reste cyclopropyle, cyclobutyle, cyclopentyle, cyclohexyle, cycloheptyle, cyclo-octyle, cyclononyle, cyclodécyle, cyclo-undécyle, cyclododécyle, tétralinyle, décalinyle, cyclododécatriényle, indanyle, norbornyle ou adamantyle portant chacun un à deux substituants méthyle, éthyle, méthoxy ou éthoxy ;
un reste phényle, naphtyle, furyle, benzofurannyle, pyrrolyle, indolyle, thiényle, benzothiényle, pyridyle, quinolyle, pyrimidyle, pyridazinyle ou pyrazinyle portant chacun, le cas échéant, un à trois substituants, les substituants possibles étant choisis parmi ceux qui sont énumérés ci-après :
cyano, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-fôrmyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy ayant chacun deux liaisons et portant chacun, le cas échéant, un à quatre substituants fluoro, chloro, méthyle, trifluorométhyle, éthyle, n-propyle ou isopropyle identiques ou différents,
et/ou phényle, phénoxy, phénylthio, benzyle, phényl-1-éthyle, phényl-2-éthyle, benzyloxy, benzylthio, phénoxyméthyle ou phénylthiométhyle, portant chacun, le cas échéant, un à quatre substituants, identiques ou différents, fluoro, chloro, bromo, cyano, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétylamino, formylamino, N-formyl-N-méthylamino, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, méthoximinométhyle, éthoximinométhyle, méthoximino-éthyle ou éthoximino-éthyle,
triméthylène-(propane-1,3-diyle), tétraméthylène-(butane-1,4-diyle), méthylènedioxy ou éthylènedioxy portant chacun, le cas échéant, un ou plusieurs substituants fluoro, chloro, méthyle, trifluorométhyle, éthyle, n-propyle ou isopropyle identiques ou différents.

14. Utilisation de composés de formule (V) telle que définie dans la revendication 11 pour combattre des parasites.
